(19) Europäisches Patentamt / European Patent Office / Office européen des brevets

(11) EP 4 497 747 A1

(12) EUROPEAN PATENT APPLICATION
published in accordance with Art. 153(4) EPC

(43) Date of publication:
**29.01.2025 Bulletin 2025/05**

(21) Application number: **23795450.8**

(22) Date of filing: **26.04.2023**

(51) International Patent Classification (IPC):
*C07D 239/04* (2006.01) *C07D 401/04* (2006.01)
*C07D 403/14* (2006.01) *A61K 31/506* (2006.01)
*A61P 35/00* (2006.01)

(52) Cooperative Patent Classification (CPC):
**A61K 31/505; A61K 31/506; A61K 31/5377; A61K 31/541; A61P 35/00; C07D 239/04; C07D 239/28; C07D 401/04; C07D 401/14; C07D 403/04; C07D 403/14; C07D 409/04; C07D 409/14; C07D 413/14; C07D 417/04;** (Cont.)

(86) International application number:
**PCT/CN2023/090761**

(87) International publication number:
**WO 2023/208018 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.04.2022 CN 202210475301**

(71) Applicant: **Arigenx Therapeutics Co., Ltd.**
**Shenzhen, Guangdong 518116 (CN)**

(72) Inventor: **ZHANG, Suoming**
**Shenzhen, Guangdong 518116 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) SUBSTITUTED PYRIMIDINE HYDRAZIDE COMPOUND, METHOD FOR PREPARING SAME, AND USE THEREOF

(57) The present disclosure discloses a substituted pyrimidinyl hydrazide compound shown in the following formula I and an optical isomer, prodrug, or pharmaceutically-acceptable salt thereof, a pharmaceutical composition including the substituted pyrimidinyl hydrazide compound, and a use thereof in preparation of an aryl hydrocarbon receptor (AHR) disorder inhibitor. The substituted pyrimidinyl hydrazide compound of the present disclosure can bind to AHR and inhibit those functions and signaling pathways controlled by AHR, thereby affecting the growth and proliferation of cancer cells and the invasiveness of tumor cells. Therefore, the substituted pyrimidinyl hydrazide compound of the present disclosure can be used to inhibit the growth of cancer cells and inhibit the metastasis and invasion of tumor cells.

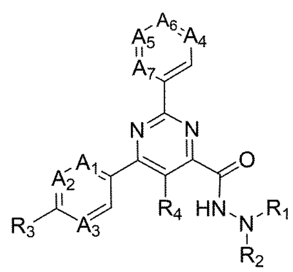

formula I



Processed by Luminess, 75001 PARIS (FR)

(Cont. next page)

(52) Cooperative Patent Classification (CPC): (Cont.)
**C07D 417/14**

**Description**

## TECHNICAL FIELD

The present disclosure belongs to the technical field of biopharmaceuticals, and specifically relates to a substituted pyrimidinyl hydrazide compound and a preparation method and use thereof.

## BACKGROUND TECHNOLOGY

The aryl hydrocarbon receptor (AHR) is a member of the bHLH-PER-ARNT-SIM (bHLH-PAS) subfamily of the basic helix-loop-helix (bHLH) superfamily. AHR is the only receptor in the bHLH-PAS family that can be activated by a ligand (Murray et al., Nat. Rev. Cancer, 2014, 14 (12), 801-814; Berstenetal., Nat. Rev. Cancer, 2013, 13 (12), 827-841). AHR present in the cytoplasm can be stimulated by aryl hydrocarbon xenobiotics such as 2,3,7,8-tetrachlorodibenzo-p-dioxin (TCDD) and then migrate into the cell nucleus to produce a heterodimer with the aryl hydrocarbon receptor nuclear translocator (ARNT), and the AHR/ARNT complex then interacts with the xenobiotic response element (XRE) upstream of the AHR regulatory gene to regulate the transcription of a corresponding gene. AHR can also activate XRE-independent protein-protein interaction pathways.

The AHR signaling pathway is one of the most well-known mechanisms to bind to environmental toxins and induce metabolism, such as regulated cytochrome P450 (CYP450) enzymes (such as CYP1A1, CYP1A2, and CYP1B1), and these enzymes can metabolize environmental toxins (Reyes et al., Science, 1992, 256 (5060), 1193-1195; Murray et al., Nat. Rev. Cancer, 2014, 14 (12), 801-814). Roles of AHR in a wide range of cell processes, such as embryonic development, tumorigenesis, and inflammation, have been proven through the activation of AHR by environmental toxins. AHR is expressed in many cells of the immune system, including dendritic cells, macrophages, T-cells, and natural killer (NK) cells, and plays an important role in immune regulation (Nguyen et al., Front. Immunol., 2014, 5, 511). Classical exogenous AHR ligands such as TCDD can induce the deep immunosuppression, promote the carcinogenesis, and induce the tumor growth (Gramatzkietal., Oncogene, 2009, 28 (28), 2593-2605; Buietal., Oncogene, 2009, 28 (41), 3642-3651; Esseretal., Trends. Immunol., 2009, 30 (9), 447-454).

Through an XRE-dependent or independent activity, AHR modulates a variety of key innate and adaptive immune responses. Studies have shown that some AHR agonists can promote the differentiation of Th17 cells (T-helper cells) and the secretion of IL-17. Other AHR agonists can induce the trans-differentiation of Th17 cells into Treg cells and enhance the inhibitory activity of Treg (Quintana et al., Nature, 2008, 453 (7191), 65-71; Mezrich et al., J. Immunol., 2010, 185 (6), 3190-3198). Studies have shown that the activation of AHR can inhibit the generation of innate inflammatory responses regulated by macrophages (such as reducing the expression of IL-1b, IL-6, IL-12, and TNFalpha induced by lipopolysaccharide (LPS)) and inhibit dendritic cells (reducing the activation of dendritic cells and promoting the expression of IL-10) (Kimura et al., J. Exp. Med., 2009, 206 (9), 2027-2035; Wang et al., Clin. Exp. Immunol., 2014, 177 (2), 521-530; Weiet al., Lab. Invest., 2014, 94 (5), 528-535; Nguyen et al., Proc. Natl. Acad. Sci. USA, 2010, 107 (46), 19961-19966).

In order to establish effective anti-tumor immune responses, antigen-presenting cells (APCs) need to process and present tumor antigens and then activate helper CD4+ T-cells (Th) and cytotoxic CD8+ T-cells (Tc), and these cells play a synergistic role to effectively kill tumor cells. Tumor cells have developed several mechanisms to evade the immunity regulated by Th and Tc cell lysis. One of the mechanisms is the release of kynurenine and other potential AHR ligands at high concentrations in a tumor microenvironment (TME).

AHR ligands at high concentrations in TME can lead to the direct inhibition of APCs, Th, and Tc and can also enhance the recruitment, production, and activation of Treg, thereby further inhibiting the activities of Tc and Th. Through these mechanisms, tumors can evade anti-tumor immune responses (Opitz et al., Nature, 2011, 478 (7368), 197-203). Therefore, AHR inhibitors can block the AHR-dependent immune escape pathways adopted by malignant tumor cells to restore the anti-tumor immunity.

Recent studies on tumor immunobiology have shown that malignant tumor cells adopt a variety of immune escape mechanisms to evade the immune system. Preclinical and clinical studies have demonstrated that blocking or strengthening these mechanisms through a combination of therapeutic applications (such as immune checkpoint inhibitors and vaccines) can provide the optimal recovery of an anti-tumor immune response. Research data has shown that AHR modulators can be used alone to restore the anti-tumor immunity. Due to the unique nature of the AHR signaling pathway, it can be expected that AHR inhibitors can be used in combination with other tumor immunotherapies or even can play a synergistic role with other treatments to enhance immunotherapy responses. Clinically, the response rate of an immune checkpoint inhibitor alone cannot reach a high level currently. Therefore, the development of novel anti-tumor immunomodulatory targets is of great significance for the treatment of tumors. Since AHR is a promising target, the development of corresponding inhibitors provides a new idea for novel anti-tumor therapies.

## CONTENT OF THE INVENTION

Therefore, according to a first aspect of the present disclosure, a first objective of the present disclosure is to provide a substituted pyrimidinyl hydrazide compound shown in the following formula I and an optical isomer, prodrug, or pharmaceutically-acceptable salt thereof:

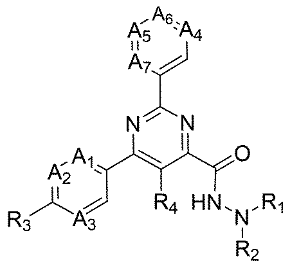

formula I

where

$A_1$, $A_2$, and $A_3$ each are independently $CR_A$ or N, the substituent $R_A$ is independently selected from hydrogen, deuterium, halogen, cyano, hydroxyl, amino, saturated or unsaturated $C_1$-$C_8$ alkyl, $C_3$-$C_8$ cycloalkyl, saturated or unsaturated $C_1$-$C_8$ alkyl substituted with halogen, hydroxyl, amino, or $R_C$, $C_3$-$C_8$ cycloalkyl substituted with halogen, hydroxyl, amino, or $R_C$, saturated or unsaturated $C_1$-$C_8$ alkoxy, $C_3$-$C_8$ cycloalkoxy, saturated or unsaturated $C_1$-$C_8$ alkoxy substituted with halogen, hydroxyl, amino, or $R_C$, $C_3$-$C_8$ cycloalkoxy substituted with halogen, hydroxyl, amino, or $R_C$, -$S(O)_nRc$, saturated or unsaturated substituted or unsubstituted $C_1$-$C_8$ acyl, and $NR_CR_D$,

$A_4$ is O, S, N, C, or a bond, and $A_5$, $A_6$, and $A_7$ each are independently O, S, N, or C;

when $A_4$, $A_5$, $A_6$, and $A_7$ are N or C, $A_4$, $A_5$, $A_6$, and $A_7$ each are independently substituted with $R_B$, $R_B$ is independently selected from hydrogen, deuterium, halogen, cyano, hydroxyl, -$S(O)_nRc$, saturated or unsaturated $C_1$-$C_8$ alkyl, $C_3$-$C_8$ cycloalkyl, saturated or unsaturated $C_1$-$C_8$ alkyl substituted with halogen, hydroxyl, amino, or $R_C$, $C_3$-$C_8$ cycloalkyl substituted with halogen, hydroxyl, amino, or $R_C$, saturated or unsaturated $C_1$-$C_8$ alkoxy, $C_3$-$C_8$ cycloalkoxy, saturated or unsaturated $C_1$-$C_8$ alkoxy substituted with halogen, hydroxyl, amino, or $R_C$, $C_3$-$C_8$ cycloalkoxy substituted with halogen, hydroxyl, amino, or $R_C$, saturated or unsaturated substituted or unsubstituted $C_1$-$C_8$ acyl, saturated or unsaturated substituted or unsubstituted $C_1$-$C_8$ alkoxycarbonyl, saturated or unsaturated substituted or unsubstituted $C_1$-$C_8$ alkylaminocarbonyl, and $NR_CR_D$,

the substituents $R_C$ and $R_D$ each are independently selected from hydrogen, halogen, cyano, hydroxyl, amino, saturated or unsaturated $C_1$-$C_6$ alkyl, $C_3$-$C_6$ cycloalkyl, saturated or unsaturated $C_1$-$C_6$ alkyl substituted with halogen, hydroxyl, -$S(O)_nR_E$, or $NR_FR_G$, $C_3$-$C_6$ cycloalkyl substituted with halogen, hydroxyl, -$S(O)_nR_E$, or $NR_FR_G$, saturated or unsaturated $C_1$-$C_6$ alkoxy, $C_3$-$C_6$ cycloalkoxy, saturated or unsaturated $C_1$-$C_6$ alkoxy substituted with halogen, hydroxyl, -$S(O)_nR_E$, or $NR_FR_G$, and $C_3$-$C_6$ cycloalkoxy substituted with halogen, hydroxyl, -$S(O)_nR_E$, or $NR_FR_G$, and

$R_E$, $R_F$, and $R_G$ each are independently selected from hydrogen, deuterium, halogen, cyano, hydroxyl, amino, saturated or unsaturated $C_1$-$C_6$ alkyl, $C_3$-$C_6$ cycloalkyl, saturated or unsaturated $C_1$-$C_6$ alkyl substituted with halogen, hydroxyl, or amino, $C_3$-$C_6$ cycloalkyl substituted with halogen, hydroxyl, or amino, saturated or unsaturated $C_1$-$C_6$ alkoxy, $C_3$-$C_6$ cycloalkoxy, saturated or unsaturated $C_1$-$C_6$ alkoxy substituted with halogen, hydroxyl, or amino, and $C_3$-$C_6$ cycloalkoxy substituted with halogen, hydroxyl, or amino;

$R_1$ and $R_2$ each are independently hydrogen, saturated or unsaturated substituted or unsubstituted $C_1$-$C_8$ alkyl, saturated or unsaturated substituted or unsubstituted $C_3$-$C_8$ cycloalkyl, -OC(=O)$C_{1-8}$ alkyl, -OC(=O)$C_3$-$C_8$ cycloalkyl, -C(=O)O$C_{1-8}$ alkyl, -C(=O)O$C_3$-$C_8$ cycloalkyl, -$S(O)_nRc$, saturated or unsaturated substituted or unsubstituted $C_1$-$C_8$ sulfonyl, saturated or unsaturated substituted or unsubstituted $C_1$-$C_8$ acyl, $C_6$-$C_{14}$ aryl, and 4 to 14-membered heterocycloalkyl or heteroaryl with 1 to 3 heteroatoms selected from N, O, and S, where the "substitution" refers to selective containing of 1 to 4 substituents selected from hydroxyl, halogen, cyano, sulfonyl, amino, -$S(O)_nRc$, $NR_CR_D$, saturated or unsaturated $C_1$-$C_8$ alkyl, saturated or unsaturated $C_3$-$C_8$ cycloalkyl, saturated or unsaturated $C_1$-$C_8$ alkoxy, saturated or unsaturated $C_3$-$C_8$ cycloalkoxy, -OC(=O)$C_{1-8}$ alkyl, -OC(=O)$C_3$-$C_8$ cycloalkyl, -C(=O)O$C_{1-8}$ alkyl, -C(=O)O$C_3$-$C_8$ cycloalkyl, saturated or unsaturated $C_1$-$C_8$ sulfonyl, saturated or unsaturated $C_1$-$C_8$ acyl, saturated or unsaturated $C_1$-$C_8$ alkoxycarbonyl, $C_6$-$C_{14}$ aryl, and 4 to 14-membered heterocycloalkyl or heteroaryl with 1 to 3 heteroatoms selected from N, O, and S; or

$R_1$ and $R_2$, together with N atoms attached thereto, produce substituted or unsubstituted 4 to 14-membered heterocycloalkyl with 1 to 3 heteroatoms selected from N, O, and S or substituted or unsubstituted 5 to 14-membered heteroaryl with 1 to 3 heteroatoms selected from N, O, and S, where the "substitution" refers to selective containing of 1

to 4 substituents selected from deuterium, hydroxyl, halogen, cyano, sulfonyl, amino, $-S(O)_nRc$, $NR_CR_D$, $C_1$-$C_8$ alkyl, $C_3$-$C_8$ cycloalkyl, $C_1$-$C_8$ alkoxy, $C_3$-$C_8$ cycloalkoxy, $-OC(=O)C_{1-8}$ alkyl, $-OC(=O)C_3$-$C_8$ cycloalkyl, $-C(=O)OC_{1-8}$ alkyl, $-C(=O)OC_3$-$C_8$ cycloalkyl, $C_1$-$C_8$ alkyl substituted with halogen, hydroxyl, amino, or $R_H$, $C_3$-$C_8$ cycloalkyl, $C_1$-$C_8$ alkoxy, and $C_3$-$C_8$ cycloalkoxy, where $R_H$ is selected from hydrogen, deuterium, halogen, cyano, hydroxyl, amino, saturated or unsaturated $C_1$-$C_6$ alkyl, $C_3$-$C_6$ cycloalkyl, $-S(O)_nRc$, $NR_CR_D$, saturated or unsaturated $C_1$-$C_6$ alkyl substituted with deuterium, halogen, hydroxyl, or amino, $C_3$-$C_6$ cycloalkyl substituted with deuterium, halogen, hydroxyl, or amino, saturated or unsaturated $C_1$-$C_6$ alkoxy, $C_3$-$C_6$ cycloalkoxy, saturated or unsaturated $C_1$-$C_6$ alkoxy substituted with deuterium, halogen, hydroxyl, or amino, and $C_3$-$C_6$ cycloalkoxy substituted with deuterium, halogen, hydroxyl, or amino;

$R_3$ is halogen, hydroxyl, $-S(O)_nRc$, $NR_CR_D$, saturated or unsaturated $C_1$-$C_8$ alkyl, $C_3$-$C_8$ cycloalkyl, $C_1$-$C_8$ alkoxy, $C_3$-$C_8$ cycloalkoxy, saturated or unsaturated $C_1$-$C_8$ alkyl substituted with halogen, hydroxyl, amino, or $R_H$, $C_3$-$C_8$ cycloalkyl, $C_1$-$C_8$ alkoxy, and $C_3$-$C_8$ cycloalkoxy, where the substituent $R_H$ is selected from hydrogen, deuterium, halogen, cyano, hydroxyl, amino, saturated or unsaturated $C_1$-$C_6$ alkyl, $C_3$-$C_6$ cycloalkyl, saturated or unsaturated $C_1$-$C_6$ alkyl substituted with halogen, hydroxyl, or amino, $C_3$-$C_6$ cycloalkyl substituted with halogen, hydroxyl, or amino, saturated or unsaturated $C_1$-$C_6$ alkoxy, $C_3$-$C_6$ cycloalkoxy, saturated or unsaturated $C_1$-$C_6$ alkoxy substituted with halogen, hydroxyl, or amino, and $C_3$-$C_6$ cycloalkoxy substituted with halogen, hydroxyl, or amino;

R4 is hydrogen or deuterium; and

n is an integer of 0, 1, or 2.

Preferably, $A_1$ and $A_3$ each are $CR_A$, $A_2$ is N, and the substituent $R_A$ is independently selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, amino, or $R_C$, $C_3$-$C_6$ cycloalkyl substituted with halogen, hydroxyl, amino, or $R_C$, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, amino, or $R_C$, $C_3$-$C_6$ cycloalkoxy substituted with halogen, hydroxyl, amino, or $R_C$, $-S(O)_nRc$, saturated or unsaturated substituted or unsubstituted $C_1$-$C_3$ acyl, and $NR_CR_D$.

More preferably, $A_1$ and $A_3$ each are $CR_A$, $A_2$ is N, and the substituent $R_A$ is hydrogen.

Preferably, $A_4$ is N, C, or a bond, $A_5$, $A_6$, and $A_7$ each are independently N or C,

$A_4$, $A_5$, $A_6$, and $A_7$ each are independently linked to the substituent $R_B$, and the substituent $R_B$ is independently selected from hydrogen, halogen, cyano, hydroxyl, amino, $-S(O)_nRc$, saturated or unsaturated $C_1$-$C_3$ alkyl, $C_3$-$C_6$ cycloalkyl, saturated or unsaturated $C_1$-$C_3$ alkyl substituted with halogen, hydroxyl, amino, or $R_C$, $C_3$-$C_6$ cycloalkyl substituted with halogen, hydroxyl, amino, or $R_C$, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, amino, or $R_C$, $C_3$-$C_6$ cycloalkoxy substituted with halogen, hydroxyl, amino, or R', saturated or unsaturated substituted or unsubstituted $C_1$-$C_3$ acyl, saturated or unsaturated substituted or unsubstituted $C_1$-$C_3$ alkoxycarbonyl, and $NR_CR_D$.

Preferably, the substituents $R_C$ and $R_D$ each are independently selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, $-S(O)_nR_E$, or $NR_FR_G$, $C_3$-$C_6$ cycloalkyl substituted with halogen, hydroxyl, $-S(O)_nR_E$, or $NR_FR_G$, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, $-S(O)_nR_E$, or $NR_FR_G$, and $C_3$-$C_6$ cycloalkoxy substituted with halogen, hydroxyl, $-S(O)_nR_E$, or $NR_FR_G$.

Preferably, $R_E$, $R_F$, and $R_G$ each are independently selected from hydrogen, deuterium, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

Preferably, $A_7$ is C.

Preferably, $R_1$ and $R_2$ each are independently hydrogen, saturated or unsaturated substituted or unsubstituted $C_1$-$C_6$ alkyl, saturated or unsaturated substituted or unsubstituted $C_3$-$C_6$ cycloalkyl, $-OC(=O)C_{1-6}$ alkyl, $-OC(=O)C_3$-$C_6$ cycloalkyl, $-C(=O)OC_{1-6}$ alkyl, $-C(=O)OC_3$-$C_6$ cycloalkyl, saturated or unsaturated substituted or unsubstituted $C_1$-$C_6$ alkoxy, saturated or unsaturated substituted or unsubstituted $C_3$-$C_6$ cycloalkoxy, $-S(O)_nRc$, $NR_CR_D$, saturated or unsaturated substituted or unsubstituted $C_1$-$C_6$ sulfonyl, saturated or unsaturated substituted or unsubstituted $C_1$-$C_6$ acyl, $C_6$-$C_{10}$ aryl, and 5 to 10-membered heteroaryl with 1 to 3 heteroatoms selected from N, O, and S, where the "substitution" refers to selective containing of 1 to 4 substituents selected from hydroxyl, halogen, cyano, sulfonyl, amino, $-S(O)_nRc$, $NR_CR_D$, saturated or unsaturated $C_1$-$C_6$ alkyl, saturated or unsaturated $C_3$-$C_6$ cycloalkyl, $-OC(=O)C_{1-6}$ alkyl, $-OC(=O)C_3$-$C_6$ cycloalkyl, $-C(=O)OC_{1-6}$ alkyl, $-C(=O)OC_3$-$C_6$ cycloalkyl, saturated or unsaturated $C_1$-$C_6$ sulfonyl, saturated or unsaturated $C_1$-$C_6$ acyl, $C_6$-$C_{10}$ aryl, and 6 to 10-membered heterocycloalkyl or heteroaryl with 1 to 3 heteroatoms selected from N, O, and S; or

$R_1$ and $R_2$, together with N atoms attached thereto, produce substituted or unsubstituted 4 to 10-membered heterocycloalkyl with 1 to 3 heteroatoms selected from N, O, and S or substituted or unsubstituted 5 to 10-membered heteroaryl

with 1 to 3 heteroatoms selected from N, O, and S, where the "substitution" refers to selective containing of 1 to 4 substituents selected from hydroxyl, halogen, cyano, sulfonyl, amino, $-S(O)_nRc$, $NR_CR_D$, $C_1$-$C_6$ alkyl, $C_3$-$C_6$ cycloalkyl, $C_1$-$C_6$ alkoxy, $C_3$-$C_6$ cycloalkoxy, $-OC(=O)C_{1-6}$ alkyl, $-OC(=O)C_3$-$C_6$ cycloalkyl, $-C(=O)OC_{1-6}$ alkyl, $-C(=O)OC_3$-$C_6$ cycloalkyl, $C_1$-$C_6$ alkyl substituted with halogen, hydroxyl, amino, or $R_E$, $C_3$-$C_6$ cycloalkyl, $C_1$-$C_6$ alkoxy, and $C_3$-$C_6$ cycloalkoxy, where $R_E$ is selected from hydrogen, halogen, cyano, hydroxyl, amino, saturated or unsaturated $C_1$-$C_6$ alkyl, $C_3$-$C_6$ cycloalkyl, $-S(O)_nRc$, $NR_CR_D$, saturated or unsaturated $C_1$-$C_6$ alkyl substituted with deuterium, halogen, hydroxyl, or amino, $C_3$-$C_6$ cycloalkyl substituted with deuterium, halogen, hydroxyl, or amino, saturated or unsaturated $C_1$-$C_6$ alkoxy, $C_3$-$C_6$ cycloalkoxy, saturated or unsaturated $C_1$-$C_6$ alkoxy substituted with deuterium, halogen, hydroxyl, or amino, and $C_3$-$C_6$ cycloalkoxy substituted with deuterium, halogen, hydroxyl, or amino.

Preferably, $R_3$ is halogen, $-S(O)_nRc$, $NR_CR_D$, saturated or unsaturated $C_1$-$C_6$ alkyl, $C_3$-$C_6$ cycloalkyl, $C_1$-$C_6$ alkoxy, $C_3$-$C_6$ cycloalkoxy, saturated or unsaturated $C_1$-$C_6$ alkyl substituted with halogen, hydroxyl, amino, or $R_H$, $C_3$-$C_6$ cycloalkyl, $C_1$-$C_6$ alkoxy, and $C_3$-$C_6$ cycloalkoxy, where the substituent $R_H$ is selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

More preferably, $R_1$ and $R_2$ each are independently hydrogen, saturated or unsaturated substituted or unsubstituted $C_1$-$C_3$ alkyl, saturated or unsaturated substituted or unsubstituted $C_3$-$C_6$ cycloalkyl, $-OC(=O)C_{1-3}$ alkyl, $-OC(=O)C_3$-$C_6$ cycloalkyl, $-C(=O)OC_{1-3}$ alkyl, $-C(=O)OC_3$-$C_6$ cycloalkyl, $-S(O)_nRc$, $NR_CR_D$, saturated or unsaturated substituted or unsubstituted $C_1$-$C_3$ sulfonyl, saturated or unsaturated substituted or unsubstituted $C_1$-$C_3$ acyl, $C_6$-$C_{10}$ aryl, and 6 to 8-membered heteroaryl with 1 to 3 heteroatoms selected from N, O, and S, where the "substitution" refers to selective containing of 1 to 4 substituents selected from hydroxyl, halogen, cyano, sulfonyl, amino, $-S(O)_nRc$, $NR_CR_D$, $-OC(=O)C_{1-3}$ alkyl, $-OC(=O)C_3$-$C_6$ cycloalkyl, $-C(=O)OC_{1-3}$ alkyl, $-C(=O)OC_3$-$C_6$ cycloalkyl, saturated or unsaturated $C_1$-$C_3$ alkyl, saturated or unsaturated $C_3$-$C_6$ cycloalkyl, saturated or unsaturated $C_1$-$C_3$ sulfonyl, saturated or unsaturated $C_1$-$C_3$ acyl, $C_6$-$C_{10}$ aryl, and 6 to 8-membered heteroaryl with 1 to 3 heteroatoms selected from N, O, and S; or

$R_1$ and $R_2$, together with N atoms attached thereto, produce substituted or unsubstituted 5 to 10-membered heterocycloalkyl with 1 to 3 heteroatoms selected from N, O, and S or substituted or unsubstituted 5 to 10-membered heteroaryl with 1 to 3 heteroatoms selected from N, O, and S, where the "substitution" refers to selective containing of 1 to 4 substituents selected from hydroxyl, halogen, cyano, sulfonyl, amino, $-S(O)_nRc$, $NR_CR_D$, $C_1$-$C_3$ alkyl, $C_3$-$C_6$ cycloalkyl, $C_1$-$C_3$ alkoxy, $C_3$-$C_6$ cycloalkoxy, $-OC(=O)C_{1-3}$ alkyl, $-OC(=O)C_3$-$C_6$ cycloalkyl, $-C(=O)OC_{1-3}$ alkyl, $-C(=O)OC_3$-$C_6$ cycloalkyl, $C_1$-$C_3$ alkyl substituted with halogen, hydroxyl, amino, or $R_H$, $C_3$-$C_6$ cycloalkyl, $C_1$-$C_3$ alkoxy, and $C_3$-$C_6$ cycloalkoxy, where $R_H$ is selected from hydrogen, halogen, cyano, hydroxyl, amino, saturated or unsaturated $C_1$-$C_3$ alkyl, $C_3$-$C_6$ cycloalkyl, $-S(O)_nRc$, $NR_CR_D$, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

More preferably, $R_3$ is halogen, $-S(O)_nRc$, $NR_CR_D$, saturated or unsaturated $C_1$-$C_3$ alkyl, $C_3$-$C_6$ cycloalkyl, $C_1$-$C_3$ alkoxy, $C_3$-$C_6$ cycloalkoxy, saturated or unsaturated $C_1$-$C_3$ alkyl substituted with halogen, hydroxyl, amino, or $R_F$, $C_3$-$C_6$ cycloalkyl, $C_1$-$C_3$ alkoxy, and $C_3$-$C_6$ cycloalkoxy, where the substituent $R_H$ is selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

More preferably, a structure formed by $R_1$ and $R_2$, together with N atoms attached thereto, is selected from the following groups:

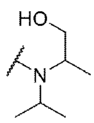 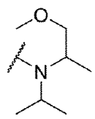 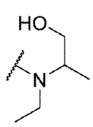 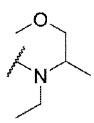 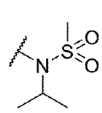 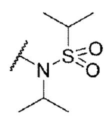 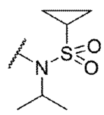

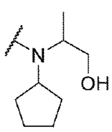 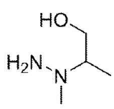 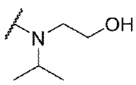 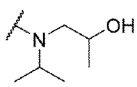 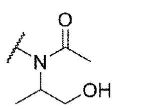 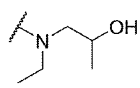 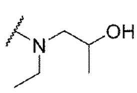

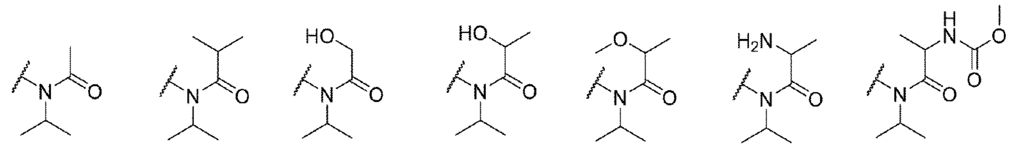

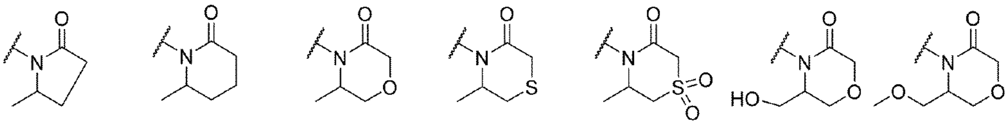

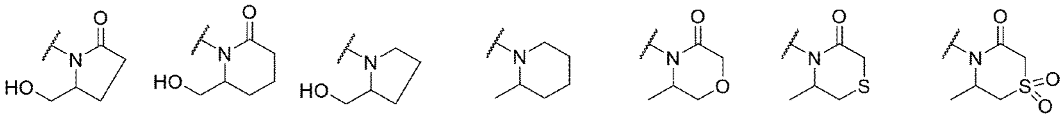

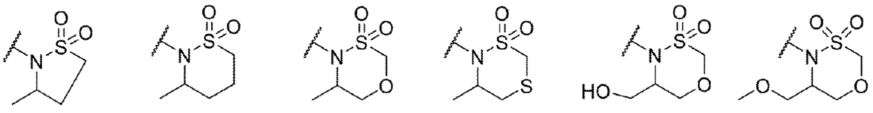

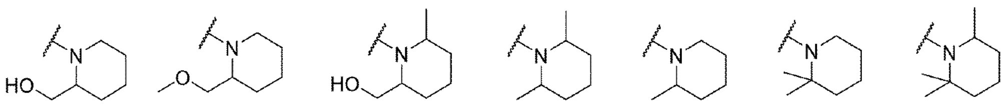

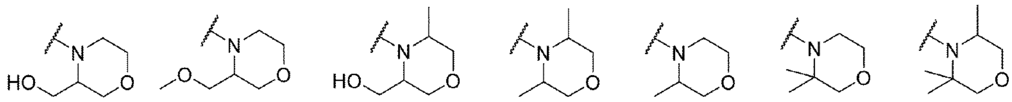

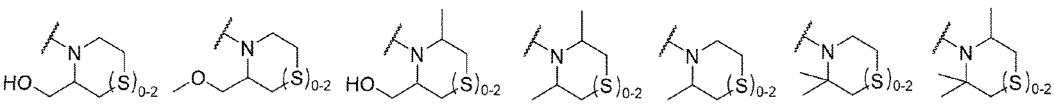

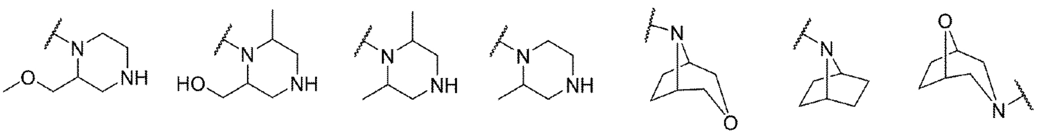

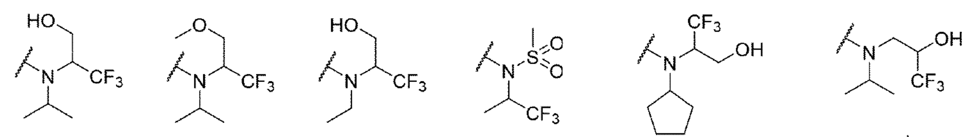

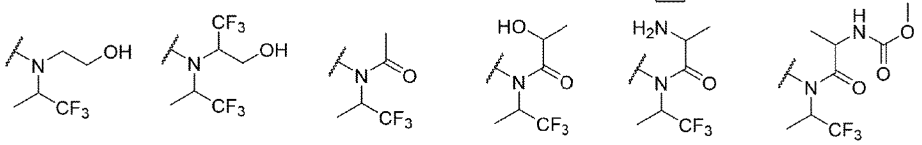

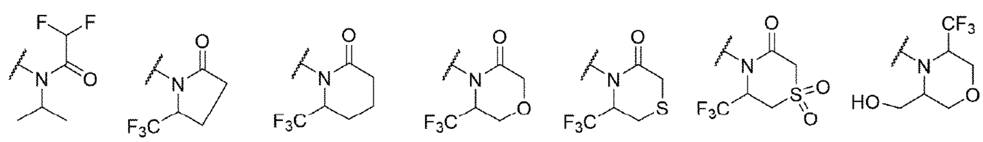

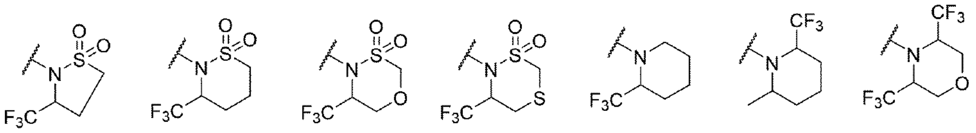

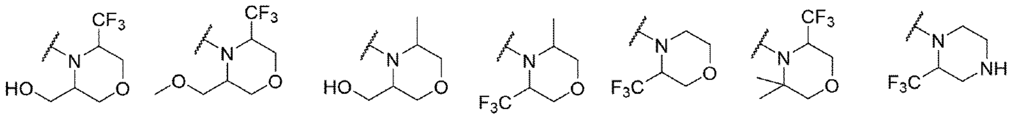

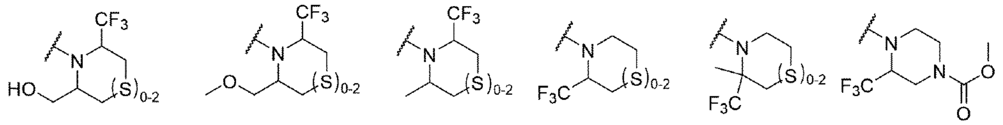

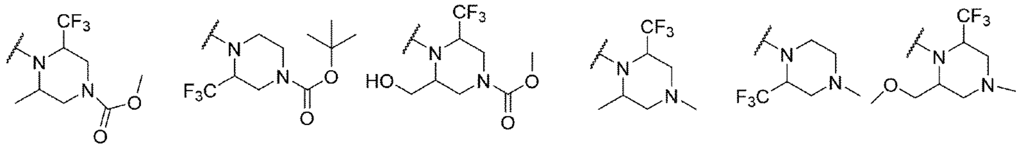

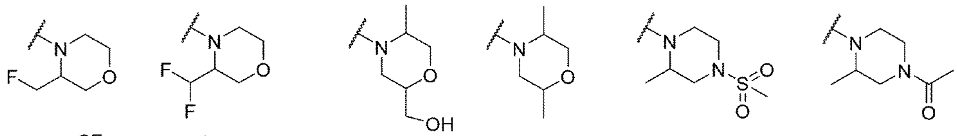

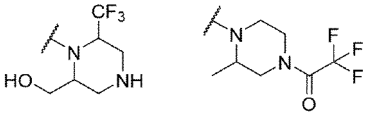

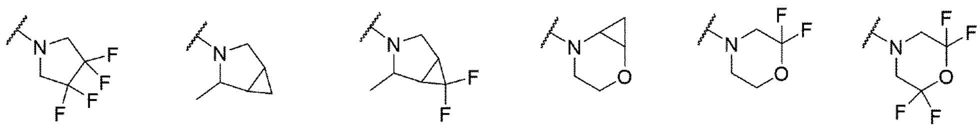

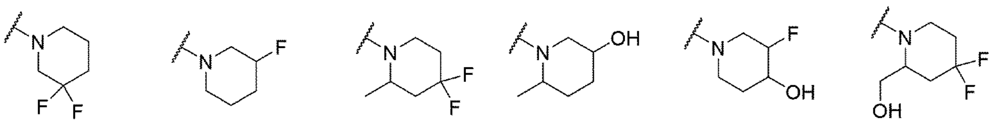

More preferably, the substituted pyrimidinyl hydrazide compound or the optical isomer, prodrug, or pharmaceutically-acceptable salt thereof according to the present disclosure is selected from the following compounds:

| No. | Structural formula | No. | Structural formula | No. | Structural formula |
|---|---|---|---|---|---|
| 1 | 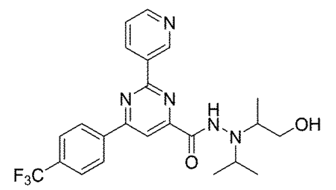 | 2 | 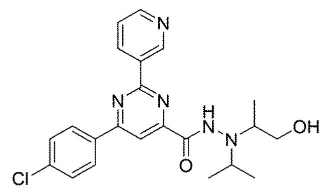 | 3 | 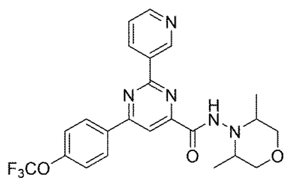 |

(continued)

| No. | Structural formula | No. | Structural formula | No. | Structural formula |
|---|---|---|---|---|---|
| 4 | 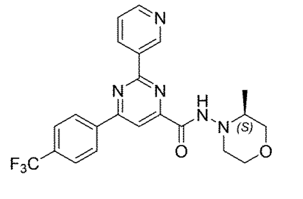 | 5 | 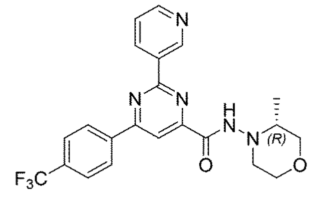 | 6 | 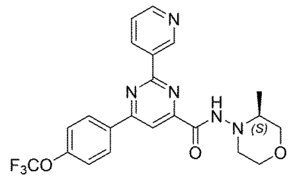 |
| 7 | 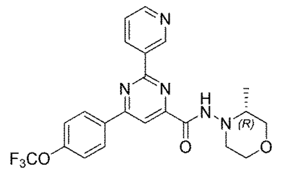 | 8 | 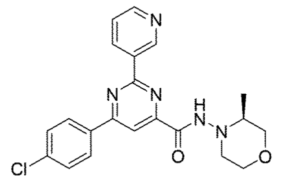 | 9 | 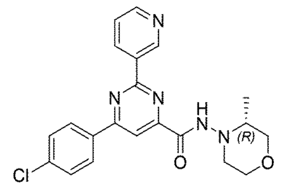 |
| 10 | 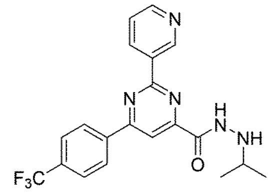 | 11 | 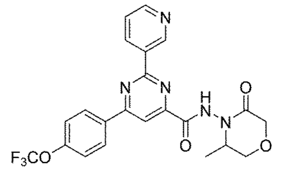 | 12 | 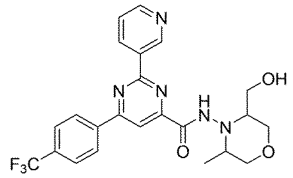 |
| 13 | 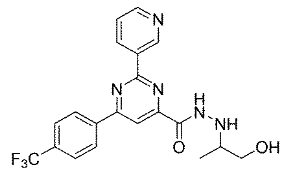 | 14 | 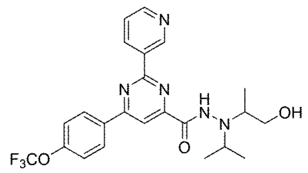 | 15 | 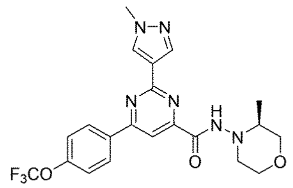 |
| 16 | 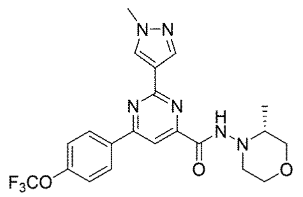 | 17 | 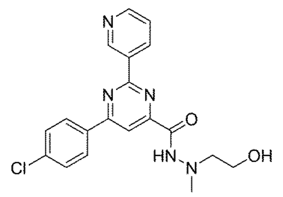 | 18 | 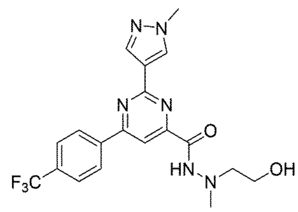 |
| 19 | 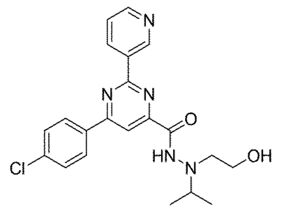 | 20 | 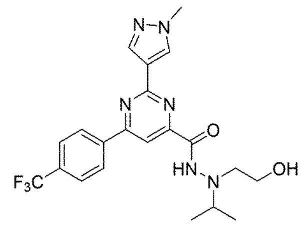 | 21 | 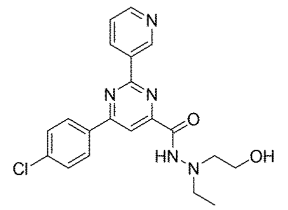 |
| 22 | 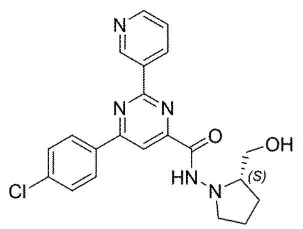 | 23 | 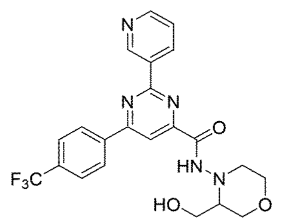 | 24 | 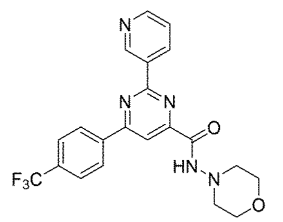 |

(continued)

| No. | Structural formula | No. | Structural formula | No. | Structural formula |
|---|---|---|---|---|---|
| 25 | 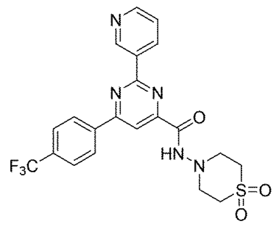 | 26 | 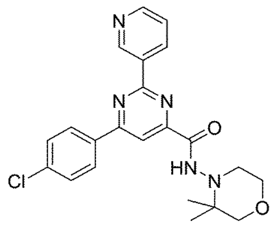 | 27 | 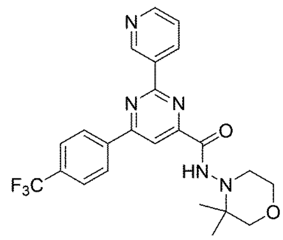 |
| 28 | 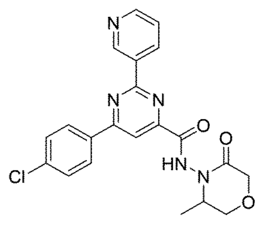 | 29 | 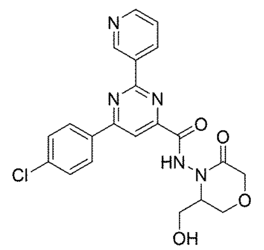 | 30 | 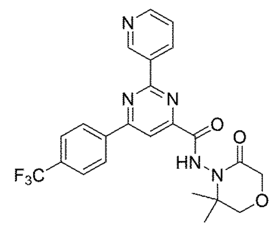 |
| 31 | 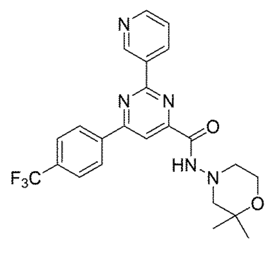 | 32 | 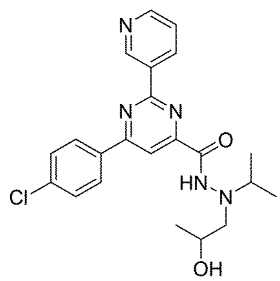 | 33 | 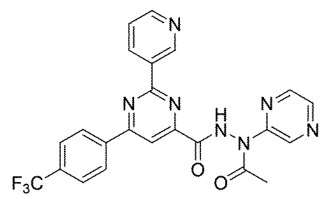 |
| 34 | 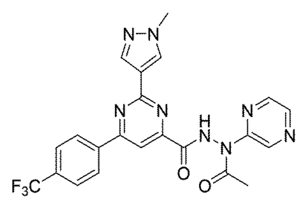 | 35 | 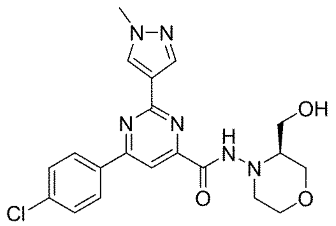 | 36 | 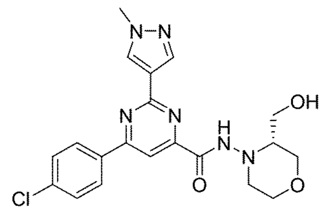 |
| 37 | 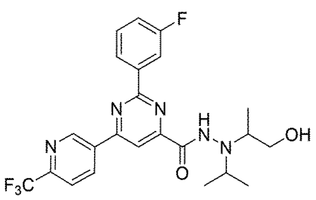 | 38 | 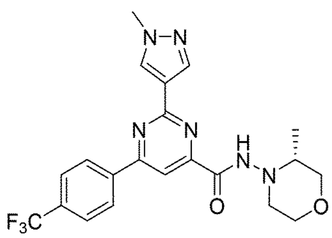 | 39 | 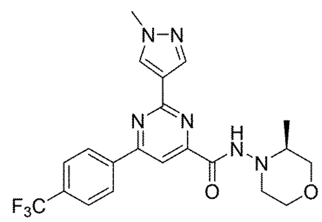 |
| 40 | 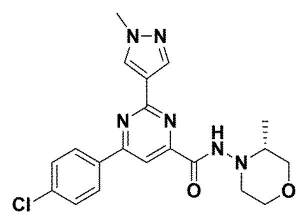 | 41 | 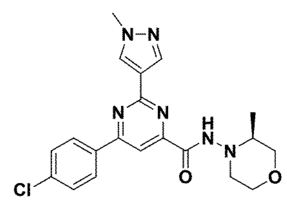 | 42 | 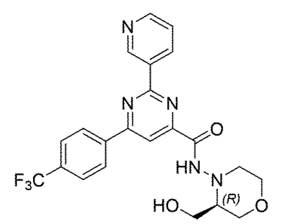 |

(continued)

| No. | Structural formula | No. | Structural formula | No. | Structural formula |
|---|---|---|---|---|---|
| 43 | 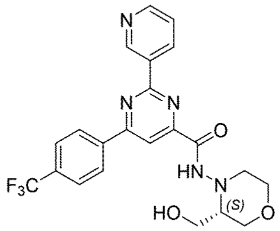 | 44 | 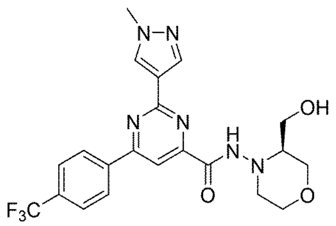 | 45 | 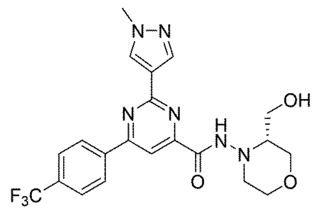 |
| 46 | 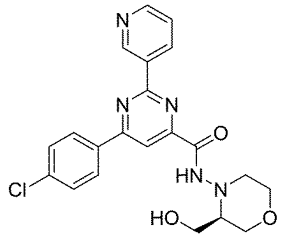 | 47 | 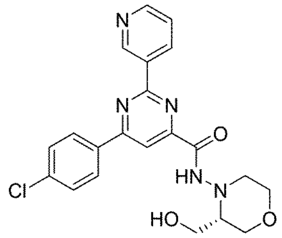 | 48 | 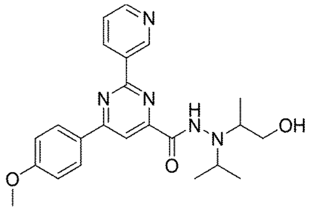 |
| 49 | 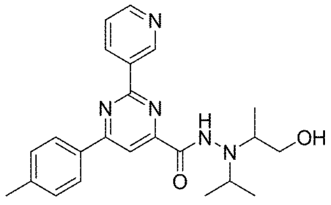 | 50 | 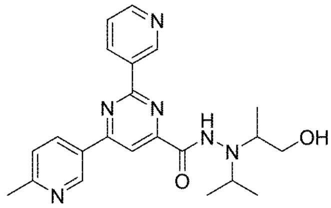 | 51 | 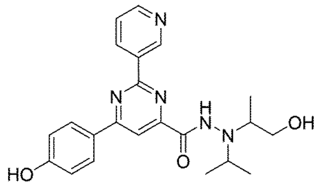 |
| 52 | 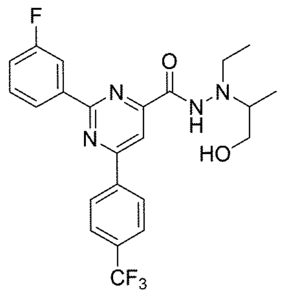 | 53 | 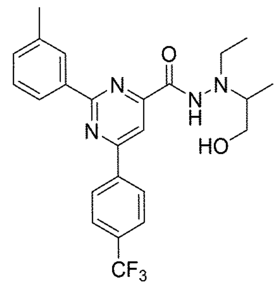 | 54 | 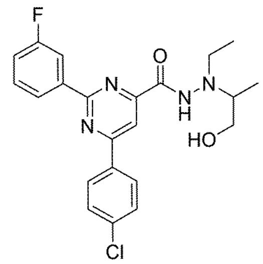 |
| 55 | 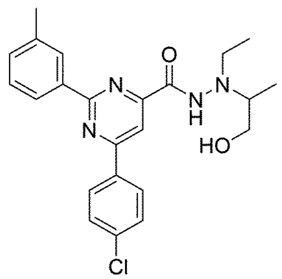 | 56 | 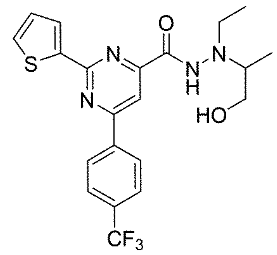 | 57 | 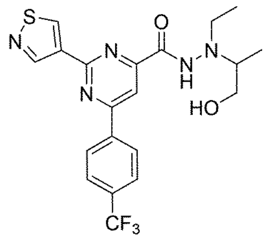 |
| 58 | 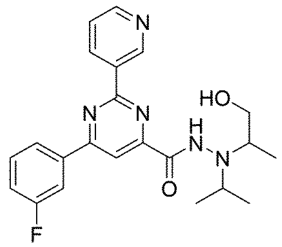 | 59 | 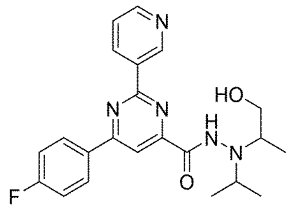 | 60 | 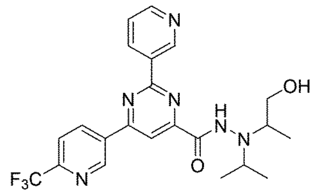 |

(continued)

| No. | Structural formula | No. | Structural formula | No. | Structural formula |
|---|---|---|---|---|---|
| 61 | 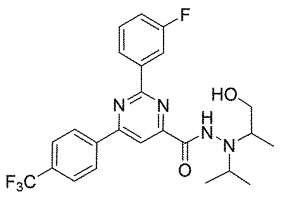 | 62 | 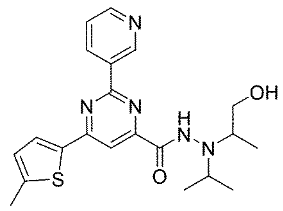 | 63 | 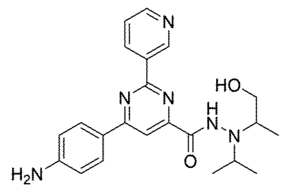 |
| 64 | 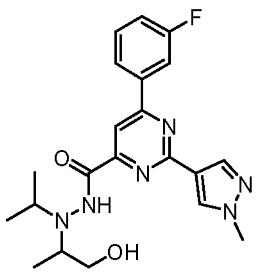 | 65 | 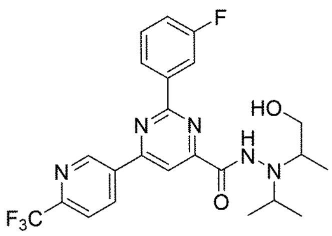 | 66 | 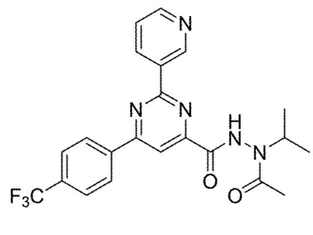 |
| 67 | 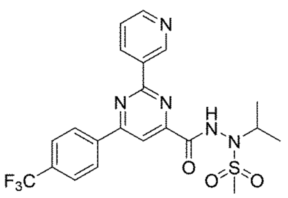 | 68 | 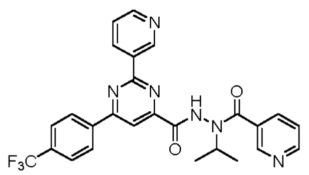 | | |

According to a second aspect of the present disclosure, a second objective of the present disclosure is to provide a pharmaceutical composition including a therapeutically-effective amount of the substituted pyrimidinyl hydrazide compound and the optical isomer, prodrug, or pharmaceutically-acceptable salt thereof according to the present disclosure and a pharmaceutically-acceptable excipient or carrier.

According to a third aspect of the present disclosure, a third objective of the present disclosure is to provide a use of the substituted pyrimidinyl hydrazide compound and the optical isomer, prodrug, or pharmaceutically-acceptable salt thereof according to the present disclosure in preparation of an AHR disorder inhibitor.

According to a fourth aspect of the present disclosure, a fourth objective of the present disclosure is to provide a use of the substituted pyrimidinyl hydrazide compound and the optical isomer, prodrug, or pharmaceutically-acceptable salt thereof according to the present disclosure in preparation of a drug for treating a tumor associated with an AHR disorder.

According to a fifth aspect of the present disclosure, a fifth objective of the present disclosure is to provide a method for treating a tumor associated with an AHR disorder, including: administering an effective amount of the substituted pyrimidinyl hydrazide compound and the optical isomer, prodrug, or pharmaceutically-acceptable salt thereof according to the present disclosure or the pharmaceutical composition according to the present disclosure to a subject in need.

According to a sixth aspect of the present disclosure, a sixth objective of the present disclosure is to provide a preparation method of the substituted pyrimidinyl hydrazide compound and the optical isomer, prodrug, or pharmaceutically-acceptable salt thereof according to the present disclosure, where the preparation method is shown in the following reaction equation 1:

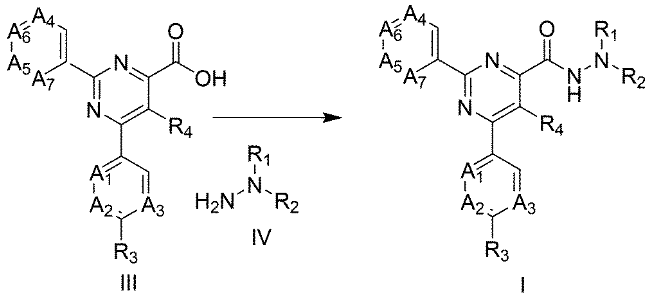

reaction equation 1;

the preparation method includes: allowing a carboxylic acid compound shown in the formula III to undergo a condensation reaction with a hydrazine compound shown in the formula IV to obtain the substituted pyrimidinyl hydrazide compound shown in the formula I;
the carboxylic acid compound shown in the formula III is prepared according to one of the following processes:

process A

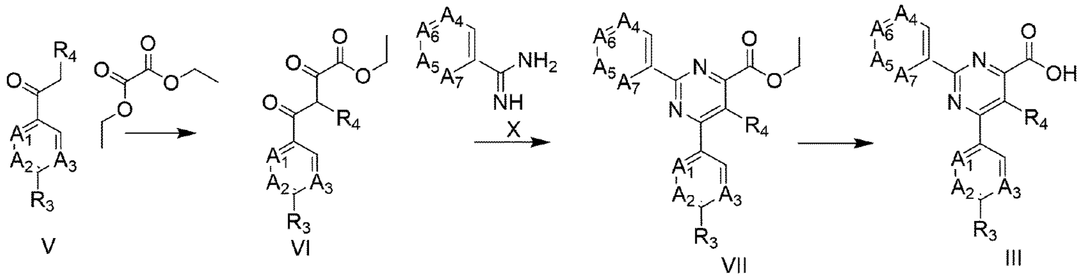

1) allowing a methyl carbonyl compound shown in the formula V to react with dimethyl oxalate to produce a compound shown in the formula VI;
2) allowing the compound shown in the formula VI to undergo a cyclization reaction with a hydrazine compound shown in the formula X to produce a pyrimidine compound shown in the formula VII; and
3) hydrolyzing the pyrimidine compound shown in the formula VII to obtain the carboxylic acid compound shown in the formula III;

process B

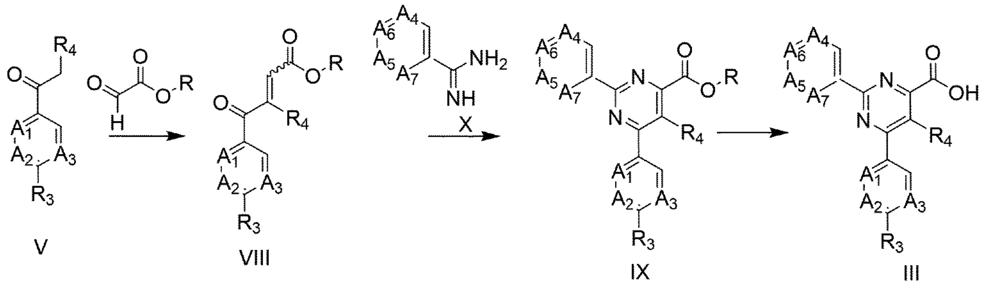

1) allowing the methyl carbonyl compound shown in the formula V to react with a monoalkyl oxalate

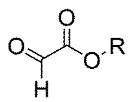

to produce a compound shown in the formula VIII, where the substituent R is methyl, ethyl, isopropyl, or tert-butyl;

2) allowing the compound shown in the formula VIII to undergo a cyclization reaction with the hydrazine compound shown in the formula X to produce a pyrimidine compound shown in the formula IX; and
3) hydrolyzing the pyrimidine compound shown in the formula IX to remove the substituent R to obtain the carboxylic acid compound shown in the formula III;

process C

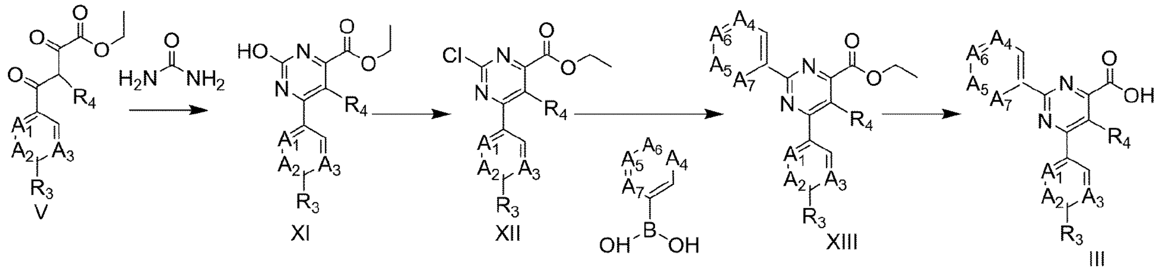
V XI XII XIII III

1) allowing the methyl carbonyl compound shown in the formula V to undergo a cyclization reaction with urea to produce a compound shown in the formula XI;
2) allowing the compound shown in the formula XI to react with a chlorination reagent including phosphorus oxychloride, phosphorus pentachloride, or thionyl chloride to produce a chlorinated compound shown in the formula XII;
3) allowing the chlorinated compound shown in the formula XII to react with a boric acid compound

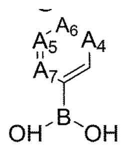 ,

, or a corresponding borate, or a trialkyl tin compound to produce a compound shown in the formula XIII; and
4) conducting ethyl removal for the compound shown in the formula XIII to generate a carboxylic acid group to obtain the compound shown in the formula III;

process D

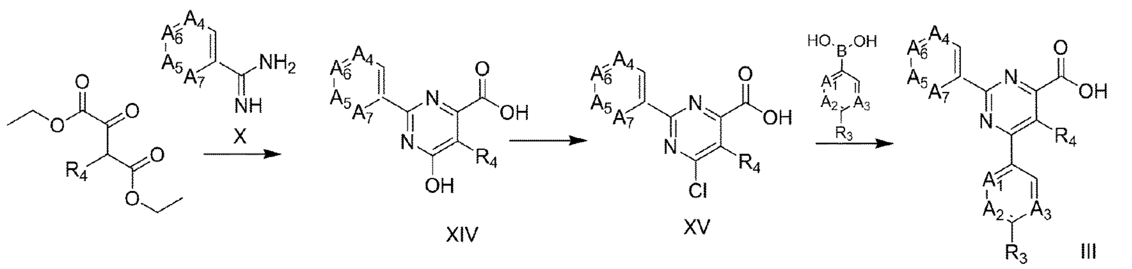

XIV XV III

1) allowing a carbonate compound

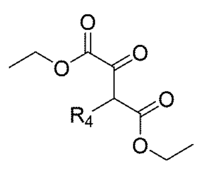

to undergo a cyclization reaction with the hydrazine compound shown in the formula X to produce a compound shown in the formula XIV;
2) allowing the compound shown in the formula XIV to react with a chlorination reagent including phosphorus oxychloride, phosphorus pentachloride, or thionyl chloride to produce a chlorinated compound shown in the

formula XV; and

3) allowing the chlorinated compound shown in the formula XV to react with a boric acid compound

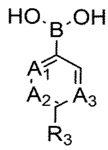

,

or a corresponding borate, or a trialkyl tin compound to obtain the compound shown in the formula III;

process E

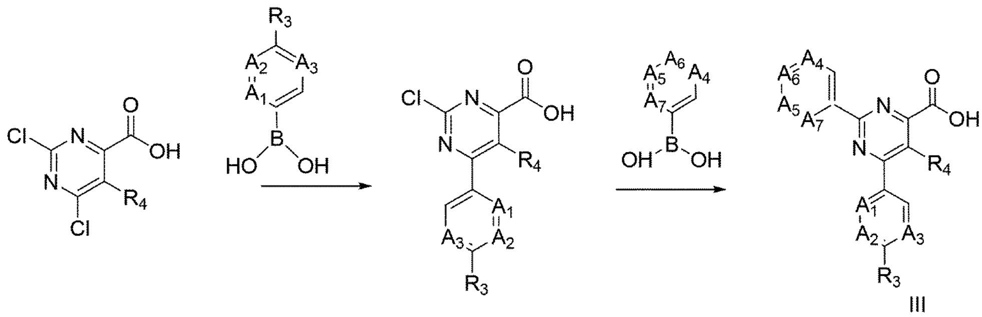

III

1) allowing a pyrimidine compound

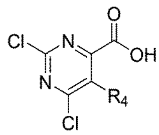

to react with a boric acid compound

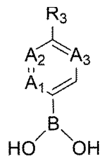

,

or a corresponding borate, or a trialkyl tin compound, and conducting separation and purification to obtain a compound

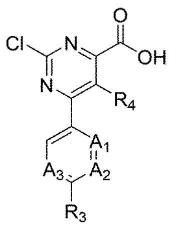

;

and

2) allowing the compound

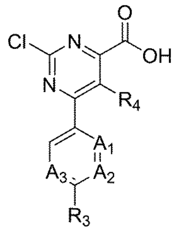

to react with the boric acid compound

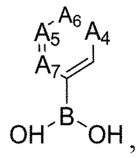

or a corresponding borate, or a trialkyl tin compound to obtain the compound shown in the formula III;

process F

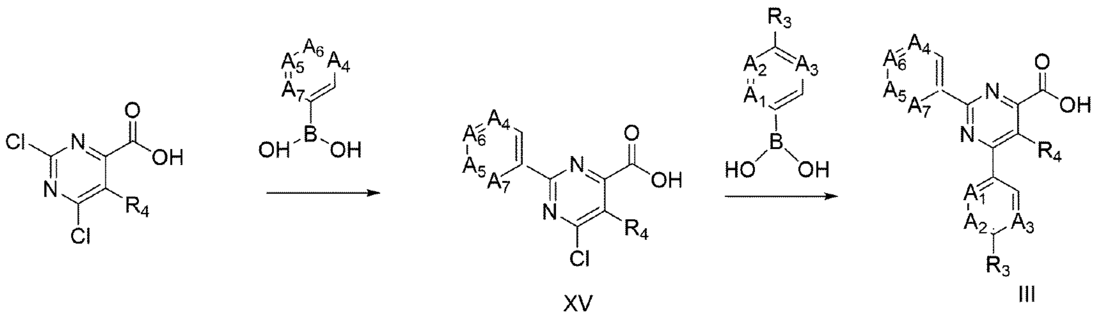

1) allowing the pyrimidine compound

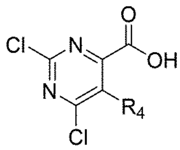

to react with the boric acid compound

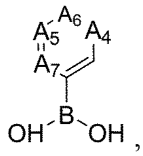

or a corresponding borate, or a trialkyl tin compound, and conducting separation and purification to obtain a compound shown in the formula XV; and

2) allowing the compound shown in the formula XV to react with the boric acid compound

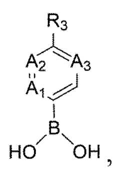

or a corresponding borate, or a trialkyl tin compound to obtain the compound shown in the formula III; and

the hydrazine compound shown in the formula IV of the present disclosure is synthesized according to the following processes:

process G

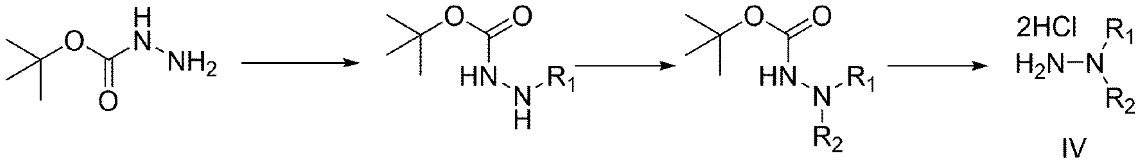

IV

1) dissolving $BocNHNH_2$ (tert-butoxycarbonyl hydrazide) in an alcohol solvent including methanol or ethanol, adding a ketone compound ($R_1(C=O)R_1$) or an aldehyde compound ($R_1(C=O)H$) to allow a reaction, adding sodium borohydride to allow hydrogenation reduction, and conducting purification and separation to produce a compound

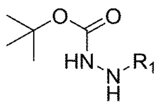

;

2) dissolving the compound

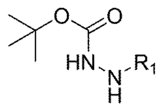

in an amine solvent including N,N-diisopropylyelethylamine (DIEA), and adding a ketone compound ($R_2(C=O)R_2$), an aldehyde compound ($R_2(C=O)H$), or a corresponding $R_2$-substituted epoxy compound to allow a reaction to obtain a compound

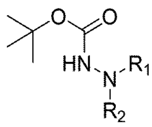

;

and

3) dissolving the compound

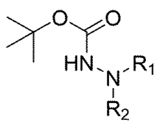

in an alcohol solvent including methanol or ethanol, and adding an excess amount of a solution of HCl in methanol to obtain a hydrochloride of the compound shown in the formula IV; and

process H

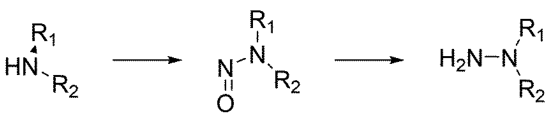

IV

1) dissolving a substituted amino compound ($HNR_1R_2$) in a solvent, adding a nitrosation reagent selected from tert-butyl nitrite and isobutyl nitrite, heating for reflux, and conducting vacuum concentration to obtain a nitrosamino compound as an intermediate ($R_1N(R_2)$-N=O); and

2) dissolving the nitrosamino compound ($R_1N(R_2)$-N=O) in a solvent, and adding a hydrogenation reagent including lithium aluminum hydride (LAH) to allow a hydrogenation reaction to obtain the compound shown in the formula IV.

## SPECIFIC IMPLEMENTATIONS

The present disclosure is described in detail below. Before the present disclosure is described, it should be understood that the terms used in the specification and the appended claims shall not be construed as being confined to general and dictionary meanings, and shall be interpreted in accordance with the meanings and concepts corresponding to the technical aspects of the present disclosure on the basis of the principle of allowing the inventors to properly define the terms for optimal explanations. Therefore, the descriptions presented here are only preferred examples for the purpose of illustration and are not intended to limit the scope of the present disclosure. Therefore, it should be understood that, without departing from the spirit and scope of the present disclosure, other equivalent or improved manners may be obtained accordingly.

When a range of values is listed, every value and sub-range within this range should be encompassed. For example, "$C_1$ to $C_8$" is intended to encompass $C_1$, $C_2$, $C_3$, $C_4$, $C_5$, $C_6$, $C_7$, $C_8$, $C_{1-8}$, $C_{1-7}$, $C_{1-6}$, $C_{1-5}$, $C_{1-4}$, $C_{1-3}$, $C_{1-2}$, $C_{2-8}$, $C_{2-7}$, $C_{2-6}$, $C_{2-5}$, $C_{2-4}$, $C_{2-3}$, $C_{3-8}$, $C_{3-7}$, $C_{3-6}$, $C_{3-5}$, $C_{3-4}$, $C_{4-8}$, $C_{4-7}$, $C_{4-6}$, $C_{4-5}$, $C_{5-8}$, $C_{5-7}$, $C_{5-6}$, $C_{6-8}$, $C_{6-7}$, and $C_{7-8}$.

Definitions

"Alkyl" refers to linear or branched saturated hydrocarbyl with 1 to 8 carbon atoms ("$C_{1-8}$ alkyl"). In some embodiments, alkyl has 1 to 7 carbon atoms ("$C_{1-7}$ alkyl"). In some embodiments, alkyl has 1 to 6 carbon atoms ("$C_{1-6}$ alkyl"). In some embodiments, alkyl has 1 to 5 carbon atoms ("$C_{1-5}$ alkyl"). In some embodiments, alkyl has 1 to 4 carbon atoms ("$C_{1-4}$ alkyl"). In some embodiments, alkyl has 1 to 3 carbon atoms ("$C_{1-3}$ alkyl"). In some embodiments, alkyl has 1 to 2 carbon atoms ("$C_{1-2}$ alkyl"). In some embodiments, alkyl has 1 carbon atom ("$C_1$ alkyl"). In some embodiments, alkyl has 1 to 6 carbon atoms ("$C_{1-6}$ alkyl"). Examples of $C_{1-6}$ alkyl include methyl ($C_1$), ethyl ($C_2$), propyl ($C_3$) (such as n-propyl and isopropyl), butyl ($C_4$) (such as n-butyl, tert-butyl, sec-butyl, and isobutyl), pentyl ($C_5$) (such as n-pentyl, 3-pentyl, neopentyl, 3-methyl-2-butyl, and tert-pentyl), and hexyl ($C_6$) (such as n-hexyl). Other examples of alkyl include n-heptyl ($C_7$), n-octyl ($C_8$), or the like. Unless otherwise specified, each example of alkyl is independently unsubstituted ("unsubstituted alkyl") or substituted with one or more substituents (for example, halogen, such as F) ("substituted alkyl"). In some embodiments, alkyl is unsubstituted $C_{1-8}$ alkyl (for example, unsubstituted $C_1$ alkyl, such as $-CH_3$). In some embodiments, alkyl is substituted $C_{1-8}$ alkyl (for example, substituted $C_1$ alkyl, such as $-CF_3$).

"Alkoxy" represents monovalent -O-alkyl, where an alkyl moiety has a specified number of carbon atoms. The alkoxy in the present disclosure generally includes 1 to 6 carbon atoms ("$C_1$-$C_6$ alkoxy") or 1 to 4 carbon atoms ("$C_1$-$C_4$ alkoxy"). For example, $C_1$-$C_4$ alkoxy includes methoxy, ethoxy, isopropoxy, tert-butoxy, or the like. Unless otherwise specified, each example of alkoxy is optionally substituted independently, that is, alkoxy is unsubstituted ("unsubstituted alkoxy") or substituted with one or more substituents ("substituted alkoxy"). In some embodiments, alkoxy is unsubstituted $C_1$-$C_6$ alkoxy. In some embodiments, alkoxy is substituted $C_1$-$C_6$ alkoxy.

"Cycloalkyl" refers to non-aromatic cyclohydrocarbyl with 3 to 8 cyclocarbon atoms ("$C_{3-8}$ cycloalkyl") and zero heteroatom in a non-aromatic ring system. In some embodiments, cycloalkyl has 3 to 8 cyclocarbon atoms ("$C_{3-8}$ cycloalkyl"). In some embodiments, cycloalkyl has 3 to 6 cyclocarbon atoms ("$C_{3-6}$ cycloalkyl"). In some embodiments, cycloalkyl has 5 to 8 cyclocarbon atoms ("$C_{5-8}$ cycloalkyl"). Exemplary $C_{3-6}$ cycloalkyl includes, but is not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), or the like. Exemplary $C_{3-8}$ cycloalkyl includes, but is not limited to, the above-mentioned $C_{3-6}$ cycloalkyl, and cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), or the like. Unless otherwise specified, each example of cycloalkyl is optionally substituted independently, that is, cycloalkyl is unsubstituted ("unsubstituted cycloalkyl") or substituted with one or more substituents ("substituted cycloalkyl"). In some embodiments, cycloalkyl is unsubstituted $C_{3-8}$ cycloalkyl. In some embodiments, cycloalkyl is substituted $C_{3-8}$ cycloalkyl.

"Heterocycloalkyl" refers to a group of a 5 to 14-membered non-aromatic ring system with a cyclocarbon atom and 1 to 4 cyclic heteroatoms, where each heteroatom is independently selected from nitrogen, oxygen, and sulfur ("5 to 14-membered heterocyclyl"). In heterocyclyl with one or more nitrogen atoms, a junction point can be either a carbon atom or a nitrogen atom as long as a valence is available. Heterocyclyl can be a monocyclic system ("monocyclic heterocyclyl") or a fused-ring, bridged-ring, or spiro-ring system, such as a bicyclic system ("bicyclic heterocyclyl"), and can be saturated or partially unsaturated. "Heterocyclyl" also includes a ring system in which the heterocycle as defined above is fused with

one or more cycloalkyl groups (where a junction point is on cycloalkyl or the heterocycle) or a ring system in which the heterocycle as defined above is fused with one or more aryl or heteroaryl groups (where a junction point is on the heterocycle), and in this case, a number of ring members continues to indicate a number of ring members in the heterocycle system. Unless otherwise specified, each example of heterocyclyl is optionally substituted independently, that is, heterocyclyl is unsubstituted ("unsubstituted heterocycloalkyl") or substituted with one or more substituents ("substituted heterocycloalkyl"). In some embodiments, heterocycloalkyl is unsubstituted 5 to 14-membered heterocycloalkyl. In some embodiments, heterocycloalkyl is substituted 5 to 14-membered heterocycloalkyl.

"Aryl" or "an aromatic ring" or "aromatic cyclyl" refers to a group of a monocyclic or polycyclic (such as bicyclic or tricyclic) 4n+2 aromatic ring system (for example, there are 6, 10, or 14 $\pi$ electrons shared in a ring array) with 6 to 14 cyclocarbon atoms and zero heteroatom provided in an aromatic ring ("$C_{6-14}$ aryl"). In some embodiments, aryl has 6 cyclocarbon atoms ("$C_6$ aryl", such as phenyl). In some embodiments, aryl has 10 cyclocarbon atoms ("$C_{10}$ aryl", for example, naphthyl, such as 1-naphthyl and 2-naphthyl). In some embodiments, aryl has 14 cyclocarbon atoms ("$C_{14}$ aryl", such as anthracenyl). "Aryl" also includes a ring system in which the aryl ring as defined above is fused with one or more cycloalkyl or heterocyclyl groups (where a junction point is on the aryl ring), and in this case, a number of carbon atoms continues to indicate a number of carbon atoms in the aryl ring system. Unless otherwise specified, each example of aryl is optionally substituted independently, that is, aryl is unsubstituted ("unsubstituted aryl") or substituted with one or more substituents ("substituted aryl"). In some embodiments, aryl is unsubstituted $C_{6-14}$ aryl. In some embodiments, aryl is substituted $C_{6-14}$ aryl.

"Heteroaryl" is a 5 to 14-membered aromatic ring system with a cyclocarbon atom and 1 to 4 cyclic heteroatoms provided in an aromatic ring, where each heteroatom is independently selected from nitrogen, oxygen, and sulfur ("5 to 14-membered heteroaryl"). In some embodiments, "heteroaryl" is a 5 to 8-membered aromatic ring system with a cyclocarbon atom and 1 to 4 cyclic heteroatoms provided in an aromatic ring, where each heteroatom is independently selected from nitrogen, oxygen, and sulfur ("5 to 8-membered heteroaryl"). In some embodiments, "heteroaryl" is a 5 to 6-membered aromatic ring system with a cyclocarbon atom and 1 to 4 cyclic heteroatoms provided in an aromatic ring, where each heteroatom is independently selected from nitrogen, oxygen, and sulfur ("5 to 6-membered heteroaryl"). In some embodiments, 5 to 6-membered heteroaryl has 1 to 3 cyclic heteroatoms selected from nitrogen, oxygen, and sulfur. In some embodiments, 5 to 6-membered heteroaryl has 1 to 2 cyclic heteroatoms selected from nitrogen, oxygen, and sulfur. In some embodiments, 5 to 6-membered heteroaryl has 1 cyclic heteroatom selected from nitrogen, oxygen, and sulfur. Unless otherwise specified, each example of heteroaryl is optionally substituted independently, that is, heteroaryl is unsubstituted ("unsubstituted heteroaryl") or substituted with one or more substituents ("substituted heteroaryl"). In some embodiments, heteroaryl is unsubstituted 5 to 14-membered heteroaryl. In some embodiments, heteroaryl is substituted 5 to 14-membered heteroaryl.

The "halogen" or "halogenation element" refers to fluorine (-F), chlorine (-Cl), bromine (-Br), or iodine (-I).

The "substituted" or "optionally substituted" refers to the substitution of an atom such as a hydrogen atom in a group. In some embodiments, alkyl, cycloalkyl, heterocyclyl, aryl, and heteroaryl are substituted (such as, "substituted" alkyl, "substituted" cycloalkyl, "substituted" heterocycloalkyl, "substituted" aryl, or "substituted" heteroaryl). In general, the term "substituted", whether or not it is preceded by the term "optionally", means that at least one hydrogen present on a group (such as a carbon or nitrogen atom) is substituted with a permissible substituent. For example, after the substitution is conducted with the substituent, a stable compound will be produced, for example, the compound will not be spontaneously transformed (for example, through rearrangement, cyclization, elimination, or other reactions). Unless otherwise specified, a "substituted" group has a substituent at one or more substitutable positions, and when two or more positions in any given structure are substituted, substituents at these positions are the same or different. It is expected that the term "substituted" includes substitutions with all permissible substituents of organic compounds and substitutions with any substituents described herein that lead to the production of stable compounds. The present disclosure anticipates any and all of these combinations to obtain stable compounds. For the objectives of the present disclosure, heteroatoms such as nitrogen may have hydrogen substituents and/or any suitable substituents as described herein that satisfy valences of the heteroatoms and result in the production of stable moieties. In some embodiments, the substituent is a carbon atom substituent. In some embodiments, the substituent is a nitrogen atom substituent. In some embodiments, the substituent is an oxygen atom substituent. In some embodiments, the substituent is a sulfur atom substituent.

The "unsaturated" or "partially unsaturated" means that a group includes at least one double or triple bond. A "partially unsaturated" ring system is also intended to encompass a ring with a plurality of unsaturated sites, but is not intended to include an aromatic group (such as aryl or heteroaryl). Similarly, the "saturated" means that a group does not include a double or triple bond, that is, the group includes only single bonds.

In the present disclosure, when there are a plurality of substitutions in a defined substituent, there may be repeated definitions in the textual description for the plurality of substitutions, but this description should be understood as at least conforming to the basic laws of general medicinal chemistry. For example, when repeated definitions are provided for a substituent, those skilled in the art can determine whether such repeated definitions are feasible or infeasible according to the common knowledge of general medicinal chemistry. With the definition of the substituent $R_A$ as an

example, $R_A$ can be *"saturated or unsaturated $C_1$-$C_8$ alkyl"* or *"saturated or unsaturated $C_1$-$C_8$ alkyl substituted with halogen, hydroxyl, amino, or $R_C$*", and the substituent Rc can also be defined as *"hydrogen, halogen, hydroxyl, amino, or the like".* Those skilled in the art can determine whether these repeated definitions are feasible according to the general knowledge of medicinal chemistry, and make a reasonable choice.

The term "pharmaceutically-acceptable" as used herein means that compounds, materials, compositions, and/or dosage forms are suitable for use in contact with human and animal tissues in a reliable medical determination range, which does not have excessive toxicity, irritability, anaphylaxis, or other problems or complications and is commensurate with a reasonable benefit/risk ratio.

The term "pharmaceutically-acceptable salt" refers to a salt of the compound in the present disclosure, which is prepared from the compound with a specific substituent discovered by the present disclosure and a relatively-nontoxic acid or base. When the compound of the present disclosure includes a relatively-acidic functional group, a base-addition salt can be obtained by making a neutral form of the compound in contact with a sufficient amount of a base in a pure solution or a suitable inert solvent. Pharmaceutically-acceptable base-addition salts include sodium, potassium, calcium, ammonium, and organic amine salts, or magnesium salts, or similar salts. When the compound of the present disclosure includes a relatively-basic functional group, an acid-addition salt (namely, a pharmaceutically-acceptable salt) can be obtained by making a neutral form of the compound in contact with a sufficient amount of an acid in a pure solution or a suitable inert solvent. Examples include an inorganic acid salt and an organic acid salt. An inorganic acid includes, for example, hydrochloric acid, hydrobromic acid, nitric acid, carbonic acid, a bicarbonate, phosphoric acid, a monohydrogen phosphate, a dihydrogen phosphate, sulfuric acid, a bisulfate, hydroiodic acid, phosphorous acid, or the like. An organic acid includes, for example, benzoic acid, 2-hydroxyethanesulfonic acid, sulfamic acid, benzenesulfonic acid, phenylacetic acid, mandelic acid, malonic acid, propionic acid, oxalic acid, p-amino benzenesulfonic acid, p-toluenesulfonic acid, polygalacturonic acid, pantothenic acid, fumaric acid, glutamic acid, succinic acid, methanesulfonic acid, tartaric acid, ascorbic acid, phthalic acid, maleic acid, citric acid, malic acid, glucoheptonic acid, gluconic acid, hydroxyethanesulfonic acid, lactic acid, lactobionic acid, dodecanesulfonic acid, dihydroxynaphthoic acid, salicylic acid, suberic acid, folinic acid, edetic acid, glycolic acid, acetic acid, ethanesulfonic acid, isobutyric acid, stearic acid, or the like. The examples further include salts of amino acids (such as arginine) and salts of organic acids such as glucuronic acid (see Berge et al., "Pharmaceutical Salts", Journal of Pharmaceutical Science 66: 1-19 (1977)). Some specific compounds of the present disclosure include both basic and acidic functional groups and thus can be transformed into any base or acid-addition salts. A parent form of a compound is different from various salt forms of the compound in some physical properties, such as different solubilities in a polar solvent.

As used herein, the modifier term "about" refers to a possible change in a value, for example, through a routine test and treatment; through an unintentional error in such a test and treatment; through a difference in the manufacture, origin, or purity of an ingredient used in the present disclosure; or the like. As used herein, when "about" is used before a specific value, the specific value is also encompassed, for example, about 10% includes 10%. Whether or not it is modified by the term "about", the claims include an equivalent form of a listed quantity. In an embodiment, the term "about" refers to an error within 20% of a reported value.

As used herein, the term "treatment" refers to elimination, alleviation, or improvement of a disease or a disorder and/or symptoms associated therewith. Although there is no corresponding exclusion, the treatment of a disease or a disorder does not require the complete elimination of the disease or the disorder or symptoms associated therewith. As used herein, the term "treatment" may include "preventive treatment", and refers to the likelihood of reducing the re-development of a disease or disorder or the recurrence of a previously-controlled disease or disorder in a subject who does not develop or develops the disease or disorder, or is prone to the disease or disorder, or is at risk of undergoing recurrence of the disease or disorder. The term "treatment" and synonyms thereof consider the administration of a therapeutically-effective amount of the compound described herein to a subject in need of such a treatment.

In the case of a drug or a pharmacologically-active agent, the term "effective amount" or "therapeutically-effective amount" refers to a sufficient amount of the drug or agent that is non-toxic, but can allow a desired effect. For an oral dosage form of the present disclosure, the "effective amount" of an active substance in a composition refers to an amount of the active substance required to allow a desired effect when the active substance is used in combination with another active substance in the composition. The determination of the effective amount varies from person to person, and the effective amount depends on an age and general conditions of a recipient and also depends on a specific active substance. An appropriate effective amount in each case can be determined by a person skilled in the art based on routine testing.

The disease associated with an AHR disorder according to the present disclosure, includes, but is not limited to, tumors.

The following examples are merely examples of the embodiments of the present disclosure, and do not constitute any limitation on the present disclosure. Those skilled in the art can understand that any modifications made without departing from the essence and conception of the present disclosure fall within the protection scope of the present disclosure. Unless otherwise specified, the reagents and instruments used in the following examples are commercially-available products.

**Experimental section:**

**Example 1: Preparation of an intermediate III-A according to a preparation process A**

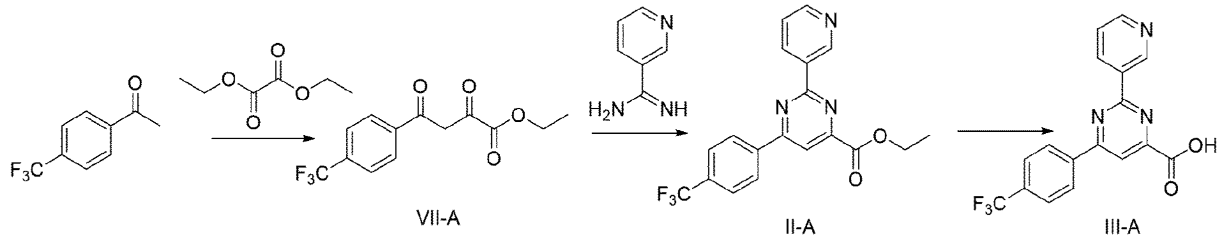

VII-A II-A III-A

Step 1) Preparation of a compound VII-A

A mixed solution of diethyl oxalate (15.2 g, 104 mmol) and sodium ethoxide (10.9 g, 160 mmol) in absolute ethanol (250 mL) was cooled to 10°C or lower and stirred for 30 min, then a solution of 4-trifluoromethylbenzophenone (18.8 g, 100 mmol) in absolute ethanol (80 mL) was added dropwise, and an ice bath was removed, followed by stirring overnight at room temperature, filtration, washing with a small amount of ethanol, and drying to obtain the compound VII-A (28 g, yield: 97%), LC/MS (ESI): *m*/*z* 289 [M+1]$^+$.

Step 2) Preparation of II-A

The intermediate VII-A (288 mg, 1 mmol) and 3-amidinopyridine hydrochloride (157 mg, 1 mmol) were dissolved in 30 mL of ethanol, sodium ethoxide (312 mg, 4.59 mmol) was added to obtain a reaction suspension, and the reaction suspension was stirred overnight at 70°C. When LCMS results showed that 60% of the intermediate II-A and 40% of an intermediate III-A were produced, vacuum concentration was conducted until dryness to obtain a II-A/III-A mixture, which could be directly used for the next reaction without purification, II-A: LC/MS (ESI): *m*/*z* 374[M+1]$^+$, III-A: 346 [M+1]$^+$, 344 [M-1]$^+$.

Step 3) Preparation of the compound III-A

The II-A/III-A mixture obtained in the step 2) was dissolved in a methanol/tetrahydrofuran (THF)/water (1/1/1) mixed solvent, and lithium hydroxide monohydrate (4 equivalents) was added, followed by stirring at room temperature for 5 h, neutralization with 1 N hydrochloric acid to a pH of 7, vacuum concentration to a small volume, and filtration, and the obtained solid was washed with ethanol-water (1:1) and dried to obtain the intermediate III-A (205 mg, 79%), LC/MS (ESI): *m*/*z*346 [M+1]$^+$, 344[M-1]$^+$.

**Example 2: Preparation of an intermediate III-A according to a preparation process C**

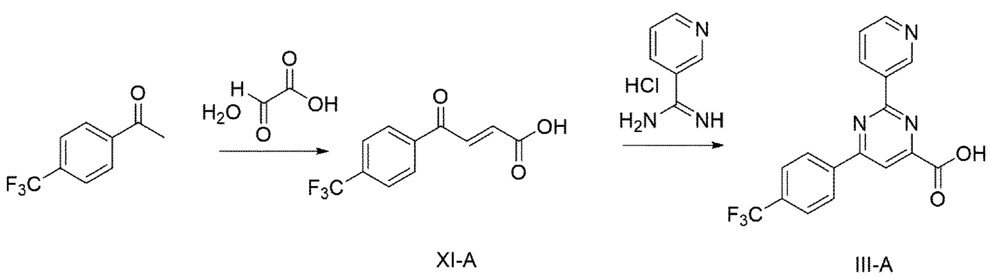

XI-A III-A

Step 1) Preparation of a compound XI-A

4-trifluoromethylacetophenone (11.3 g, 60 mmol) was added to a solution of glyoxylic acid (8.83 g, 96 mmol) in acetic acid (50 mL) at room temperature, followed by stirring overnight at 120°C and vacuum concentration until dryness,

and water was added, followed by stirring for 30 min, filtration, washing with water, and drying to obtain a compound XI-A (12.3 g, yield: 84%), LC/MS (ESI): *m/z* 243[M-1]$^+$.

Step 2) Preparation of the compound III-A

Sodium ethoxide (310 mg, 4.6 mmol) was added to a solution of the compound XI-A (245 mg, 1 mmol) and 3-amidinopyridine hydrochloride (157.6 mg, 1 mmol) in ethanol (30 mL), followed by stirring overnight at 70°C and vacuum concentration, and water and ethyl acetate (EA) were added, followed by stirring for 30 min and filtration to obtain the product III-A (200 mg, yield: 58%), $^1$H NMR (400 MHz, DMSO-$d_6$): δ9.10(d, J=1.6Hz, 1H), 8.60-9.00(m, 1H), 8.80-8.81(dd, J=4.8, 1.6Hz, 1H), 8.66(d, J=8.0Hz, 2H), 8.57(s, 1H), 7.98(d, J=8.0Hz, 2H),7.64-7.67(m, 1H). LC/MS (ESI): *m/z* 346 [M+1]$^+$.

**Example 3: Preparation of an intermediate III-B according to a preparation process B**

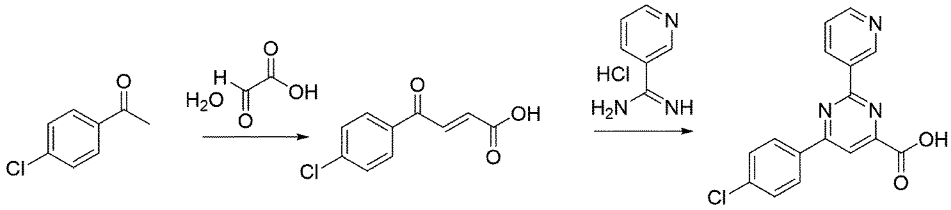

XI-B III-B

Step 1) Preparation of a compound XI-B

P-chloroacetophenone (9.3 g, 60 mmol) was added to a solution of glyoxylic acid (8.83 g, 96 mmol) in acetic acid (50 mL) at room temperature, followed by stirring overnight at 120°C and vacuum concentration until dryness, and water was added, followed by stirring for 30 min, filtration, washing with water, and drying to obtain a compound XI-B (9.4 g, yield: 75%), LC/MS (ESI): *m/z* 209[M-1]$^+$.

Step 2) Preparation of the compound III-B

Sodium ethoxide (310 mg, 4.6 mmol) was added to a solution of the compound XI-B (210 mg, 1 mmol) and 3-amidinopyridine hydrochloride (157.6 mg, 1 mmol) in ethanol (30 mL), followed by stirring overnight at 70°C until a reaction was completed and vacuum concentration, and water and EA were added, followed by stirring for 30 min and filtration to obtain the product III-B (149 mg, yield: 48%), LC/MS (ESI): *m/z* 312[M+1]$^+$.

The following intermediates III-C to III-G could be prepared by a preparation process similar to the preparation process B in Example 3.

| Intermediate No. | Structural formula | Structural formula of a starting material | LC/MS(ESI): *m/z*[M+1]$^+$ / [M-1]$^+$ |
|---|---|---|---|
| III-C | 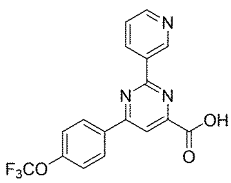 | 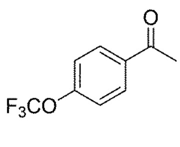 | 362/360 |
| III-D | 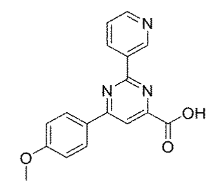 | 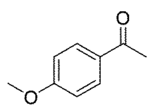 | 308/306 |

(continued)

| Intermediate No. | Structural formula | Structural formula of a starting material | LC/MS(ESI): *m/z*[M+1]$^+$ / [M-1]$^+$ |
|---|---|---|---|
| III-E | 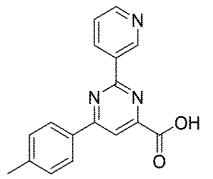 | 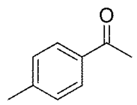 | 292/290 |
| III-F | 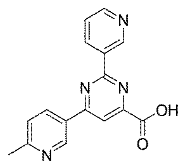 | 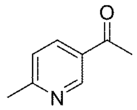 | 293/291 |
| III-G | 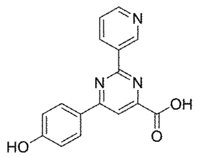 | 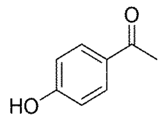 | 294/292 |

**Example 4: Preparation of an intermediate III-H according to a preparation process C**

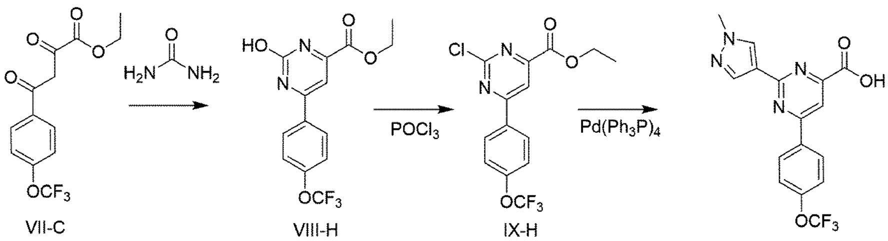


VII-C VIII-H IX-H III-H

Step 1) Preparation of an intermediate VIII-H

1 mL of concentrated hydrochloric acid was added to a solution of urea (66 mg, 1.1 mmol) and a compound VII-C (305 mg, 1 mmol) in ethanol (4 mL), followed by stirring overnight at 80°C, cooling to room temperature, filtration, washing with cold ethanol, and drying to obtain VIII-H, LC/MS (ESI): *m/z* 329[M+1]$^+$.

Step 2) Preparation of an intermediate IX-H

The compound VIII-H (81 mg, 0.25 mmol) was added to 2 mL of phosphorus oxychloride, followed by heating to 85°C under nitrogen protection, stirring for 6 h, and vacuum concentration until dryness, and EA (10 mL) and 20 g of crushed ice were added, followed by stirring for 20 min, filtration, and drying to obtain the compound IX-H, LC/MS (ESI): *m/z* 347[M+1]$^+$.

Step 3) Preparation of the intermediate III-H

Tetrakis(triphenylphosphine)palladium (10 mg) and $PdCl_2(dppf)_2$ (10 mg) were added to a mixed solution of the compound IX-H (348 mg, 1 mmol) and 1-methylpyrazole-4-boronic acid pinacol ester (208 mg, 1 mmol) in dioxane (5 mL) and a saturated sodium carbonate aqueous solution (1 mL) under nitrogen protection, followed by heating to 100°C and stirring overnight to obtain an organic phase, and the organic phase was vacuum-concentrated until dryness and purified through silica gel column chromatography to obtain the intermediate III-H, LC/MS (ESI): *m/z* 365[M+1]$^+$, 363[M-1]$^+$.

The following intermediates III-I to III-P could be prepared by a preparation process similar to the preparation

process C in Example 4.

| Intermediate No. | Structural formula | Structural formula of a starting material | LC/MS(ESI): *m*/*z*[M+1]$^+$/[M-1]$^+$ |
|---|---|---|---|
| III-I | 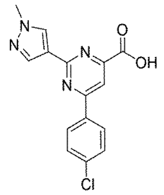<br>III-I | 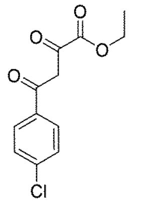<br>VII-B | 315/313 |
| III-J | 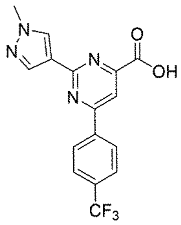<br>$^1$H NMR (400 MHz, DMSO-d6): δ13.91(brs,1H), 8.58(s,1H), 8.56(d, *J*=8.4Hz, 2H), 8.30(s, 1H), 8.20(s,1H),7.94(d,*J*=8.4Hz,2 H), 3.96(s,3H) | 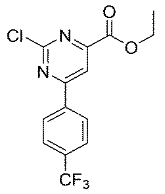<br>$^1$H NMR (400 MHz, DMSO-d6): δ8.61(s, 1H), 8.47(d, *J*=8.0Hz, 2H), 7.94(d, *J*=8.0Hz, 2H), 4.45(q, *J*=7.2Hz, 2H), 1.40(t, *J*=7.2Hz, 3H) | 349/347 |
| III-K | 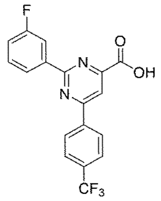 | 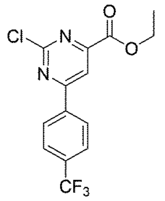 | 363/361 |
| III-L | 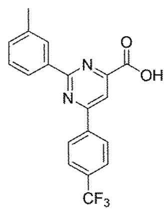 | 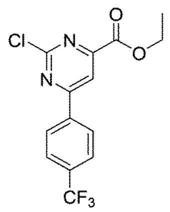 | 359/357 |
| III-M | 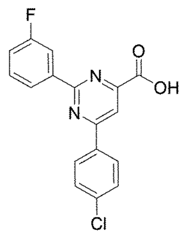 | 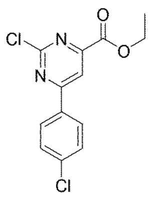 | 329/327 |
| III-N | 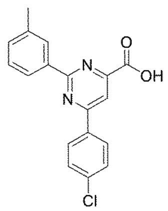 | 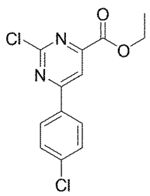 | 325/323 |

(continued)

| Intermediate No. | Structural formula | Structural formula of a starting material | LC/MS(ESI): $m/z$[M+1]$^+$/[M-1]$^+$ |
|---|---|---|---|
| III-O | 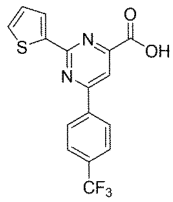 | 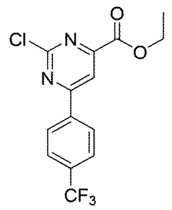 | 351/349 |
| III-P | 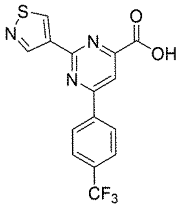 | 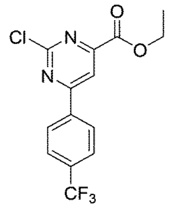 | 352/350 |

**Example 5: Preparation of an intermediate III-Q according to a preparation process D**

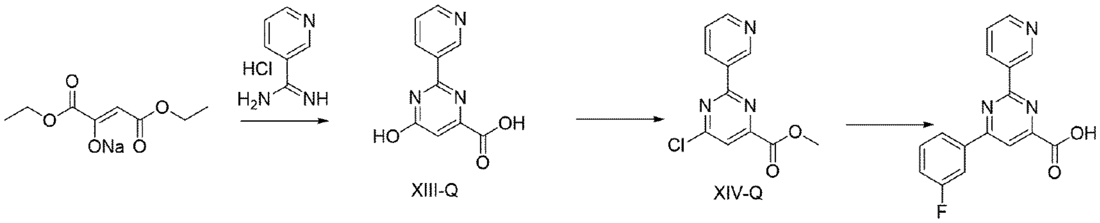

XIII-Q XIV-Q III-Q

Step 1) Preparation of an intermediate XIII-Q

A NaOH solution (2.17 g, 54 mmol, 4.5 mL of water) was added dropwise to a suspension of a diethyl oxalacetate sodium salt (6.65 g, 31.7 mmol) in water (40 mL), followed by stirring until solids were completely dissolved, and then 3-amidinopyridine hydrochloride (3.85 g, 31.8 mmol) was added, followed by heating to 70°C, stirring overnight, cooling to room temperature, adjusting with concentrated hydrochloric acid to a pH of 1, and filtration to obtain a solid, and the solid was washed with EA to obtain the intermediate XIII-Q (4.42 g, yield: 83%). $^1$H NMR (DMSO-d6): $\delta$ 9.30 (d, $J$=2.0Hz, 1H), 8.76(dd, $J$=1.2, 4.8Hz, 1H), 8.50 (dt, $J$=7.6, 1.2Hz, 1H), 7.60(dd, $J$=4.8, 7.6Hz, 1H), 6.96(s, 1H); and LC/MS (ESI): $m/z$ 218 [M+1]$^+$, 216[M-1]$^+$.

Step 2) Preparation of an intermediate XIV-Q

A suspension of the compound XIII-Q (218 mg, 1 mmol) in $POCl_3$ (5 mL) was stirred overnight at 85°C under nitrogen protection and then vacuum-concentrated until dryness, 50 mL of EA was added, followed by stirring for 10 min, and 1 mL of methanol was added, followed by stirring, washing with water, drying with anhydrous sodium sulfate, filtration, and vacuum concentration to obtain the intermediate XIV-Q, which could be directly used for the next step without purification.

Step 3) Preparation of the intermediate III-Q

Tetrakis(triphenylphosphine)palladium (15 mg) was added to a mixed solution of the intermediate XIV-Q (250 mg, 1 mmol) and 3-fluorophenylboronic acid (140 mg, 1 mmol) in dioxane (10 mL) and a saturated sodium carbonate aqueous solution (1 mL) under nitrogen protection, followed by heating to 100°C and stirring overnight to obtain an organic phase, and the organic phase was vacuum-concentrated until dryness and purified through silica gel column chromatography to obtain the intermediate III-Q, LC/MS (ESI): *m*/*z* 296$[M+1]^+$, 294$[M-1]^+$.

The following intermediates III-R to III-V could be prepared by a preparation process similar to the preparation process D in Example 5.

| Intermediate No. | Structural formula | Structural formula of a starting material | LC/MS(ESI): *m*/*z*$[M+1]^+$/$[M-1]^+$ |
|---|---|---|---|
| III-R | 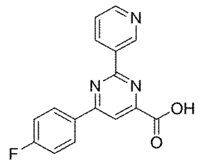 | 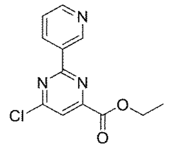 | 315/313 |
| III-S | 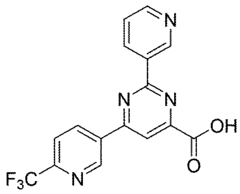 | 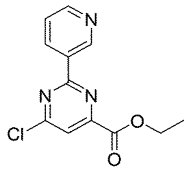 | 345/347 |
| III-T | 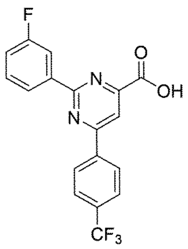 | 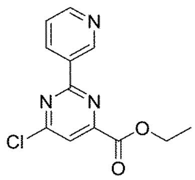 | 363/361 |
| III-U | 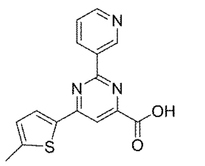 | 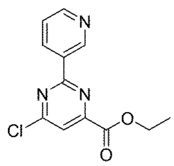 | 296/298 |
| III-V | 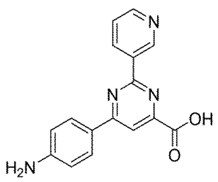 | 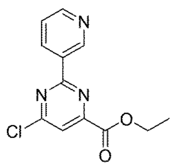 | 291/293 |

**Example 6: Preparation of intermediates III-W and III-Y according to preparation processes E and F, respectively**

Process E

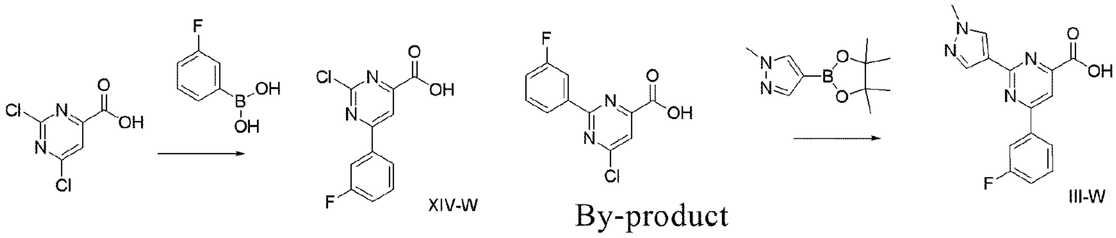
XIV-W
By-product
III-W

Step 1) Preparation of an intermediate XIV-W

Tetrakis(triphenylphosphine)palladium (50 mg) and $PdCl_2(dppf)_2$ (50 mg) were added to a mixed solution of 2,6-dichloropyrimidine-4-carboxylic acid (1.93 g, 10 mmol) and 3-trifluorophenylboronic acid (2.27 g, 11 mmol) in toluene (50 mL) and a saturated sodium carbonate aqueous solution (10 mL), followed by heating to 110°C under nitrogen protection and stirring overnight to obtain an organic phase, and the organic phase was vacuum-concentrated until dryness and purified through silica gel column chromatography to obtain the intermediate XIV-W (2.04 g, 376%) and 1.4 g of a by-product (XV-Y), XIV-W: $^1$HNMR: (400 MHz, DMSO-$d_6$) δ8.34(s, 1H), 7.20-7.44(m, 3H), 6.97(m, 1H); and LC/MS (ESI): *m/z* 251[M-1]$^+$.

Step 2) Preparation of the III-W

The compound XIV-W (504 mg, 2 mmol) and 1-methylpyrazole-4-boronic acid pinacol ester (624 mg, 3 mmol) were dissolved in 20 mL of dioxane, and a saturated sodium carbonate solution (1 mL) and tetrakis(triphenylphosphine) palladium (231 mg, 0.2 mmol) were added, followed by stirring overnight at 100°C under nitrogen protection, vacuum concentration until dryness, adjusting with 2 N HCl to a pH of 6 to 7, and filtration to obtain a crude product, and the crude product was washed with dichloromethane (DCM) and purified through silica gel column chromatography to obtain the compound III-W, LC/MS (ESI): *m/z* 299[M+1]$^+$, 297[M-1]$^+$.

Process F

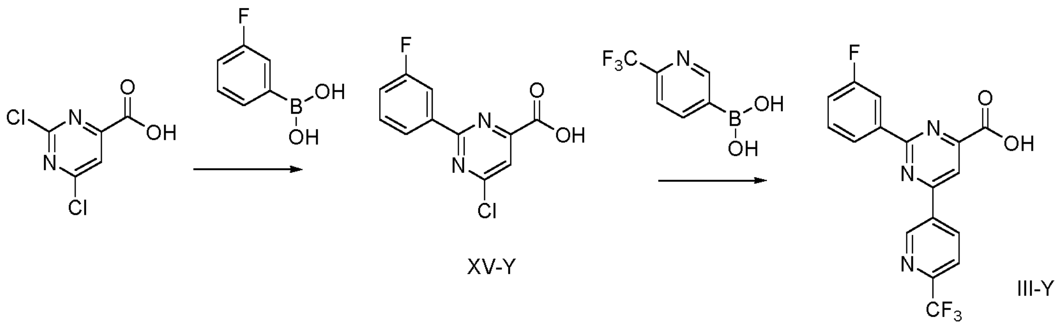

XV-Y

III-Y

Step 1) Preparation of an intermediate XV-Y The intermediate XV-Y was prepared with reference to the process E (see the step 1) of the process E), XV-Y: LC/MS (ESI): *m/z* 253[M+1]$^+$.

Step 2) Preparation of the III-W

The compound XV-Y (504 mg, 2 mmol) and 6-(trifluoromethyl)pyridin-3-boronic acid (573 mg, 3 mmol) were dissolved in 20 mL of dioxane, and a saturated sodium carbonate solution (1 mL) and tetrakis(triphenylphosphine) palladium (231 mg, 0.2 mmol) were added, followed by stirring overnight at 100°C under nitrogen protection, vacuum concentration until dryness, adjusting with 2 N HCl to a pH of 6 to 7, filtration, washing with DCM, and purification through silica gel column chromatography to obtain the compound III-Y, LC/MS (ESI): *m/z* 364[M+1]$^+$, 362[M-1]$^+$.

**Example 7: Preparation of an intermediate IV-1**

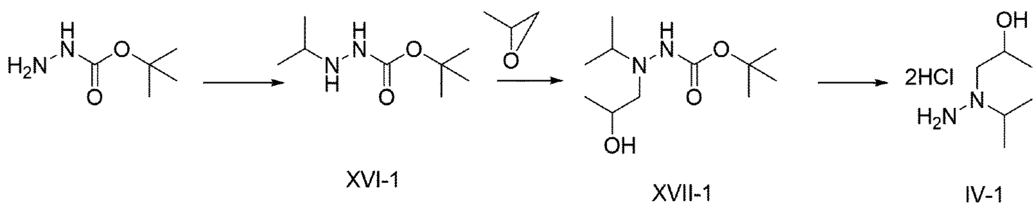

XVI-1 XVII-1 IV-1

Step 1) Preparation of an intermediate XVI-1

$BocNHNH_2$ (13.2 g, 100 mmol) was dissolved in 100 mL of acetone and 50 mL of methanol, and anhydrous magnesium sulfate (12 g, 100 mmol) was added, followed by stirring overnight at room temperature and filtration for magnesium sulfate removal to obtain a filtrate. The filtrate was vacuum-concentrated until dryness, 100 mL of methanol was added for dissolution, and sodium borohydride (15.2 g, 400 mmol) was added in batches at room temperature, followed by stirring at room temperature for 1 h and vacuum concentration until dryness. 200 mL of distilled water was added, and extraction was conducted with 100 mL of DCM 3 times. Organic phases were combined, dried with anhydrous sodium sulfate, filtered, vacuum-concentrated until dryness, and purified through silica gel column chromatography (20% EA/petroleum ether (PE)) to obtain the XVI-1 (15.1 g, yield: 87%), LC/MS (ESI): *m*/*z* 175 $[M+1]^+$.

Step 2) Preparation of an intermediate XVII-1

The intermediate XVI-1 (1.74 g, 10 mmol), propylene oxide (2.9 g, 50 mmol), and DIPEA (2.58 g, 20 mmol) were dissolved in 20 mL of ethanol in a sealed reaction flask, followed by stirring overnight at 80°C, vacuum concentration until dryness, and purification through silica gel column chromatography (20%-50% EA/PE) to obtain the XVII-1 (1.82 g, yield: 78%), LC/MS (ESI): *m*/*z* 233 $[M+1]^+$.

Step 3) Preparation of the intermediate IV-1

1.82 g of the compound XVII-1 was dissolved in 10 mL of methanol, and a solution of HCl in methanol (at an excess amount) was added, followed by stirring overnight at room temperature and vacuum concentration at 40°C until dryness to obtain a hydrochloride of the intermediate IV-1 (2.3 g, yield: 95%), LC/MS (ESI): *m*/*z* 133 $[M+1]^+$.

**Example 8: Preparation of an intermediate IV-2**

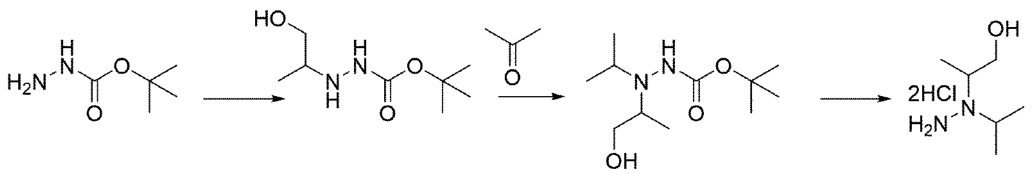

XVI-2 XVII-2 IV-2

Step 1) Preparation of an intermediate XVI-2

$BocNHNH_2$ (30 g, 227 mmol), 300 mL of methanol, 20.4 g of hydroxyacetone, and anhydrous magnesium sulfate (68 g) were added successively to a 500 mL round-bottom flask, stirred at room temperature for 3.5 h, and filtered for magnesium sulfate removal to obtain a filter cake, and the filter cake was washed with anhydrous methanol to obtain a filtrate. Sodium borohydride (15.2 g, 400 mmol) was added in batches at 0°C to the filtrate, followed by stirring at room temperature for 1 h and vacuum concentration until dryness. 200 mL of distilled water was added, and extraction was conducted with 100 mL of DCM 3 times. Organic phases were combined, dried with anhydrous sodium sulfate, filtered, vacuum-concentrated until dryness, and purified through silica gel column chromatography (20%-40% EA/PE) to obtain the XVI-2 (36 g, yield: 84%), LC/MS (ESI): *m*/*z* 191 $[M+1]^+$.

Step 2) Preparation of an intermediate XVII-2

The intermediate XVI-2 (19.0 g, 100 mmol), acetone (17.4 g, 300 mmol), 300 mL of methanol, and 1 mL of acetic acid were added to a 1 L round-bottom flask, and then sodium cyanoborohydride (15.7 g, 250 mmol) was added at room temperature, followed by stirring overnight at room temperature and vacuum concentration until dryness. Distilled water was added, and extraction was conducted with EA, followed by drying, filtration, and concentration. And a white solid XVII-2 (14.1 g, yield: 61%) was purified and obtained by silica gel column chromatography (10%-50% EA/PE), LC/MS (ESI): *m*/*z* 233$[M+1]^+$.

Step 3) Preparation of the intermediate IV-2: The intermediate IV-2 was prepared by the same process as the intermediate IV-1 in the step 3 of Example 7, with a yield of 90%, LC/MS (ESI): *m*/*z* 133 $[M+1]^+$.

**Example 9: Preparation of an intermediate IV-3**

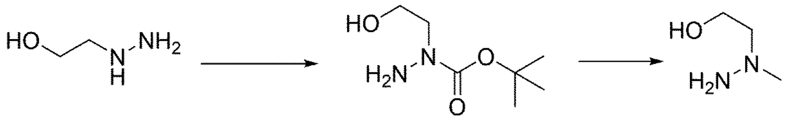

XVII-3 IV-3

Step 1) Preparation of an intermediate XVII-3

13 mL of triethylamine and $Boc_2O$ (17.2 g, 78 mmol) were added to a solution of 2-hydroxyethylhydrazine (5 g, 65 mmol) in methanol (100 mL) at 0°C, followed by stirring at room temperature for 16 h under nitrogen protection and vacuum concentration until dryness. A citric acid solution was added, extraction was conducted with EA, drying was conducted with anhydrous sodium sulfate, followed by filtration and vacuum concentration until dryness. And the XVII-3 (6.8 g, yield: 89%) was purified and obtained by silica gel column chromatography (20%-40% EA/PE), LC/MS (ESI): *m/z* 177 $[M+1]^+$.

Step 2) Preparation of the intermediate XVII-3

The intermediate XVII-3 (2 g, 11.2 mmol) was dissolved in 50 mL of THF, LAH (1.7 g, 44.8 mmol) was added, followed by heating under nitrogen protection to allow reflux for 5 h and then cooling to 0°C or lower. $Na_2SO_4 \cdot 10H_2O$ was added in batches, followed by filtration to obtain a filter cake, and the filter cake was washed with THF to obtain a filtrate. The filtrate was dried with anhydrous magnesium sulfate and concentrated to obtain a crude product IV-3, which could be directly used for the next step without purification, LC/MS (ESI): *m/z* 91$[M+1]^+$.

**Example 9: Preparation of an intermediate IV-4**

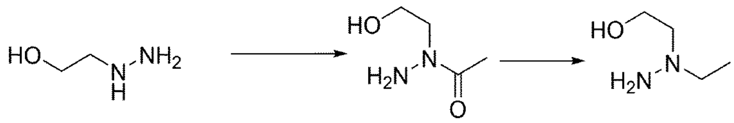

XVII-4 IV-4

Step 1) Preparation of an intermediate XVII-4

2-hydroxyethylhydrazine (7.6 g, 100 mmol) was added to a mixed solvent of methanol (100 mL) and acetone (50 mL) at 0°C, and anhydrous magnesium sulfate (12 g, 100 mmol) was added, followed by stirring overnight at room temperature and filtered for magnesium sulfate removal to obtain a filtrate. The filtrate was vacuum-concentrated until dryness, 100 mL of DCM was added for dissolution, triethylamine (20.2 g, 200 mmol) was added, and acetic anhydride (10.7 g, 105 mmol) was added dropwise at 0°C, followed by stirring overnight at room temperature. 200 mL of distilled water was added, and extraction was conducted with DCM. Organic phases were combined and vacuum-concentrated until dryness, THF (50 mL) and 5 mL of hydrochloric acid were added, followed by stirring at room temperature for 12 h and vacuum concentration until dryness to obtain the XVII-4, which could be directly used for the next step without purification, LC/MS (ESI): *m/z* 119 $[M+1]^+$.

Step 2) Preparation of the intermediate IV-4

The intermediate XVII-4 (1.2 g, 10 mmol) was dissolved in 50 mL of THF, and LAH (1.5 g, 44.8 mmol) was added, followed by heating under nitrogen protection to allow reflux for 4 h and then cooling to 0°C or lower. $Na_2SO_4 \cdot 10H_2O$ was added in batches for reaction quenching=, followed by filtration to obtain a filter cake, and the filter cake was washed with THF to obtain a filtrate. The filtrate was dried with anhydrous magnesium sulfate, filtered, and vacuum-concentrated until dryness to obtain the IV-4, LC/MS (ESI): *m/z* 105 $[M+1]^+$.

**Example 10: Preparation of an intermediate IV-5**

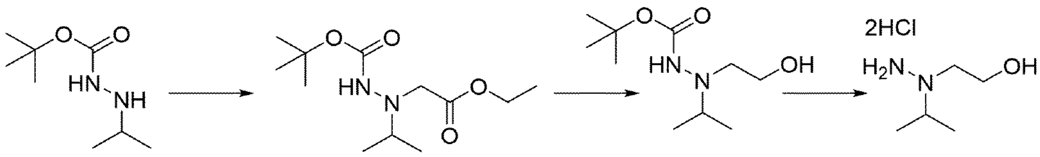

XVI-1 XVII-5 XVIII-5 IV-5

Step 1) Preparation of an intermediate XVII-5

The intermediate XVI-1 (12.8 g, 73.5 mmol), ethyl bromoacetate (36.6 g, 219 mmol), and anhydrous potassium carbonate (30.2 g, 219 mmol) were dissolved in 250 mL of DMF, followed by heating to 80°C under nitrogen protection, stirring overnight, and cooling to room temperature. Water was added, extraction was conducted with EA, drying was conducted with anhydrous sodium sulfate, followed by filtration and vacuum concentration until dryness to obtain 18 g of a crude product XVII-5, LC/MS (ESI): *m/z* 261$[M+1]^+$.

Step 2) Preparation of an intermediate XVII-5

380 mL of ethanol, $NaBH_4$ (7.2 g, 189 mmol), and LiCl (7.9 g, 188 mmol) were added successively to a 2 L round-bottom flask, and then a solution of XVII-5 (18 g, 69 mmol) in THF (380 mL) was added dropwise at room temperature, followed by stirring for 6 h and vacuum concentration to remove a large amount of the solvent. Water was added, and extraction was conducted with EA. Organic phases were combined, dried with anhydrous $Na_2SO_4$, filtered, and vacuum-concentrated until dryness to obtain the XVII-5 (12.9 g, 86%), LC/MS (ESI): *m/z* 219$[M+1]^+$.

Step 3) Preparation of the intermediate IV-5

The XVIII-5 (12.9 g) was dissolved in 50 mL of dioxane, and a solution of HCl in dioxane (6 N, 30 mL) was added, followed by stirring overnight at room temperature and vacuum concentration until dryness to obtain the intermediate IV-5 (12.8 g), LC/MS (ESI): *m/z* 119$[M+1]^+$.

**Example 11: Preparation of an intermediate IV-6**

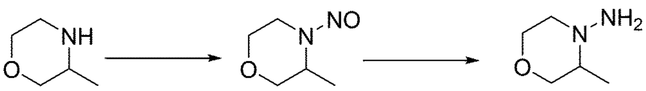

XIX-6 IV-6

Step 1) Preparation of an intermediate XIX-6

Tert-butyl nitrite (1.1 g, 11 mmol) was added to a solution of 3-methylmorpholine (1.0 g, 10 mmol) in THF (15 mL), followed by heating to allow reflux for 12 h and vacuum concentration until dryness to obtain the XIX-6, which could be directly used for the next step without purification, LC/MS (ESI): *m/z* 131$[M+1]^+$.

Step 2) Preparation of the intermediate IV-6

The intermediate XIX-6 (80 mmol) obtained in the previous step was dissolved in 200 mL of THF, and LAH (6.1 g, 160 mmol) was added in batches at 0°C, followed by stirring overnight at room temperature and cooling. $Na_2SO_4 \cdot 10H_2O$ was added in batches, followed by filtration to obtain a filter cake, and the filter cake was washed with THF to obtain a filtrate. The filtrate was dried with anhydrous magnesium sulfate, filtered, and vacuum-concentrated to obtain the compound IV-6 (yield: 72%), LC/MS (ESI): *m/z* 117 $[M+1]^+$.

The following hydrazines could be prepared by the process in Example 11.

| Intermediate No. | Structural formula | Structural formula of a starting material | LC/MS(ESI): $m/z$[M+1]$^+$ |
|---|---|---|---|
| IV-6(R) | 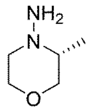 | 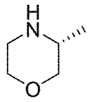 | 117 |
| IV-6(S) | 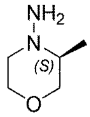 | 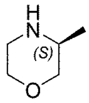 | 117 |
| IV-7 | 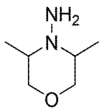 | 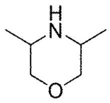 | 131 |
| IV-8 | 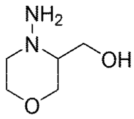 | 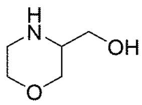 | 132 |
| IV-9 | 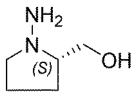 | 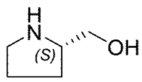 | 117 |
| IV-10 | 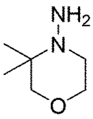 | 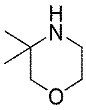 | 131 |
| IV-11 | 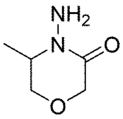 | 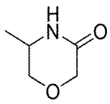 | 131 |
| IV-12 | 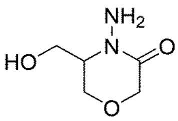 | 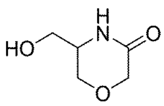 | 147 |
| IV-13 | 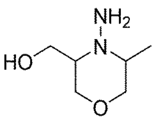 | 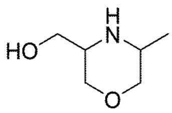 | 147 |
| IV-14 | 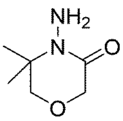 | 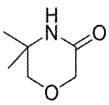 | 145 |
| IV-15 | 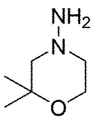 | 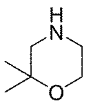 | 131 |
| IV-16(R) | 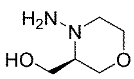 | 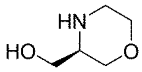 | 133 |

(continued)

| Intermediate No. | Structural formula | Structural formula of a starting material | LC/MS(ESI): *m/z*[M+1]+ |
|---|---|---|---|
| IV-16(S) | 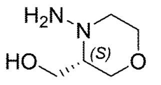 | 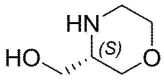 | 133 |

**Example 12: Preparation of 6-(4-trifluoromethylphenyl)-N-(1-hydroxypropyl-2-yl)-N-isopropyl-2-(pyridin-3-yl) pyrimi din-4-hydrazide (Compound 1)**

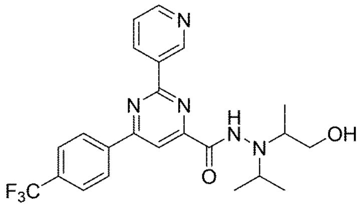

Compound 1

The intermediate III-A (36 mg, 0.1 mmol), hydrazine hydrochloride (IV-2) (20 mg, 0.12 mmol), and 2-(7-azabenzotriazol-1-yl)-N,N,N',N'-tetramethyluronium hexafluorophosphate (HATU) (57 mg, 0.15 mmol) were dissolved in 2 mL of N,N-dimethylformamide (DMF), and DIPEA (47 mg, 0.36 mmol) was added, followed by stirring overnight under nitrogen protection, dilution with EA, and washing with water to obtain an organic phase. The organic phase was dried with anhydrous sodium sulfate, filtered, vacuum-concentrated until dryness, and purified through silica gel chromatography (PE/EA: 1:1) to obtain the compound 1 (23 mg, yield: 50%), LC/MS (ESI): *m/z* 459[M+1]+; and $^1$H NMR (400 MHz, DMSO-d6): δ10.22(s, 1H), 9.88(d, 1H), 9.03(t, 1H), 9.00(dt, 1H), 8.82(dd, 1H), 8.66(d, 2H), 8.56(s, 1H), 7.96(d, 2H), 4.34-4.37(m, 1H), 3.40-3.48(m, 1H), 3.28-3.35(m, 1H), 3.16-3.23(m, 2H), 1.14-1.20(m, 6H), 0.98(d, *J*=6.8Hz, 3H).

The compounds in Table 1 below could be prepared by a process similar to the process in Example 1.

Table 1

| Compound | Raw material III (acid)/IV (hydrazine) | Structural formula of a compound | LC/MS (ESI) and $^1$H NMR (400 MHz) (δ) |
|---|---|---|---|
| | | | |
| 2 | III-B/IV-2 | 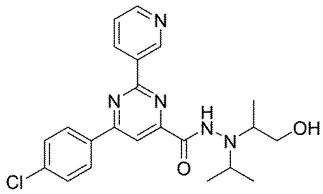 | LC/MS: *m/z* 426 [M+1]+. $^1$H NMR (400 MHz, DMSO-d6): δ10.20(s,1H), 9.88(d,1H), 8.94-9.05(m, 2H), 8.80(dd, 1H), 8.48-8.54(m, 3H), 7.62 -7.72 (m, 2H), 4.34-4.37(m, 1H), 3.41-3.48(m, 1H), 3.28-3.35 (m, 1H), 3.17-3.23(m, 2H), 1.14-1.21 (m, 6H), 0.99(d, *J*=6.8Hz, 3H). |
| 3 | III-C/IV-7 | 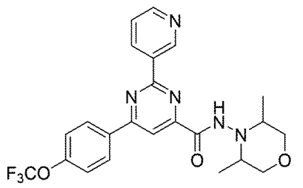 | LC/MS: *m/z* 474 [M+1]+. |

(continued)

| Compound | Raw material III (acid)/IV (hydrazine) | Structural formula of a compound | LC/MS (ESI) and $^{1}$H NMR (400 MHz) (δ) |
|---|---|---|---|
| 4 | III-A/IV-6(S) | 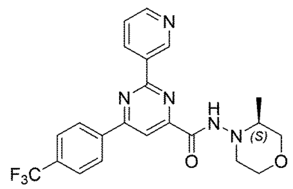 | LC/MS: *m/z* 444[M+1]$^{+}$. $^{1}$H NMR (400 MHz, DMSO-d6): δ10.06(s,1H), 9.95 (d,*J*=1.6Hz, 1H), 9.07(ddd, *J*=1.6, 2.0,7.6Hz, 1H), 8.82 (dd,*J*=1.2,4.8Hz, 1H), 8.65(d, *J*=8.0Hz, 2H), 8.53(s, 1H), 7.97 (d,*J*=8.0Hz,2H), 7.66(dd, *J*=7.6,4.8Hz,1H), 3.86 (d, *J*=11.2Hz, 1H), 3.80(d, *J*=8.0Hz, 1H), 3.64(dt, *J=1.6,* 11.2Hz,1H), 3.17-3.25 (m,3H), 2.94(d, *J*=10.4Hz,1H), 0.92(d, *J*=8.0Hz, 3H). |
| 5 | III-A/IV-6(R) | 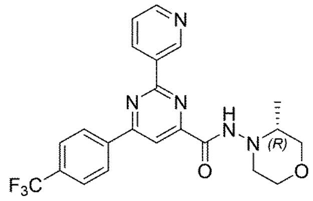 | LC/MS: *m/z* 444[M+1]$^{+}$. $^{1}$H NMR (400 MHz, DMSO-d6): δ10.08(s,1H), 9.95(d, *J*=1.6Hz,1H), 9.07(ddd, *J=1.6,* 2.0, 7.6Hz, 1H), 8.82(dd, *J*=1.2,4.8Hz,1H), 8.65(d, *J*=8.0Hz,2H), 8.53(s,1H), 7.97 (d, *J*=8.0Hz,2H), 7.66(dd, *J*=7.6,4.8Hz,1H), 3.87(d,J=11.2Hz, 1H), 3.80(d, *J*=8.0Hz,1H), 3.64(dt,*J*=1.6, 11.2Hz, 1H), 3.17-3.25 (m,3H), 2.94(d, *J*=10.4Hz, 1H), 0.93(d, *J*=8.0Hz, 3H). |
| 6 | III-C/IV-6(S) | 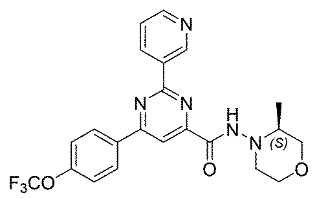 | LC/MS: *m/z* 460[M+1]$^{+}$. |
| 7 | III-C/IV-6(R) | 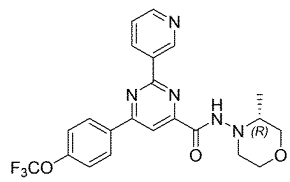 | LC/MS: *m/z* 460[M+1]$^{+}$. |
| 8 | III-B/IV-6(S) | 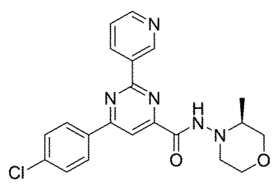 | LC/MS: *m/z* 410[M+1]$^{+}$. |
| 9 | III-B/IV-6(R) | 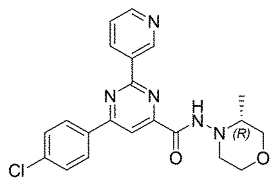 | LC/MS: *m/z* 410[M+1]$^{+}$. |

(continued)

| Compound | Raw material III (acid)/IV (hydrazine) | Structural formula of a compound | LC/MS (ESI) and $^1H$ NMR (400 MHz) ($\delta$) |
|---|---|---|---|
| 10 | III-A/IV (isopropylhydra-zine) | 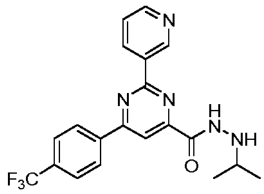 | LC/MS: *m/z* 402[M+1]$^+$. |
| 11 | III-C/IV-10 | 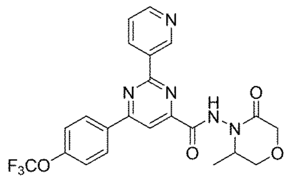 | LC/MS: *m/z* 474[M+1]$^+$. |
| 12 | III-A/IV-12 | 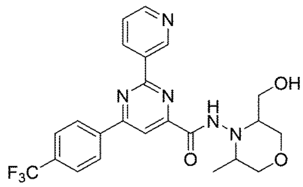 | LC/MS: *m/z* 474[M+1]$^+$. |
| 13 | III-A/IV (1-hydroxyisopro-pylhydr azine) | 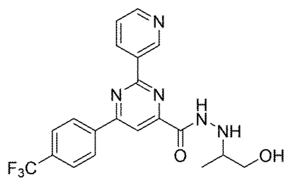 | LC/MS: *m/z* 418[M+1]$^+$. |
| 14 | III-C/IV-2 | 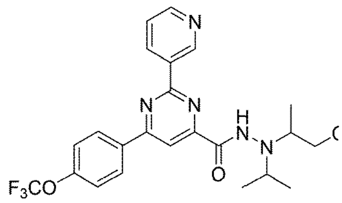 | LC/MS: *m/z* 476[M+1]$^+$. $^1H$ NMR (400 MHz, DMSO-d6): $\delta$10.25(s,1H), 9.93(d, 1H), 9.17(d, 1H), 9.04(dt, 1H), 8.82(dd, 1H), 8.56-8.63(m, 2H), 8.50(s, 1H), 7.61(d, 2H), 4.34 -4.37(m, 1H), 3.41-3.49(m, 1H), 3.28-3.36(m, 1H), 3.15-3.22(m, 2H), 1.14-1.20(m, 6H), 0.97(d, *J*=6.8Hz, 3H). |
| 15 | III-H/IV-6(S) | 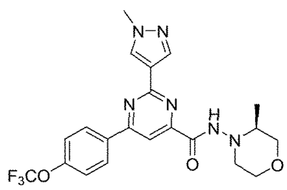 | LC/MS: *m/z* 463 [M+1]$^+$. |
| 16 | III-H/IV-6(R) | 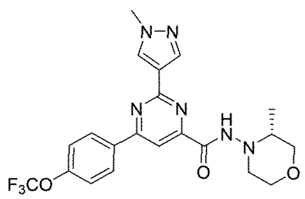 | LC/MS: *m/z* 463 [M+1]$^+$. |

(continued)

| Compound | Raw material III (acid)/IV (hydrazine) | Structural formula of a compound | LC/MS (ESI) and 1H NMR (400 MHz) (δ) |
|---|---|---|---|
| 17 | III-B/IV-3 | 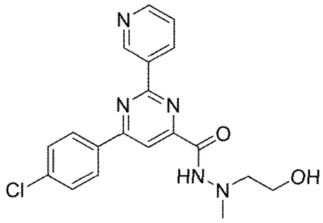 | LC/MS: *m/z* 384 $[M+1]^+$. $^1H$ NMR (400 MHz, DMSO-d6): 10.15(s, 1H),9.89(d, 1H), 8.94-9.04(m,2H),8.80(dd,1 H), 8.48-8.54(m,3H), 7.62-7.73 (m,2H), 3.85(t, *J*= 6.4 Hz, 1H), 3.62(m, 2H), 2.89(d, *J*= 4.2 Hz, 2H), 2.81(s, 3H). |
| 18 | III-I/IV-3 | 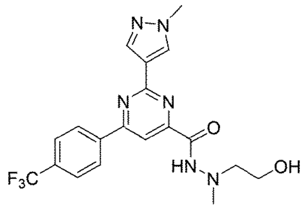 | LC/MS: *m/z* 421 $[M+1]^+$. |
| 19 | III-B/IV-5 | 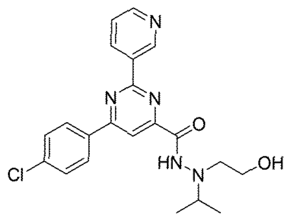 | LC/MS: *m/z* 412 [M+1]+. |
| 20 | III-I/IV-5 | 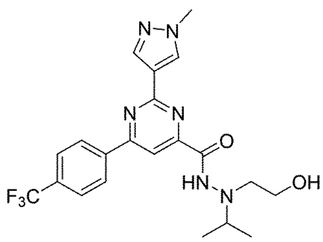 | LC/MS: *m/z* 449 $[M+1]^+$. |
| 21 | III-B/IV-4 | 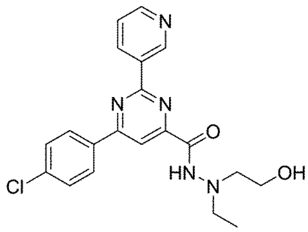 | LC/MS: *m/z* 398 $[M+1]^+$. $^1H$ NMR (400 MHz, DMSO-d6): δ10.22(s,1H),9.83(d,1H), 8.92-9.05(m,2H), 8.81(dd,1H), 8.48-8.56(m,3H), 7.62-7.72 (m,2H), 3.62(t, *J*=5.2Hz, 2H), 2.92-2.99(m, 2H), 2.87-2.93(m, 2H), 1.18(t, *J*=7.2Hz, 3H). |
| 22 | III-B/IV-9 | 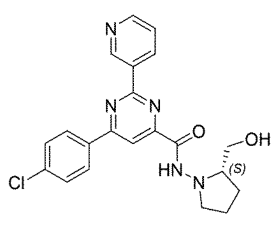 | LC/MS: *m/z* 410 $[M+1]^+$. $^1H$ NMR (400 MHz, DMSO-d6): δ10.30(s,1H),9.85(d,1H), 8.92-9.07(m,2H),8.80(dd,1 H), 8.47-8.56(m,3H), 7.61-7.72 (m,2H), 3.95(dd, *J*=4.2, 7.6Hz, 1H), 3.64(dt, *J* =12.4, 3.6Hz, 1H), 3.41-3.52(m, 2H), 2.86-2.91(m, 2H), 1.85-2.01(m, 4H). |

(continued)

| Compound | Raw material III (acid)/IV (hydrazine) | Structural formula of a compound | LC/MS (ESI) and $^1H$ NMR (400 MHz) (δ) |
|---|---|---|---|
| 23 | III-A/IV-8 | 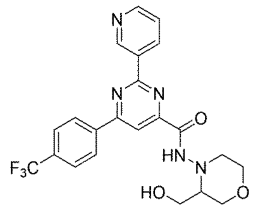 | LC/MS: *m/z* 460 $[M+1]^+$. |
| 24 | III-A/N-aminomorpholin e | 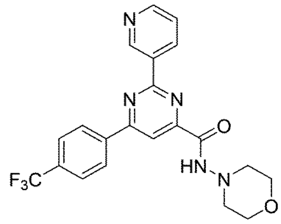 | LC/MS: *m/z* 430 $[M+1]^+$. |
| 25 | III-A/N-aminothiomorph oline 1,1-dioxide | 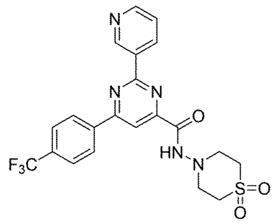 | LC/MS: *m/z* 478 $[M+1]^+$. |
| 26 | III-B/IV-10 | 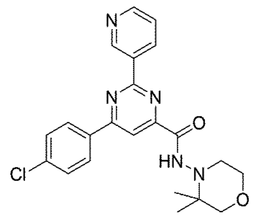 | LC/MS: *m/z* 424 [M+1]+. |
| 27 | III-A/IV-10 | 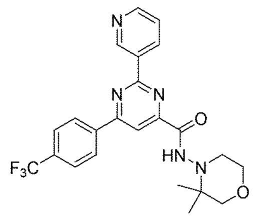 | LC/MS: *m/z* 458 $[M+1]^+$. |
| 28 | III-B/IV-11 | 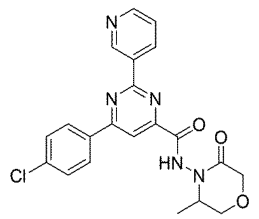 | LC/MS: *m/z* 424 $[M+1]^+$. |
| 29 | III-B/IV-12 | 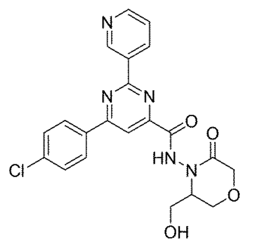 | LC/MS: *m/z* 440 $[M+1]^+$. |

(continued)

| Compound | Raw material III (acid)/IV (hydrazine) | Structural formula of a compound | LC/MS (ESI) and $^1$H NMR (400 MHz) (δ) |
|---|---|---|---|
| 30 | III-A/IV-14 | 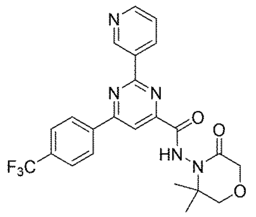 | LC/MS: *m/z* 472[M+1]$^+$. |
| 31 | III-A/IV-15 | 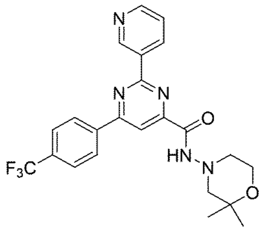 | LC/MS: *m/z* 458 [M+1]$^+$. |
| 32 | III-B/IV-1 | 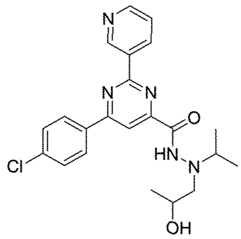 | LC/MS: *m/z* 426 [M+1]$^+$. |
| 33 | III-A/pyrazin-2-hydrazin e | 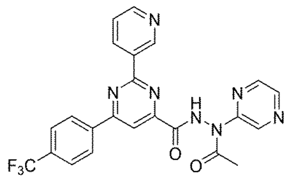 | LC/MS: *m/z* 480[M+1]$^+$. |
| 34 | III-J/pyrazin-2-hydrazine | 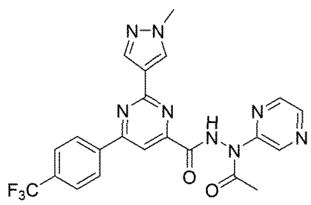 | LC/MS: *m/z* 483[M+1]$^+$. |
| 35 | III-I/IV-17 | 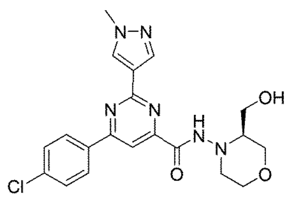 | LC/MS: *m/z* 429[M+1]$^+$. |
| 36 | III-I/IV-16 | 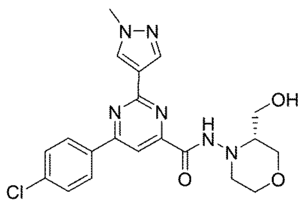 | LC/MS: *m/z* 429[M+1]$^+$. |

(continued)

| Compound | Raw material III (acid)/IV (hydrazine) | Structural formula of a compound | LC/MS (ESI) and $^1$H NMR (400 MHz) (δ) |
|---|---|---|---|
| 37 | III-Y/IV-2 | 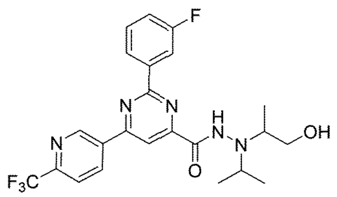 | LC/MS: *m/z* 478[M+1]$^+$. |
| 38 | III-J/IV-6(R) | 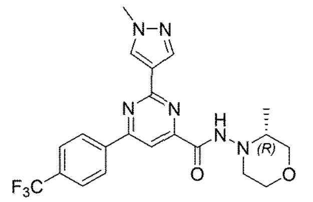 | LC/MS: *m/z* 447[M+1]$^+$. |
| 39 | III-J/IV-6(S) | 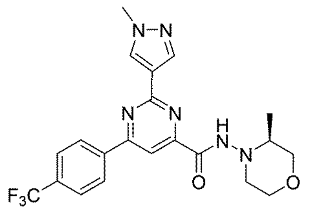 | LC/MS: *m/z* 447[M+1]$^+$. |
| 40 | III-I/IV-6(R) | 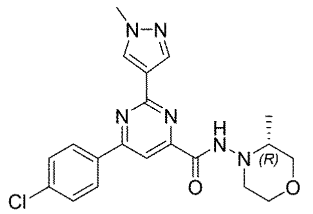 | LC/MS: *m/z* 413[M+1]$^+$. |
| 41 | III-I/IV-6(S) | 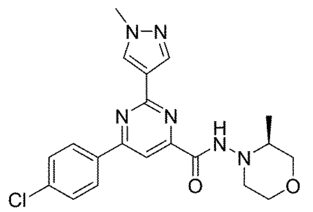 | LC/MS: *m/z* 413[M+1]$^+$. |
| 42 | III-A/IV-16(R) | 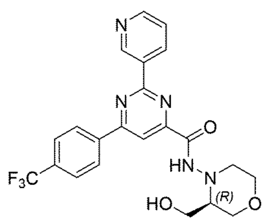 | LC/MS: *m/z* 460[M+1]$^+$. |
| 43 | III-A/IV-16(S) | 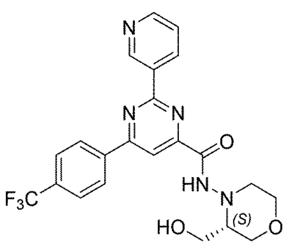 | LC/MS: *m/z* 460[M+1]$^+$. |

(continued)

| Compound | Raw material III (acid)/IV (hydrazine) | Structural formula of a compound | LC/MS (ESI) and 1H NMR (400 MHz) (δ) |
|---|---|---|---|
| 44 | III-J/IV-16(S) | 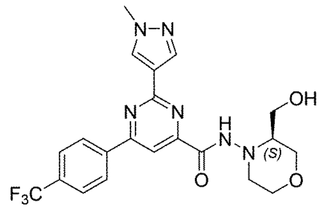 | LC/MS: *m/z* 463[M+1]+. |
| 45 | III-J/IV-16(R) | 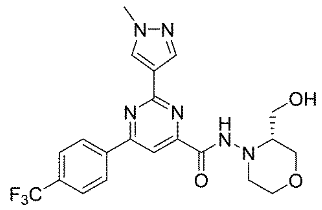 | LC/MS: *m/z* 463[M+1]+. |
| 46 | III-B/IV-16(S) | 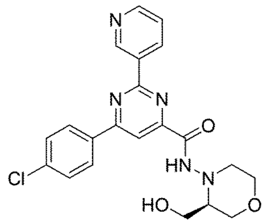 | LC/MS: *m/z* 426[M+1]+. |
| 47 | III-B/IV-16(R) | 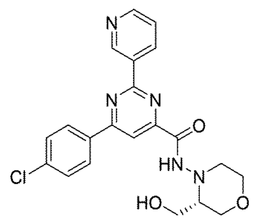 | LC/MS: *m/z* 426[M+1]+. |
| 48 | III-D/IV-2 | 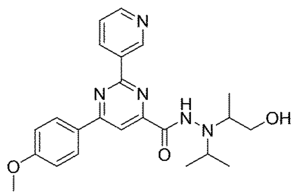 | LC/MS: *m/z* 422[M+1]+. |
| 49 | III-E/IV-2 | 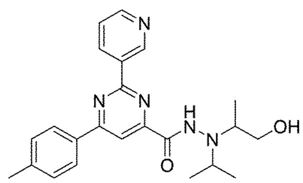 | LC/MS: *m/z* 406[M+1]+. |
| 50 | III-F/IV-2 | 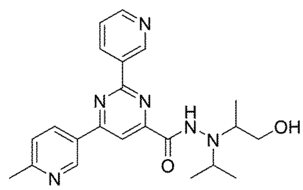 | LC/MS: *m/z* 407[M+1]+. |

(continued)

| Compound | Raw material III (acid)/IV (hydrazine) | Structural formula of a compound | LC/MS (ESI) and $^1$H NMR (400 MHz) (δ) |
|---|---|---|---|
| 51 | III-G/IV-2 | 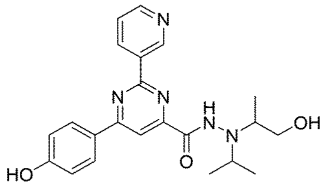 | LC/MS: *m/z* 408[M+1]$^+$. |
| 52 | III-K/IV-2 | 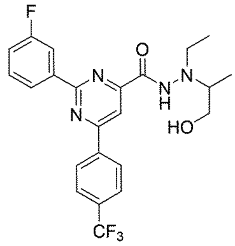 | LC/MS: *m/z* 463[M+1]$^+$. |
| 53 | III-L/IV-2 | 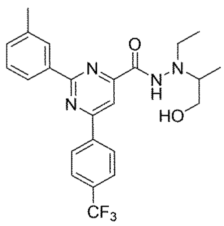 | LC/MS: *m/z* 459[M+1]$^+$. |
| 54 | III-M/IV-2 | 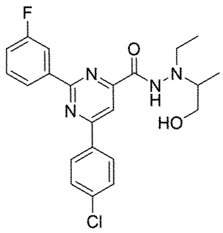 | LC/MS: *m/z* 429[M+1]$^+$. |
| 55 | III-N/IV-2 | 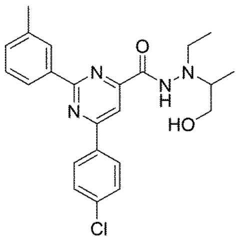 | LC/MS: *m/z* 425[M+1]$^+$. |
| 56 | III-O/IV-2 | 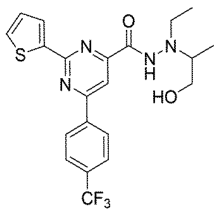 | LC/MS: *m/z* 451[M+1]$^+$. |
| 57 | III-P/IV-2 | 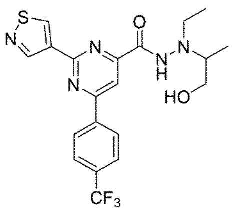 | LC/MS: *m/z* 452[M+1]$^+$. |

(continued)

| Compound | Raw material III (acid)/IV (hydrazine) | Structural formula of a compound | LC/MS (ESI) and $^1$H NMR (400 MHz) ($\delta$) |
|---|---|---|---|
| 58 | III-Q/IV-2 | 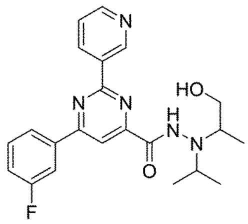 | LC/MS: *m/z* 410[M+1]$^+$. |
| 59 | III-R/IV-2 | 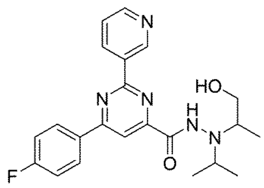 | LC/MS: *m/z* 410[M+1]$^+$. |
| 60 | III-S/IV-2 | 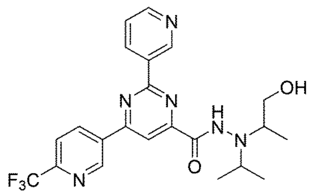 | LC/MS: *m/z* 461[M+1]$^+$. |
| 61 | III-T/IV-2 | 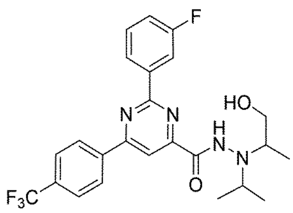 | LC/MS: *m/z* 477[M+1]$^+$. |
| 62 | III-U/IV-2 | 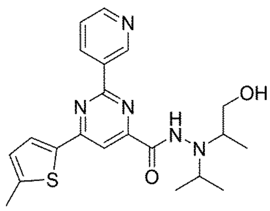 | LC/MS: *m/z* 412[M+1]$^+$. |
| 63 | III-V/IV-2 | 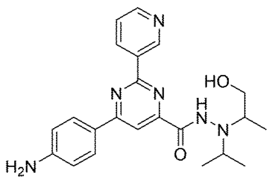 | LC/MS: *m/z* 407[M+1]$^+$. |
| 64 | III-W/IV-2 | 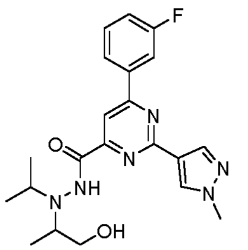 | LC/MS: *m/z* 478[M+1]$^+$. |

(continued)

| Compound | Raw material III (acid)/IV (hydrazine) | Structural formula of a compound | LC/MS (ESI) and $^1$H NMR (400 MHz) (δ) |
|---|---|---|---|
| 65 | III-Y/IV-2 | 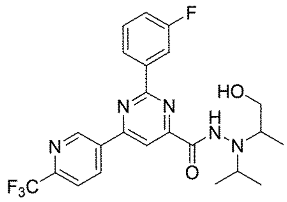 | LC/MS: *m/z* 478[M+1]$^+$. |

**Example 13: Preparation of compound 66**

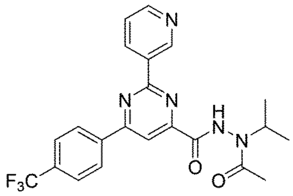

The compound 10 (40.2 mg, 0.1 mmol) was dissolved in 5 mL of DCM, and 0.1 mL of DIPEA was added, followed by cooling to 0°C. 0.06 mL of acetic anhydride was added dropwise, followed by stirring overnight at room temperature, washing with water three times, drying with anhydrous sodium sulfate, filtration, vacuum concentration until dryness, and purification through silica gel column chromatography (PE/EA: 1:1) to obtain the compound **66,** with a yield of 64%, LC/MS (ESI): *m/z* 444 [M+1]$^+$.$^1$H NMR (400 MHz, DMSO-d6): δ10.08(s,1H), 9.95 (d, *J*=1.6Hz, 1H), 9.07(ddd, *J*=1.6, 2.0, 7.6Hz, 1H), 8.82 (dd, *J*=1.2, 4.8Hz, 1H), 8.65(d, *J*=8.0Hz, 2H), 8.53(s, 1H), 7.97 (d,*J*=8.0Hz, 2H), 7.66(dd, *J*=7.6, 4.8Hz, 1H), 3.18-3.27 (m, 1H), 2.08(s, 3H), 1.18(d, *J*= 6.4Hz, 6H).

**Example 14: Preparation of compound 67**

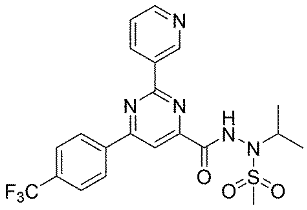

The compound 67 was prepared by a process similar to the process in Example 13. The compound **10** was allowed to react with methanesulfonyl chloride to obtain the compound **67,** with a yield of 41%, LC/MS (ESI): *m/z* 480 [M+1]$^+$; and $^1$H NMR (400 MHz, DMSO-d6): δ10.20(s,1H), 9.96 (d, *J*=1.6Hz, 1H), 9.067(ddd, *J*=1.6, 2.0, 7.6Hz, 1H), 8.82 (dd, *J*=1.2, 4.8Hz, 1H), 8.65(d, *J*=8.0Hz, 2H), 8.53(s, 1H), 7.97 (d,J=8.0Hz, 2H), 7.66(dd, *J*=7.6, 4.8Hz, 1H), 3.18-3.27 (m, 1H), 2.92(s, 3H), 1.18(d, *J*= 6.4Hz, 6H).

**Example 15: Preparation of compound 68**

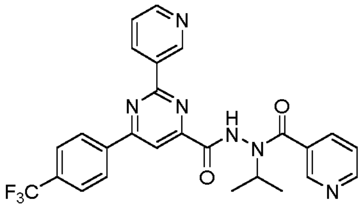

The compound 68 was prepared by a process similar to the process in Example 13. The compound **10** was allowed to react with nicotinoyl chloride hydrochloride to obtain the compound 68, with a yield of 84%, LC/MS (ESI): *m/z* 507 $[M+1]^+$.

**Effect Example 1: Testing of an antagonistic effect of each compound for AHR in a human cell line**

A human liver cancer cell line HepG2 endogenously expressing an AHR protein (provided by the Stem Cell Bank of the Chinese Academy of Sciences) was adopted for testing. The expression of a CYP1A1 gene is regulated by an activity of AHR. After being activated, AHR can bind to a sequence called XRE on a CYP1A1 enhancer and initiate the expression of the CYP1A1 gene. To quantitatively determine an impact of a compound on the activity of AHR, a 1,200 bp (-1,143 to +57) regulatory sequence upstream of a promoter of the CYP1A1 gene was synthesized through gene synthesis and then inserted into a plasmid vector carrying firefly luciferase. The plasmid was then linearized through single enzyme cleavage and then transfected into HepG2 cells through lipofectin transfection (Lipofectamine 3000), and transfected HepG2 cells were screened by puromycin to obtain a stably-expressed cell line. When an antagonistic effect of a compound for AHR was tested, the expression of firefly luciferase was initiated by an AHR agonist (such as L-kynurenine, kynurenic acid, indirubin, TCDD, and 2-(1H-indol-3-ylcarbonyl)-4-thiazolecarboxylic acid methyl ester (ITE)), and cells were treated with the compound at different concentrations. Then, the inhibition on the expression of luciferase by the compound was measured.

Stably-transfected HepG2 cells were sub-cultivated in Minimum Eagle's medium (MEM) at 37°C and 5% carbon dioxide. In the MEM, 1 mM sodium pyruvate, 2 mM GlutaMax, 1 × non-essential amino acids, 10% Australian fetal bovine serum, 100 U/mL penicillin-streptomycin, and 5 μg/mL puromycin were supplemented. Before testing, HepG2 cells were inoculated into a 96-well plate at a density of $5\text{-}8 \times 10^4$ cells/well and cultivated for 24 h to 48 h to allow a cell confluency of about 90% before a drug treatment (puromycin was not added in a medium). Before the drug treatment, AHR agonist-containing 2× compound solutions at different concentrations were prepared with a same medium, and then a half of a cell culture medium in the 96-well plate was replaced with a 2× compound solution. An AHR agonist solution including only DMSO at a corresponding concentration was set as a negative control group, and CH223191 at a corresponding concentration (commercially available) was set as a positive control group. 2 separate replicate tests were set for each compound (including a positive control and a negative control). 24 h after the compound treatment, HepG2 cells were lysed by a Steady-Glo luciferase detection system of Promega, and a luciferase signal was measured by an InfiniteM1000 Pro microplate reader of Tecan.

In terms of data processing, for each replicate test of each compound, inhibition percentages of a compound at different concentrations on an activity of AHR were calculated sequentially with a signal of a well at a compound concentration of 0 as 100% and a cell signal of an untreated agonist as 0. Corresponding $IC_{50}$ of a compound was calculated with an average value of data of two replicates according to the formula $Y = \frac{100}{1+\frac{x}{IC50}}$ through non-linear fitting, where y represents an inhibition percentage and x represents a concentration of a corresponding compound.

Reference: Buckley, S. M. K. et al. In vivo bioimaging with tissue-specific transcription factor activated luciferase reporters. Sci. Rep.5: 11842 (2015).

$IC_{50}$ for each compound was shown in Table 2, where A indicates $IC_{50} \leq 500$ nM, B indicates 500 nM < $IC_{50} \leq 1.0$ μM, C indicates 1 μM < $IC_{50} \leq 5.0$ μM, and D indicates $IC_{50} > 5.0$ μM.

Table 2 $IC_{50}$ for each compound

| Compound | $IC_{50}$ (nM) | Compound | $IC_{50}$ (nM) |
|---|---|---|---|
| 1 | A | 35 | A |
| 2 | A | 36 | A |
| 3 | A | 37 | A |
| 4 | A | 38 | B |
| 5 | A | 39 | A |

(continued)

| Compound | $IC_{50}$ (nM) | Compound | $IC_{50}$ (nM) |
|---|---|---|---|
| 6 | A | 40 | A |
| 7 | A | 41 | A |
| 8 | A | 42 | A |
| 9 | A | 43 | A |
| 10 | A | 44 | A |
| 11 | A | 45 | A |
| 12 | A | 46 | A |
| 13 | A | 47 | A |
| 14 | A | 48 | B |
| 15 | A | 49 | A |
| 16 | A | 50 | B |
| 17 | B | 51 | B |
| 18 | A | 52 | A |
| 19 | A | 53 | B |
| 20 | A | 54 | A |
| 21 | A | 55 | B |
| 22 | B | 56 | A |
| 23 | A | 57 | A |
| 24 | B | 58 | B |
| 25 | B | 59 | B |
| 26 | A | 60 | B |
| 27 | A | 61 | A |
| 28 | B | 62 | B |
| 29 | A | 63 | B |
| 30 | A | 64 | C |
| 31 | B | 65 | B |
| 32 | A | 66 | A |
| 33 | A | 67 | A |
| 34 | A | 68 | A |

It can be seen from Table 2 that the above compounds can bind to AHR and inhibit those functions and signaling pathways controlled by AHR, thereby affecting the growth and proliferation of cancer cells and the invasiveness of tumor cells. Therefore, the compound shown in the formula I of the present disclosure can be used to inhibit the growth of cancer cells and inhibit the metastasis and invasion of tumor cells.

## Claims

1. A substituted pyrimidinyl hydrazide compound shown in the following formula I and an optical isomer, prodrug, or pharmaceutically-acceptable salt thereof:

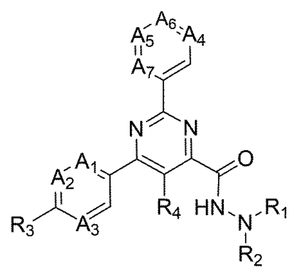

formula I

wherein

$A_1$, $A_2$, and $A_3$ each are independently $CR_A$ or N, a substituent $R_A$ is independently selected from hydrogen, deuterium, halogen, cyano, hydroxyl, amino, saturated or unsaturated $C_1$-$C_8$ alkyl, $C_3$-$C_8$ cycloalkyl, saturated or unsaturated $C_1$-$C_8$ alkyl substituted with halogen, hydroxyl, amino, or $R_C$, $C_3$-$C_8$ cycloalkyl substituted with halogen, hydroxyl, amino, or $R_C$, saturated or unsaturated $C_1$-$C_8$ alkoxy, $C_3$-$C_8$ cycloalkoxy, saturated or unsaturated $C_1$-$C_8$ alkoxy substituted with halogen, hydroxyl, amino, or $R_C$, $C_3$-$C_8$ cycloalkoxy substituted with halogen, hydroxyl, amino, or $R_C$, -$S(O)_n$Rc, saturated or unsaturated substituted or unsubstituted $C_1$-$C_8$ acyl, and $NR_CR_D$,

$A_4$ is O, S, N, C, or a bond, and $A_5$, $A_6$, and $A_7$ each are independently O, S, N, or C;

when $A_4$, $A_5$, $A_6$, and $A_7$ are N or C, $A_4$, $A_5$, $A_6$, and $A_7$ each are independently substituted with $R_B$, $R_B$ is independently selected from hydrogen, deuterium, halogen, cyano, hydroxyl, -$S(O)_n$Rc, saturated or unsaturated $C_1$-$C_8$ alkyl, $C_3$-$C_8$ cycloalkyl, saturated or unsaturated $C_1$-$C_8$ alkyl substituted with halogen, hydroxyl, amino, or $R_C$, $C_3$-$C_8$ cycloalkyl substituted with halogen, hydroxyl, amino, or $R_C$, saturated or unsaturated $C_1$-$C_8$ alkoxy, $C_3$-$C_8$ cycloalkoxy, saturated or unsaturated $C_1$-$C_8$ alkoxy substituted with halogen, hydroxyl, amino, or $R_C$, $C_3$-$C_8$ cycloalkoxy substituted with halogen, hydroxyl, amino, or $R_C$, saturated or unsaturated substituted or unsubstituted $C_1$-$C_8$ acyl, saturated or unsaturated substituted or unsubstituted $C_1$-$C_8$ alkoxycarbonyl, saturated or unsaturated substituted or unsubstituted $C_1$-$C_8$ alkylaminocarbonyl, and $NR_CR_D$,

substituents $R_C$ and $R_D$ each are independently selected from hydrogen, halogen, cyano, hydroxyl, amino, saturated or unsaturated $C_1$-$C_6$ alkyl, $C_3$-$C_6$ cycloalkyl, saturated or unsaturated $C_1$-$C_6$ alkyl substituted with halogen, hydroxyl, -$S(O)_nR_E$, or $NR_FR_G$, $C_3$-$C_6$ cycloalkyl substituted with halogen, hydroxyl, -$S(O)_nR_E$, or $NR_FR_G$, saturated or unsaturated $C_1$-$C_6$ alkoxy, $C_3$-$C_6$ cycloalkoxy, saturated or unsaturated $C_1$-$C_6$ alkoxy substituted with halogen, hydroxyl, -$S(O)_nR_E$, or $NR_FR_G$, and $C_3$-$C_6$ cycloalkoxy substituted with halogen, hydroxyl, -$S(O)_nR_E$, or $NR_FR_G$, and $R_E$, $R_F$, and $R_G$ each are independently selected from hydrogen, deuterium, halogen, cyano, hydroxyl, amino, saturated or unsaturated $C_1$-$C_6$ alkyl, $C_3$-$C_6$ cycloalkyl, saturated or unsaturated $C_1$-$C_6$ alkyl substituted with halogen, hydroxyl, or amino, $C_3$-$C_6$ cycloalkyl substituted with halogen, hydroxyl, or amino, saturated or unsaturated $C_1$-$C_6$ alkoxy, $C_3$-$C_6$ cycloalkoxy, saturated or unsaturated $C_1$-$C_6$ alkoxy substituted with halogen, hydroxyl, or amino, and $C_3$-$C_6$ cycloalkoxy substituted with halogen, hydroxyl, or amino;

$R_1$ and $R_2$ each are independently hydrogen, saturated or unsaturated substituted or unsubstituted $C_1$-$C_8$ alkyl, saturated or unsaturated substituted or unsubstituted $C_3$-$C_8$ cycloalkyl, -OC(=O)$C_{1\text{-}8}$ alkyl, -OC(=O)$C_3$-$C_8$ cycloalkyl, -C(=O)O$C_{1\text{-}8}$ alkyl, -C(=O)O$C_3$-$C_8$ cycloalkyl, -$S(O)_n$Rc, saturated or unsaturated substituted or unsubstituted $C_1$-$C_8$ sulfonyl, saturated or unsaturated substituted or unsubstituted $C_1$-$C_8$ acyl, $C_6$-$C_{14}$ aryl, and 4 to 14-membered heterocycloalkyl or heteroaryl with 1 to 3 heteroatoms selected from N, O, and S, wherein a "substitution" refers to selective containing of 1 to 4 substituents selected from hydroxyl, halogen, cyano, sulfonyl, amino, -$S(O)_n$Rc, $NR_CR_D$, saturated or unsaturated $C_1$-$C_8$ alkyl, saturated or unsaturated $C_3$-$C_8$ cycloalkyl, saturated or unsaturated $C_1$-$C_8$ alkoxy, saturated or unsaturated $C_3$-$C_8$ cycloalkoxy, -OC(=O)$C_{1\text{-}8}$ alkyl, -OC(=O) $C_3$-$C_8$ cycloalkyl, -C(=O)O$C_{1\text{-}8}$ alkyl, -C(=O)O$C_3$-$C_8$ cycloalkyl, saturated or unsaturated $C_1$-$C_8$ sulfonyl, saturated or unsaturated $C_1$-$C_8$ acyl, saturated or unsaturated $C_1$-$C_8$ alkoxycarbonyl, $C_6$-$C_{14}$ aryl, and 4 to 14-membered heterocycloalkyl or heteroaryl with 1 to 3 heteroatoms selected from N, O, and S; or

$R_1$ and $R_2$, together with N atoms attached thereto, produce substituted or unsubstituted 4 to 14-membered heterocycloalkyl with 1 to 3 heteroatoms selected from N, O, and S or substituted or unsubstituted 5 to 14-membered heteroaryl with 1 to 3 heteroatoms selected from N, O, and S, wherein a "substitution" refers to selective containing of 1 to 4 substituents selected from deuterium, hydroxyl, halogen, cyano, sulfonyl, amino, -$S(O)_n$Rc, $NR_CR_D$, $C_1$-$C_8$ alkyl, $C_3$-$C_8$ cycloalkyl, $C_1$-$C_8$ alkoxy, $C_3$-$C_8$ cycloalkoxy, -OC(=O)$C_{1\text{-}8}$ alkyl, -OC(=O) $C_3$-$C_8$ cycloalkyl, -C(=O)O$C_{1\text{-}8}$ alkyl, -C(=O)O$C_3$-$C_8$ cycloalkyl, $C_1$-$C_8$ alkyl substituted with halogen, hydroxyl, amino, or $R_H$, $C_3$-$C_8$ cycloalkyl, $C_1$-$C_8$ alkoxy, and $C_3$-$C_8$ cycloalkoxy, wherein $R_H$ is selected from hydrogen, deuterium, halogen, cyano, hydroxyl, amino, saturated or unsaturated $C_1$-$C_6$ alkyl, $C_3$-$C_6$ cycloalkyl, -$S(O)_n$Rc, $NR_CR_D$, saturated or unsaturated $C_1$-$C_6$ alkyl substituted with deuterium, halogen, hydroxyl, or amino, $C_3$-$C_6$ cycloalkyl substituted with deuterium, halogen, hydroxyl, or amino, saturated or unsaturated $C_1$-$C_6$ alkoxy, $C_3$-$C_6$ cycloalkoxy, saturated or unsaturated $C_1$-$C_6$ alkoxy substituted with deuterium, halogen, hydroxyl, or amino, and $C_3$-$C_6$ cycloalkoxy substituted with deuterium, halogen, hydroxyl, or amino;

$R_3$ is halogen, hydroxyl, -$S(O)_n$Rc, $NR_CR_D$, saturated or unsaturated $C_1$-$C_8$ alkyl, $C_3$-$C_8$ cycloalkyl, $C_1$-$C_8$ alkoxy, $C_3$-$C_8$ cycloalkoxy, saturated or unsaturated $C_1$-$C_8$ alkyl substituted with halogen, hydroxyl, amino, or $R_H$, $C_3$-$C_8$ cycloalkyl, $C_1$-$C_8$ alkoxy, and $C_3$-$C_8$ cycloalkoxy, wherein the substituent $R_H$ is selected from hydrogen, deuterium, halogen, cyano, hydroxyl, amino, saturated or unsaturated $C_1$-$C_6$ alkyl, $C_3$-$C_6$ cycloalkyl, saturated or unsaturated $C_1$-$C_6$ alkyl substituted with halogen, hydroxyl, or amino, $C_3$-$C_6$ cycloalkyl substituted with halogen, hydroxyl, or amino, saturated or unsaturated $C_1$-$C_6$ alkoxy, $C_3$-$C_6$ cycloalkoxy, saturated or unsaturated

$C_1$-$C_6$ alkoxy substituted with halogen, hydroxyl, or amino, and $C_3$-$C_6$ cycloalkoxy substituted with halogen, hydroxyl, or amino;
R4 is hydrogen or deuterium; and
n is an integer of 0, 1, or 2.

**2.** The substituted pyrimidinyl hydrazide compound shown in the formula I and the optical isomer, prodrug, or pharmaceutically-acceptable salt thereof according to claim 1, wherein
$A_1$ and $A_3$ each are $CR_A$, $A_2$ is N, and the substituent $R_A$ is independently selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, amino, or $R_C$, $C_3$-$C_6$ cycloalkyl substituted with halogen, hydroxyl, amino, or $R_C$, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, amino, or $R_C$, $C_3$-$C_6$ cycloalkoxy substituted with halogen, hydroxyl, amino, or $R_C$, $-S(O)_nRc$, saturated or unsaturated substituted or unsubstituted $C_1$-$C_3$ acyl, and $NR_CR_D$.

**3.** The substituted pyrimidinyl hydrazide compound shown in the formula I and the optical isomer, prodrug, or pharmaceutically-acceptable salt thereof according to claim 1, wherein $A_1$ and $A_3$ each are $CR_A$, $A_2$ is N, and the substituent $R_A$ is hydrogen.

**4.** The substituted pyrimidinyl hydrazide compound shown in the formula I and the optical isomer, prodrug, or pharmaceutically-acceptable salt thereof according to claim 1, wherein $A_4$ is N, C, or the bond, and $A_5$, $A_6$, and $A_7$ each are independently N or C,
$A_4$, $A_5$, $A_6$, and $A_7$ each are independently linked to the substituent $R_B$, and the substituent $R_B$ is independently selected from hydrogen, halogen, cyano, hydroxyl, amino, $-S(O)_nRc$, saturated or unsaturated $C_1$-$C_3$ alkyl, $C_3$-$C_6$ cycloalkyl, saturated or unsaturated $C_1$-$C_3$ alkyl substituted with halogen, hydroxyl, amino, or $R_C$, $C_3$-$C_6$ cycloalkyl substituted with halogen, hydroxyl, amino, or $R_C$, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, amino, or $R_C$, $C_3$-$C_6$ cycloalkoxy substituted with halogen, hydroxyl, amino, or R', saturated or unsaturated substituted or unsubstituted $C_1$-$C_3$ acyl, saturated or unsaturated substituted or unsubstituted $C_1$-$C_3$ alkoxycarbonyl, and $NR_CR_D$.

**5.** The substituted pyrimidinyl hydrazide compound shown in the formula I and the optical isomer, prodrug, or pharmaceutically-acceptable salt thereof according to claim 1, wherein

the substituents $R_C$ and $R_D$ each are independently selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, $-S(O)_nR_E$, or $NR_FR_G$, $C_3$-$C_6$ cycloalkyl substituted with halogen, hydroxyl, $-S(O)_nR_E$, or $NR_FR_G$, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, $-S(O)_nR_E$, or $NR_FR_G$, and $C_3$-$C_6$ cycloalkoxy substituted with halogen, hydroxyl, $-S(O)_nR_E$, or $NR_FR_G$; and
$R_E$, $R_F$, and $R_G$ each are independently selected from hydrogen, deuterium, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

**6.** The substituted pyrimidinyl hydrazide compound shown in the formula I and the optical isomer, prodrug, or pharmaceutically-acceptable salt thereof according to claim 1, wherein $A_7$ is C.

**7.** The substituted pyrimidinyl hydrazide compound shown in the formula I and the optical isomer, prodrug, or pharmaceutically-acceptable salt thereof according to claim 1, wherein

$R_1$ and $R_2$ each are independently hydrogen, saturated or unsaturated substituted or unsubstituted $C_1$-$C_6$ alkyl, saturated or unsaturated substituted or unsubstituted $C_3$-$C_6$ cycloalkyl, $-OC(=O)C_{1-6}$ alkyl, $-OC(=O)C_3$-$C_6$ cycloalkyl, $-C(=O)OC_{1-6}$ alkyl, $-C(=O)OC_3$-$C_6$ cycloalkyl, saturated or unsaturated substituted or unsubstituted $C_1$-$C_6$ alkoxy, saturated or unsaturated substituted or unsubstituted $C_3$-$C_6$ cycloalkoxy, $-S(O)_nRc$, $NR_CR_D$, saturated or unsaturated substituted or unsubstituted $C_1$-$C_6$ sulfonyl, saturated or unsaturated substituted or unsubstituted $C_1$-$C_6$ acyl, $C_6$-$C_{10}$ aryl, and 5 to 10-membered heteroaryl with 1 to 3 heteroatoms selected from N, O, and S, wherein a "substitution" refers to selective containing of 1 to 4 substituents selected from hydroxyl, halogen, cyano, sulfonyl, amino, $-S(O)_nRc$, $NR_CR_D$, saturated or unsaturated $C_1$-$C_6$ alkyl, saturated or un-

saturated $C_3$-$C_6$ cycloalkyl, -OC(=O)$C_{1-6}$ alkyl, -OC(=O)$C_3$-$C_6$ cycloalkyl, -C(=O)O$C_{1-6}$ alkyl, -C(=O)O$C_3$-$C_6$ cycloalkyl, saturated or unsaturated $C_1$-$C_6$ sulfonyl, saturated or unsaturated $C_1$-$C_6$ acyl, $C_6$-$C_{10}$ aryl, and 6 to 10-membered heterocycloalkyl or heteroaryl with 1 to 3 heteroatoms selected from N, O, and S; or

$R_1$ and $R_2$, together with the N atoms attached thereto, produce substituted or unsubstituted 4 to 10-membered heterocycloalkyl with 1 to 3 heteroatoms selected from N, O, and S or substituted or unsubstituted 5 to 10-membered heteroaryl with 1 to 3 heteroatoms selected from N, O, and S, wherein a "substitution" refers to selective containing of 1 to 4 substituents selected from hydroxyl, halogen, cyano, sulfonyl, amino, -S(O)$_n$Rc, $NR_CR_D$, $C_1$-$C_6$ alkyl, $C_3$-$C_6$ cycloalkyl, $C_1$-$C_6$ alkoxy, $C_3$-$C_6$ cycloalkoxy, -OC(=O)$C_{1-6}$ alkyl, -OC(=O)$C_3$-$C_6$ cycloalkyl, -C(=O)O$C_{1-6}$ alkyl, -C(=O)O$C_3$-$C_6$ cycloalkyl, $C_1$-$C_6$ alkyl substituted with halogen, hydroxyl, amino, or $R_E$, $C_3$-$C_6$ cycloalkyl, $C_1$-$C_6$ alkoxy, and $C_3$-$C_6$ cycloalkoxy, wherein $R_E$ is selected from hydrogen, halogen, cyano, hydroxyl, amino, saturated or unsaturated $C_1$-$C_6$ alkyl, $C_3$-$C_6$ cycloalkyl, -S(O)$_n$Rc, $NR_CR_D$, saturated or unsaturated $C_1$-$C_6$ alkyl substituted with deuterium, halogen, hydroxyl, or amino, $C_3$-$C_6$ cycloalkyl substituted with deuterium, halogen, hydroxyl, or amino, saturated or unsaturated $C_1$-$C_6$ alkoxy, $C_3$-$C_6$ cycloalkoxy, saturated or unsaturated $C_1$-$C_6$ alkoxy substituted with deuterium, halogen, hydroxyl, or amino, and $C_3$-$C_6$ cycloalkoxy substituted with deuterium, halogen, hydroxyl, or amino.

**8.** The substituted pyrimidinyl hydrazide compound shown in the formula I and the optical isomer, prodrug, or pharmaceutically-acceptable salt thereof according to claim 1, wherein $R_3$ is halogen, -S(O)$_n$Rc, $NR_CR_D$, saturated or unsaturated $C_1$-$C_6$ alkyl, $C_3$-$C_6$ cycloalkyl, $C_1$-$C_6$ alkoxy, $C_3$-$C_6$ cycloalkoxy, saturated or unsaturated $C_1$-$C_6$ alkyl substituted with halogen, hydroxyl, amino, or $R_H$, $C_3$-$C_6$ cycloalkyl, $C_1$-$C_6$ alkoxy, and $C_3$-$C_6$ cycloalkoxy, wherein the substituent $R_H$ is selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

**9.** The substituted pyrimidinyl hydrazide compound shown in the formula I and the optical isomer, prodrug, or pharmaceutically-acceptable salt thereof according to claim 1, wherein $R_1$ and $R_2$ each are independently hydrogen, saturated or unsaturated substituted or unsubstituted $C_1$-$C_3$ alkyl, saturated or unsaturated substituted or unsubstituted $C_3$-$C_6$ cycloalkyl, -OC(=O)$C_{1-3}$ alkyl, -OC(=O)$C_3$-$C_6$ cycloalkyl, -C(=O)O$C_{1-3}$ alkyl, -C(=O)O$C_3$-$C_6$ cycloalkyl, -S(O)$_n$Rc, $NR_CR_D$, saturated or unsaturated substituted or unsubstituted $C_1$-$C_3$ sulfonyl, saturated or unsaturated substituted or unsubstituted $C_1$-$C_3$ acyl, $C_6$-$C_{10}$ aryl, and 6 to 8-membered heteroaryl with 1 to 3 heteroatoms selected from N, O, and S, wherein a "substitution" refers to selective containing of 1 to 4 substituents selected from hydroxyl, halogen, cyano, sulfonyl, amino, -S(O)$_n$Rc, $NR_CR_D$, -OC(=O)$C_{1-3}$ alkyl, -OC(=O)$C_3$-$C_6$ cycloalkyl, -C(=O)O$C_{1-3}$ alkyl, -C(=O)O$C_3$-$C_6$ cycloalkyl, saturated or unsaturated $C_1$-$C_3$ alkyl, saturated or unsaturated $C_3$-$C_6$ cycloalkyl, saturated or unsaturated $C_1$-$C_3$ sulfonyl, saturated or unsaturated $C_1$-$C_3$ acyl, $C_6$-$C_{10}$ aryl, and 6 to 8-membered heteroaryl with 1 to 3 heteroatoms selected from N, O, and S; or

$R_1$ and $R_2$, together with the N atoms attached thereto, produce substituted or unsubstituted 5 to 10-membered heterocycloalkyl with 1 to 3 heteroatoms selected from N, O, and S or substituted or unsubstituted 5 to 10-membered heteroaryl with 1 to 3 heteroatoms selected from N, O, and S, wherein a "substitution" refers to selective containing of 1 to 4 substituents selected from hydroxyl, halogen, cyano, sulfonyl, amino, -S(O)$_n$Rc, $NR_CR_D$, $C_1$-$C_3$ alkyl, $C_3$-$C_6$ cycloalkyl, $C_1$-$C_3$ alkoxy, $C_3$-$C_6$ cycloalkoxy, -OC(=O)$C_{1-3}$ alkyl, -OC(=O)$C_3$-$C_6$ cycloalkyl, -C(=O)O$C_{1-3}$ alkyl, -C(=O)O$C_3$-$C_6$ cycloalkyl, $C_1$-$C_3$ alkyl substituted with halogen, hydroxyl, amino, or $R_H$, $C_3$-$C_6$ cycloalkyl, $C_1$-$C_3$ alkoxy, and $C_3$-$C_6$ cycloalkoxy, wherein $R_H$ is selected from hydrogen, halogen, cyano, hydroxyl, amino, saturated or unsaturated $C_1$-$C_3$ alkyl, $C_3$-$C_6$ cycloalkyl, -S(O)$_n$Rc, $NR_CR_D$, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

**10.** The substituted pyrimidinyl hydrazide compound shown in the formula I and the optical isomer, prodrug, or pharmaceutically-acceptable salt thereof according to claim 1, wherein $R_3$ is halogen, -S(O)$_n$Rc, $NR_CR_D$, saturated or unsaturated $C_1$-$C_3$ alkyl, $C_3$-$C_6$ cycloalkyl, $C_1$-$C_3$ alkoxy, $C_3$-$C_6$ cycloalkoxy, saturated or unsaturated $C_1$-$C_3$ alkyl substituted with halogen, hydroxyl, amino, or $R_F$, $C_3$-$C_6$ cycloalkyl, $C_1$-$C_3$ alkoxy, and $C_3$-$C_6$ cycloalkoxy, wherein the substituent $R_H$ is selected from hydrogen, halogen, cyano, hydroxyl, amino, saturated or unsaturated $C_1$-$C_3$ alkyl, $C_3$-$C_6$ cycloalkyl, saturated or unsaturated $C_1$-$C_3$ alkyl substituted with halogen, hydroxyl, or amino, $C_3$-$C_6$ cycloalkyl substituted with halogen, hydroxyl, or amino, saturated or unsaturated $C_1$-$C_3$ alkoxy, $C_3$-$C_6$ cycloalkoxy, saturated or unsaturated $C_1$-$C_3$ alkoxy substituted with halogen, hydroxyl, or amino, and $C_3$-$C_6$ cycloalkoxy substituted with halogen, hydroxyl, or amino.

11. The substituted pyrimidinyl hydrazide compound shown in the formula I and the optical isomer, prodrug, or pharmaceutically-acceptable salt thereof according to claim 1, wherein a structure formed by $R_1$ and $R_2$, together with the N atoms attached thereto, is selected from the following groups:

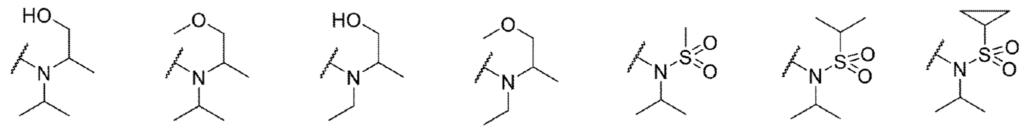

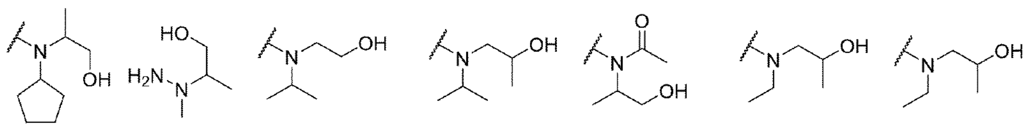

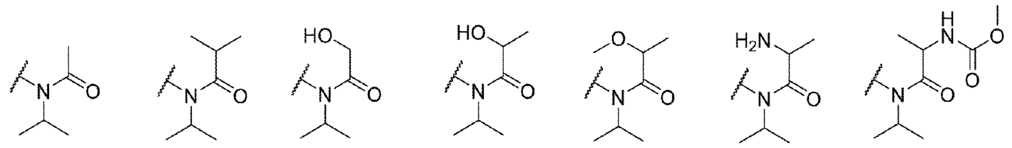

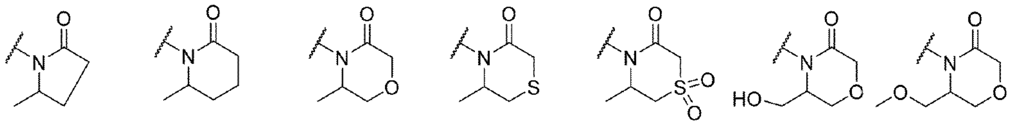

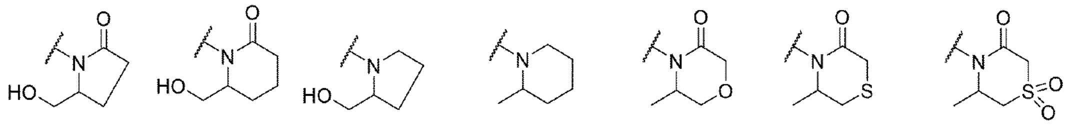

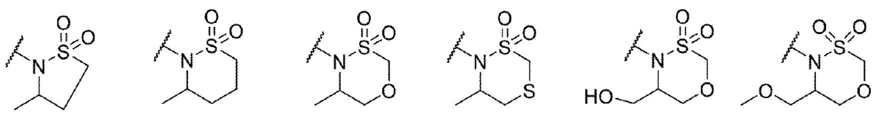

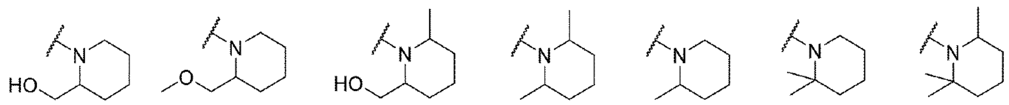

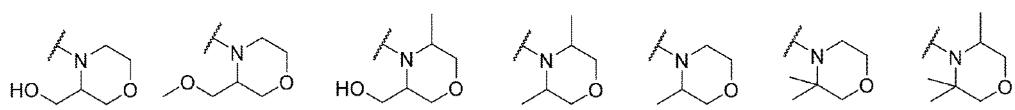

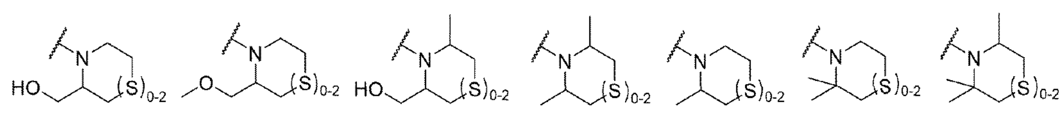

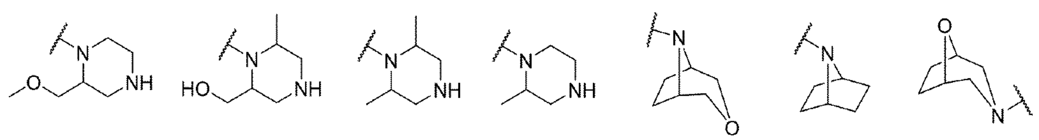

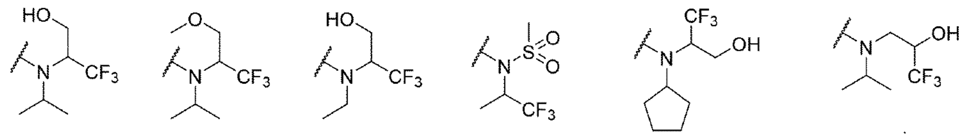

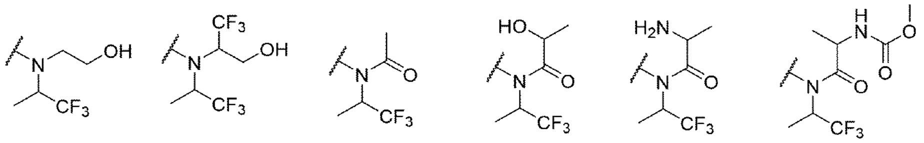

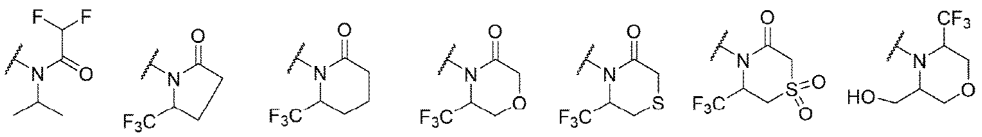

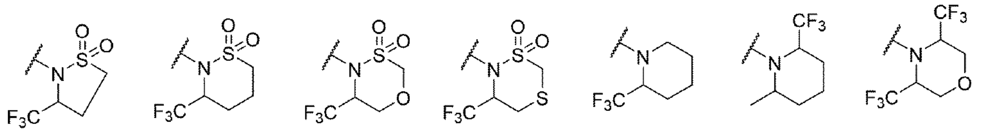

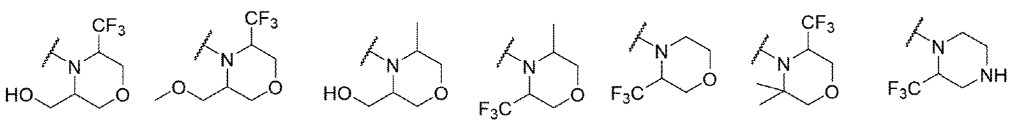

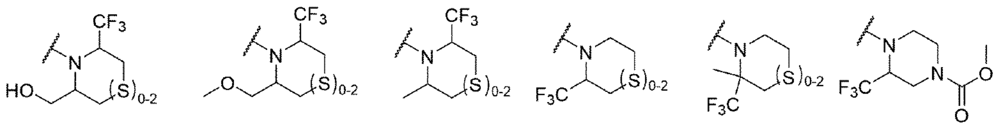

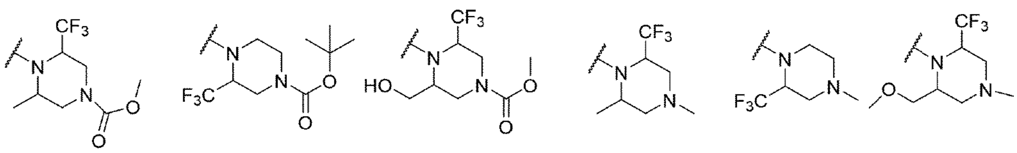

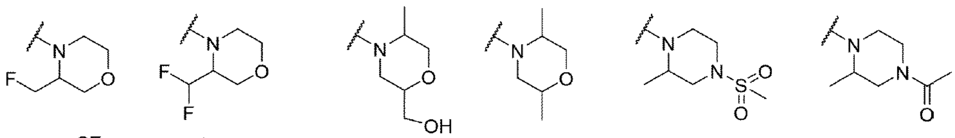

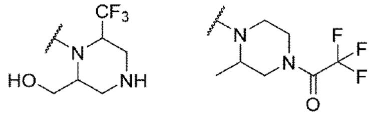

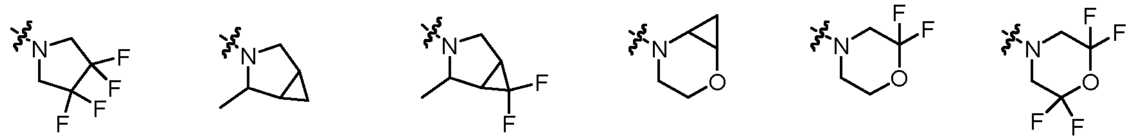

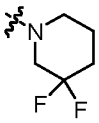 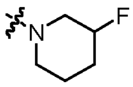 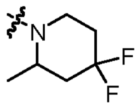 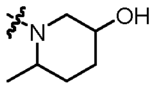 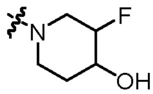 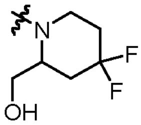 .

**12.** The substituted pyrimidinyl hydrazide compound shown in the formula I and the optical isomer, prodrug, or pharmaceutically-acceptable salt thereof according to claim 1, wherein the substituted pyrimidinyl hydrazide compound and the optical isomer, prodrug, or pharmaceutically-acceptable salt thereof each are selected from the following compounds:

| No. | structural formula | No. | structural formula | No. | structural formula |
|---|---|---|---|---|---|
| 1 | 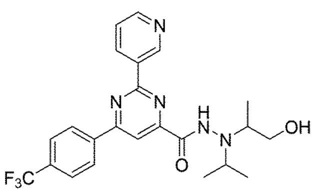 | 2 | 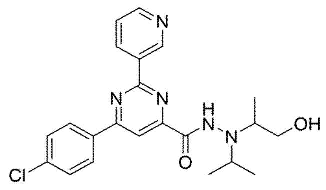 | 3 | 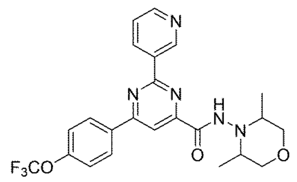 |
| 4 | 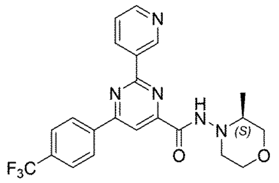 | 5 | 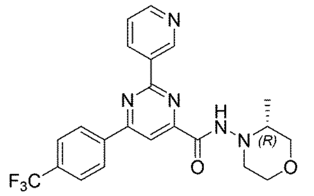 | 6 | 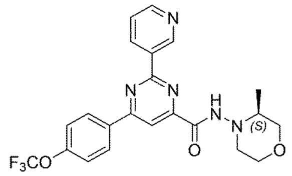 |
| 7 | 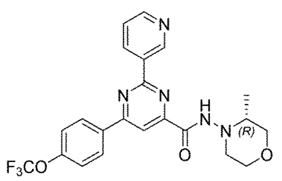 | 8 | 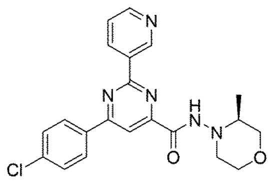 | 9 | 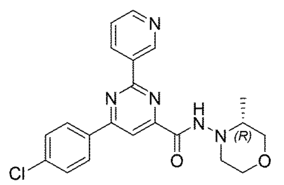 |
| 10 | 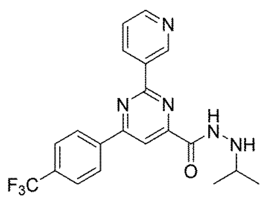 | 11 | 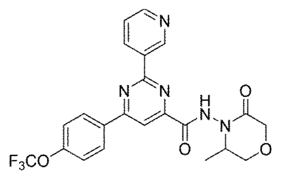 | 12 | 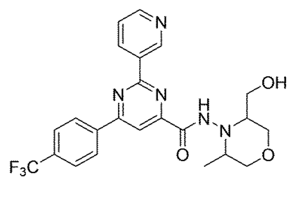 |
| 13 | 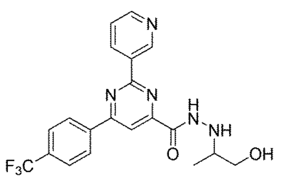 | 14 | 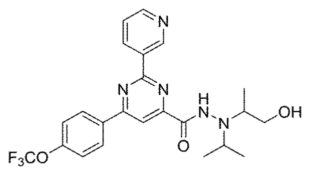 | 15 | 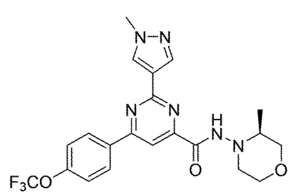 |
| 16 | 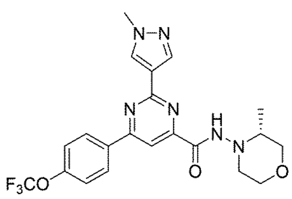 | 17 | 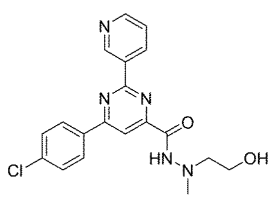 | 18 | 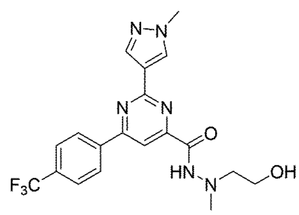 |

(continued)

| No. | structural formula | No. | structural formula | No. | structural formula |
|---|---|---|---|---|---|
| 19 | 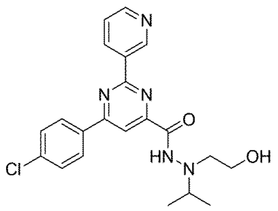 | 20 | 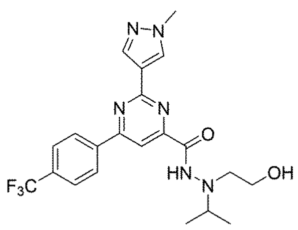 | 21 | 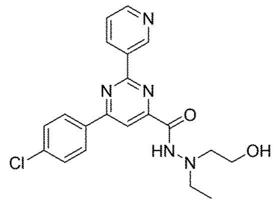 |
| 22 | 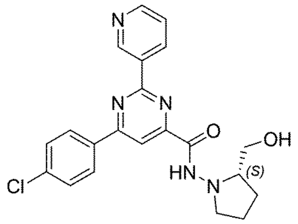 | 23 | 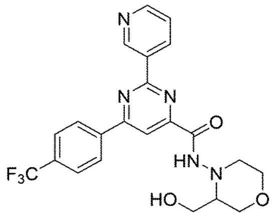 | 24 | 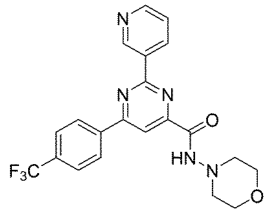 |
| 25 | 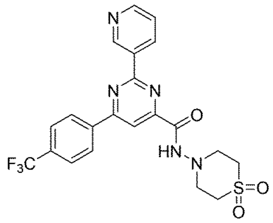 | 26 | 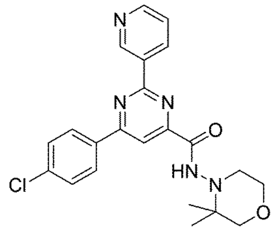 | 27 | 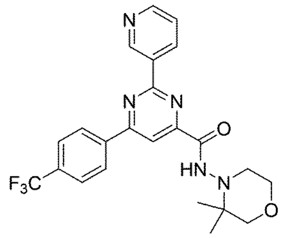 |
| 28 | 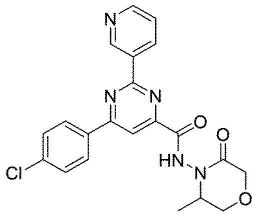 | 29 | 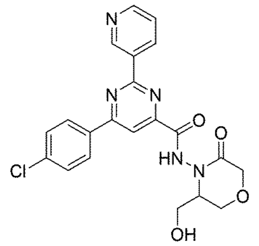 | 30 | 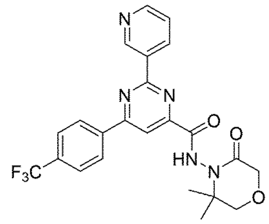 |
| 31 | 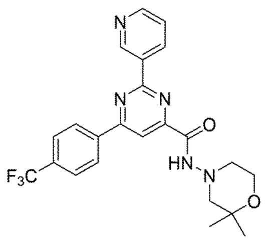 | 32 | 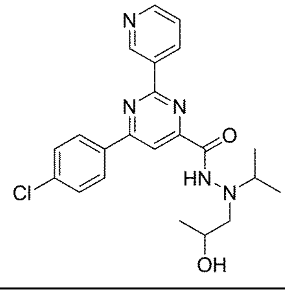 | 33 | 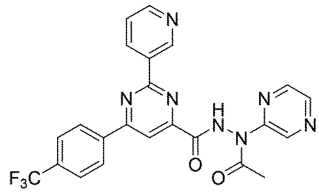 |
| 34 | 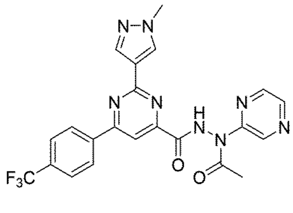 | 35 | 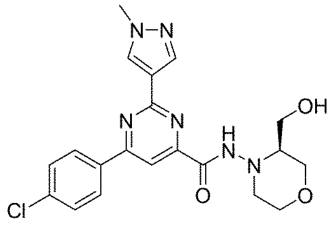 | 36 | 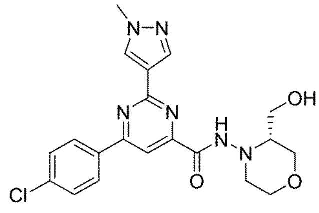 |

(continued)

| No. | structural formula | No. | structural formula | No. | structural formula |
|---|---|---|---|---|---|
| 37 | 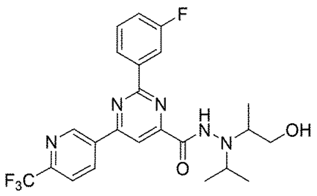 | 38 | 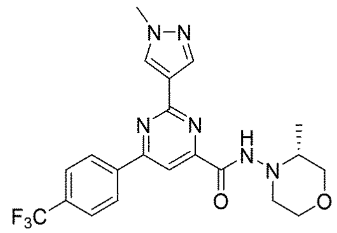 | 39 | 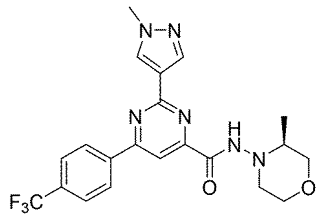 |
| 40 | 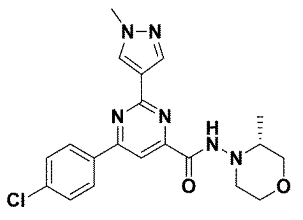 | 41 | 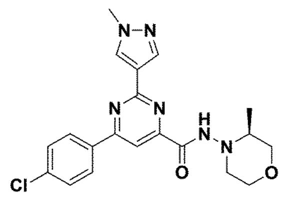 | 42 | 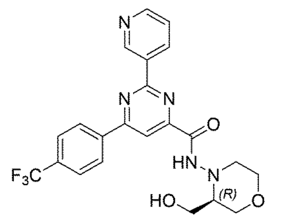 |
| 43 | 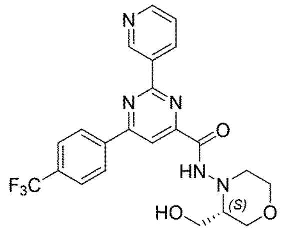 | 44 | 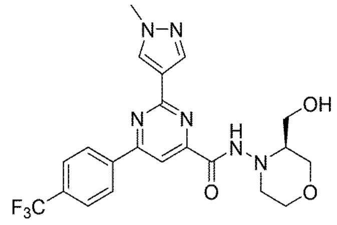 | 45 | 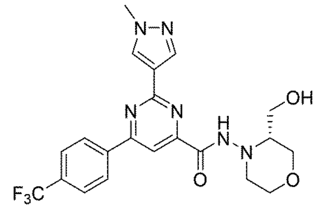 |
| 46 | 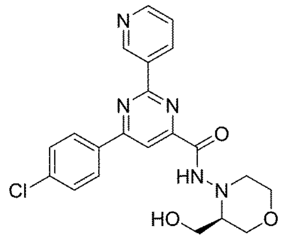 | 47 | 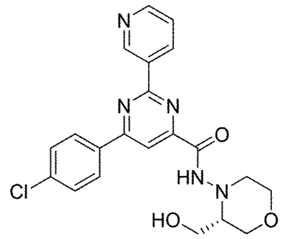 | 48 | 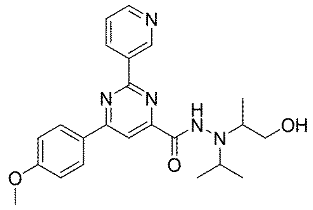 |
| 49 | 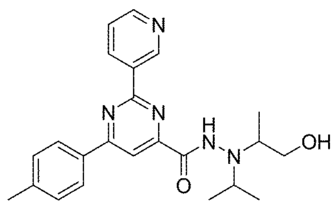 | 50 | 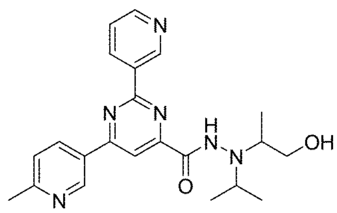 | 51 | 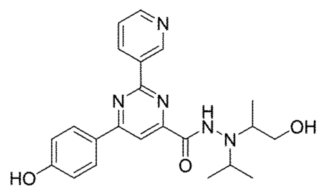 |
| 52 | 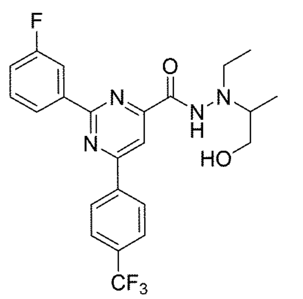 | 53 | 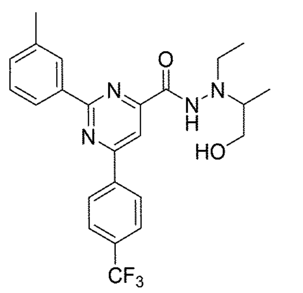 | 54 | 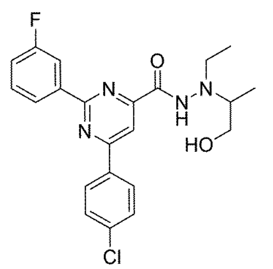 |

(continued)

| No. | structural formula | No. | structural formula | No. | structural formula |
|---|---|---|---|---|---|
| 55 | 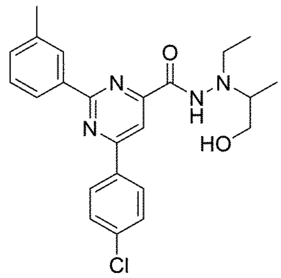 | 56 | 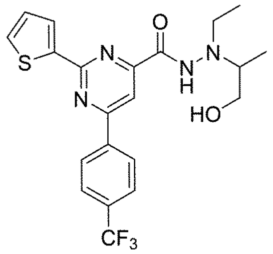 | 57 | 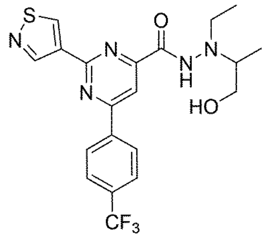 |
| 58 | 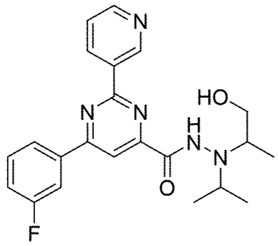 | 59 | 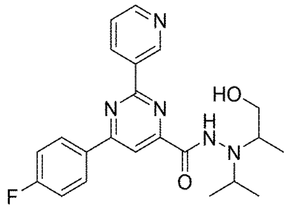 | 60 | 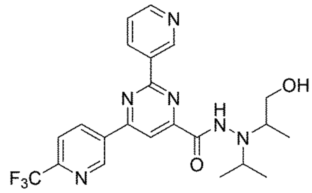 |
| 61 | 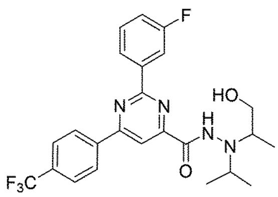 | 62 | 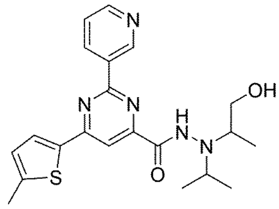 | 63 | 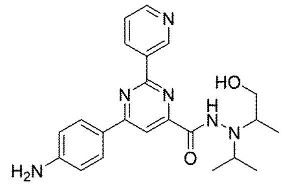 |
| 64 | 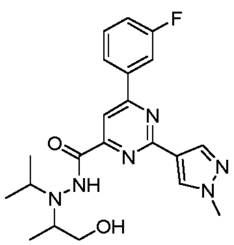 | 65 | 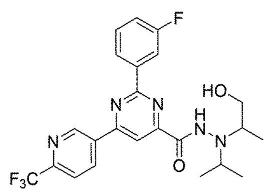 | 66 | 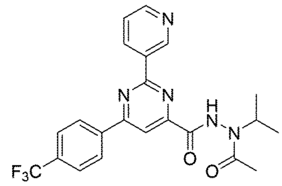 |
| 67 | 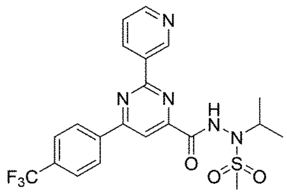 | 68 | 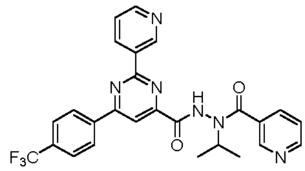 | | |

**13.** A preparation method of the substituted pyrimidinyl hydrazide compound and the optical isomer, prodrug, or pharmaceutically-acceptable salt thereof according to any one of claims 1 to 12, wherein the preparation method is shown in the following reaction equation 1:

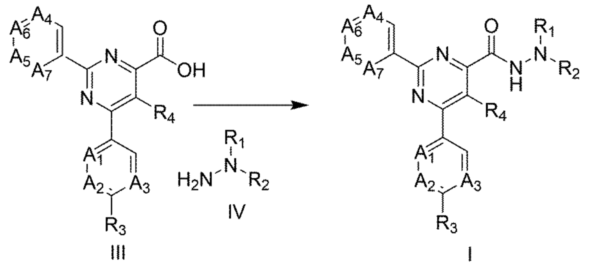


III I

reaction equation 1;

the preparation method comprises: allowing a carboxylic acid compound shown in a formula III to undergo a condensation reaction with a hydrazine compound shown in a formula IV to obtain the substituted pyrimidinyl hydrazide compound shown in the formula I;
the carboxylic acid compound shown in the formula III is prepared according to one of the following processes:

process A

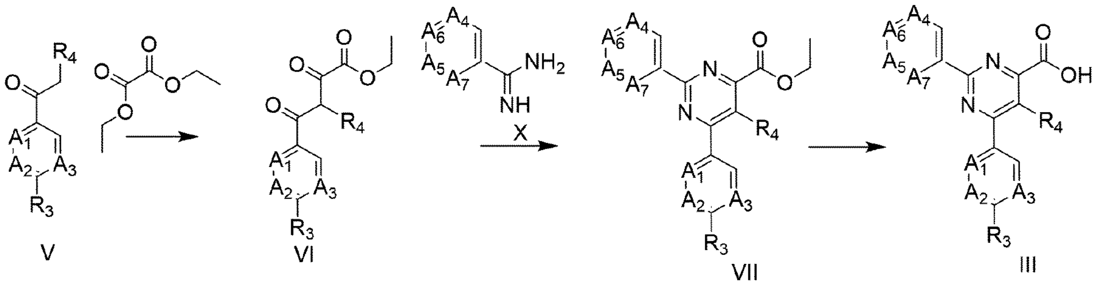


V VI VII III ;

1) allowing a methyl carbonyl compound shown in a formula V to react with dimethyl oxalate to produce a compound shown in a formula VI;
2) allowing the compound shown in the formula VI to undergo a cyclization reaction with a hydrazine compound shown in a formula X to produce a pyrimidine compound shown in a formula VII; and
3) hydrolyzing the pyrimidine compound shown in the formula VII to obtain the carboxylic acid compound shown in the formula III;

process B

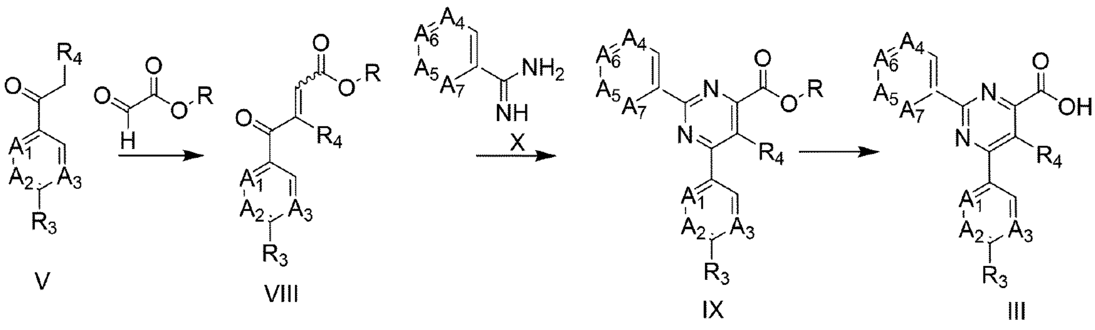


V VIII IX III ;

1) allowing the methyl carbonyl compound shown in the formula V to react with a monoalkyl oxalate

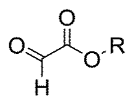

to produce a compound shown in a formula VIII, wherein a substituent R is methyl, ethyl, isopropyl, or

tert-butyl;

2) allowing the compound shown in the formula VIII to undergo a cyclization reaction with the hydrazine compound shown in the formula X to produce a pyrimidine compound shown in a formula IX; and

3) hydrolyzing the pyrimidine compound shown in the formula IX to remove the substituent R to obtain the carboxylic acid compound shown in the formula III;

process C

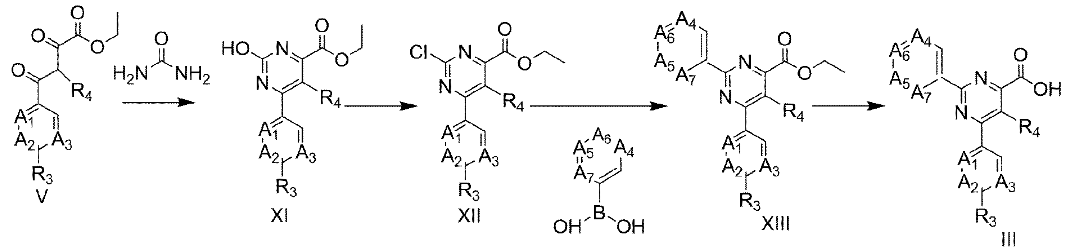

V XI XII XIII III

;

1) allowing the methyl carbonyl compound shown in the formula V to undergo a cyclization reaction with urea to produce a compound shown in a formula XI;

2) allowing the compound shown in the formula XI to react with a chlorination reagent comprising phosphorus oxychloride, phosphorus pentachloride, or thionyl chloride to produce a chlorinated compound shown in a formula XII;

3) allowing the chlorinated compound shown in the formula XII to react with a boric acid compound

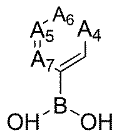

,

or a corresponding borate, or a trialkyl tin compound to produce a compound shown in a formula XIII; and

4) conducting ethyl removal for the compound shown in the formula XIII to generate a carboxylic acid group to obtain the compound shown in the formula III;

process D

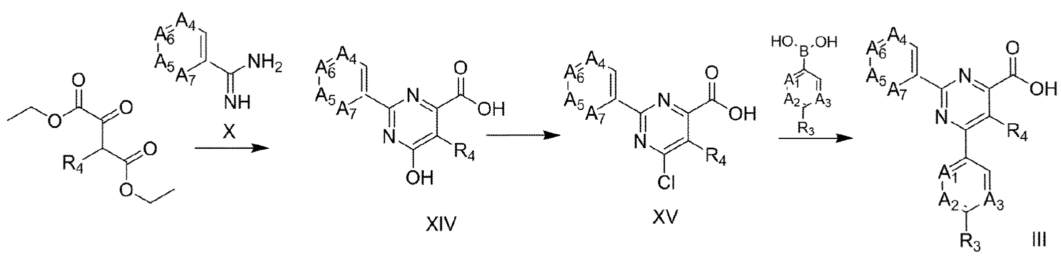

X XIV XV III

;

1) allowing a carbonate compound

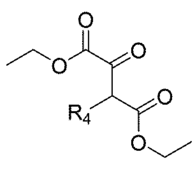

to undergo a cyclization reaction with the hydrazine compound shown in the formula X to produce a

compound shown in a formula XIV;

2) allowing the compound shown in the formula XIV to react with a chlorination reagent comprising phosphorus oxychloride, phosphorus pentachloride, or thionyl chloride to produce a chlorinated compound shown in a formula XV;

3) allowing the chlorinated compound shown in the formula XV to react with a boric acid compound

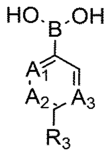,

or a corresponding borate, or a trialkyl tin compound to obtain the compound shown in the formula III;

process E

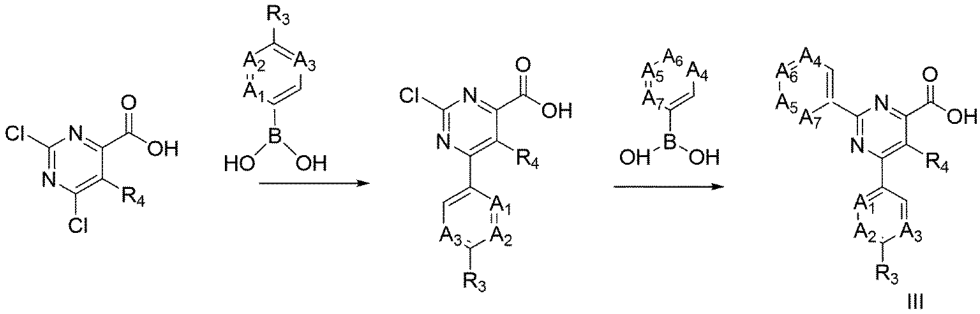

III

1) allowing a pyrimidine compound

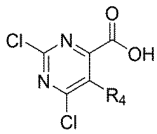

to react with a boric acid compound

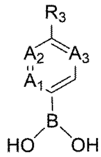,

or a corresponding borate, or a trialkyl tin compound, and conducting separation and purification to obtain a compound

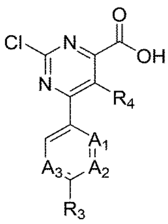;

and

2) allowing the compound

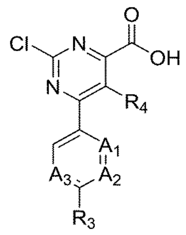

to react with the boric acid compound

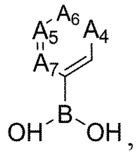

or a corresponding borate, or a trialkyl tin compound to obtain the compound shown in the formula III;

process F

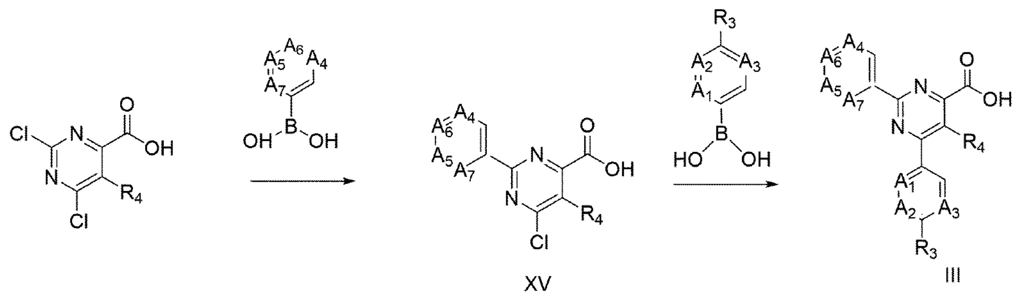

1) allowing the pyrimidine compound

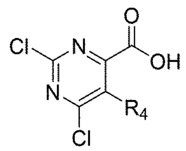

to react with the boric acid compound

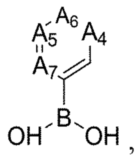

or a corresponding borate, or a trialkyl tin compound, and conducting separation and purification to obtain a compound shown in the formula XV; and
2) allowing the compound shown in the formula XV to react with the boric acid compound

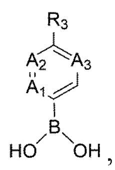

or a corresponding borate, or a trialkyl tin compound to obtain the compound shown in the formula III; and

the hydrazine compound shown in the formula IV is synthesized according to one of the following processes:

process G

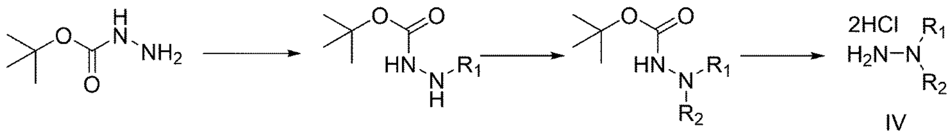

1) dissolving $BocNHNH_2$ (tert-butoxycarbonyl hydrazide) in an alcohol solvent comprising methanol or ethanol, adding a ketone compound ($R_1(C=O)R_1$) or an aldehyde compound ($R_1(C=O)H$) to allow a reaction, adding sodium borohydride to allow hydrogenation reduction, and conducting purification and separation to produce a compound

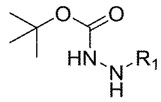
;

2) dissolving the compound

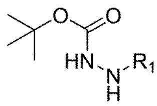

in an amine solvent comprising N,N-diisopropylyelethylamine (DIEA), and adding a ketone compound ($R_2(C=O)R_2$), an aldehyde compound ($R_2(C=O)H$), or a corresponding $R_2$-substituted epoxy compound to allow a reaction to obtain a compound

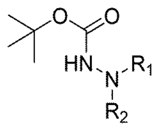
;

and
3) dissolving the compound

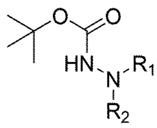

in an alcohol solvent comprising methanol or ethanol, and adding an excess amount of a solution of HCl in methanol to obtain a hydrochloride of the compound shown in the formula IV; and

process H

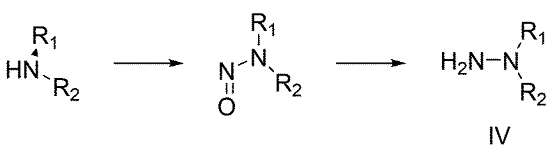

1) dissolving a substituted amino compound ($HNR_1R_2$) in a solvent, adding a nitrosation reagent selected from tert-butyl nitrite and isobutyl nitrite, heating for reflux, and conducting vacuum concentration to obtain a nitrosamino compound ($R_1N(R_2)$-N=O) as an intermediate; and
2) dissolving the nitrosamino compound ($R_1N(R_2)$-N=O) in a solvent, and adding a hydrogenation reagent comprising lithium aluminum hydride (LAH) to allow a hydrogenation reaction to obtain the compound shown in the formula IV,

wherein substituents $A_1$ to $A_7$ and $R_1$ to $R_4$ each have the same definition as in claim 1.

**14.** A pharmaceutical composition comprising a therapeutically-effective amount of the substituted pyrimidinyl hydrazide compound shown in the formula I and the optical isomer, prodrug, or pharmaceutically-acceptable salt thereof according to any one of claims 1 to 12 and a pharmaceutically-acceptable excipient or carrier.

**15.** A use of the substituted pyrimidinyl hydrazide compound shown in the formula I and the optical isomer, prodrug, or pharmaceutically-acceptable salt thereof according to any one of claims 1 to 12 in preparation of an aryl hydrocarbon receptor (AHR) disorder inhibitor.

**16.** A use of the substituted pyrimidinyl hydrazide compound shown in the formula I and the optical isomer, prodrug, or pharmaceutically-acceptable salt thereof according to any one of claims 1 to 12 in preparation of a drug for treating a tumor associated with an AHR disorder.

**17.** A method for treating a tumor associated with an AHR disorder, comprising: administering an effective amount of the substituted pyrimidinyl hydrazide compound shown in the formula I and the optical isomer, prodrug, or pharmaceutically-acceptable salt thereof according to any one of claims 1 to 12 or the pharmaceutical composition according to claim 13 to a subject in need.

**INTERNATIONAL SEARCH REPORT**

International application No.
**PCT/CN2023/090761**

**A. CLASSIFICATION OF SUBJECT MATTER**

C07D239/04(2006.01)i；C07D401/04(2006.01)i；C07D403/14(2006.01)i；A61K31/506(2006.01)i；A61P35/00(2006.01)i

According to International Patent Classification (IPC) or to both national classification and IPC

**B. FIELDS SEARCHED**

Minimum documentation searched (classification system followed by classification symbols)

IPC：C07D239/-，C07D401/-，C07D403/-，A61K31/-

Documentation searched other than minimum documentation to the extent that such documents are included in the fields searched

Electronic data base consulted during the international search (name of data base and, where practicable, search terms used)

CNTXT, VEN, STN(REG, CAPLUS): 嘧啶, 酰肼, 芳香烃受体, 肿瘤, 癌, pyrimidine, aryl hydrocarbon receptor, AHR, tumor, cancer, 基于式I的结构检索, search based on structural formula I

**C. DOCUMENTS CONSIDERED TO BE RELEVANT**

| Category* | Citation of document, with indication, where appropriate, of the relevant passages | Relevant to claim No. |
|---|---|---|
| A | WO 2006084017 A2 (BRISTOL-MYERS SQUIBB COMPANY) 10 August 2006 (2006-08-10)<br>entire document | 1-16 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 August 2023** | **07 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/090761**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:
The subject matter of claim 17 relates to a method for treatment of the human or animal body by therapy (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/090761**

| Patent document cited in search report | Publication date (day/month/year) | Patent family member(s) | Publication date (day/month/year) |
|---|---|---|---|
| WO 2006084017 A2 | 10 August 2006 | WO 2006084017 A3 | 14 December 2006 |
| | | ES 2336025 T3 | 07 April 2010 |
| | | EP 1848714 A2 | 31 October 2007 |
| | | EP 1848714 B1 | 30 December 2009 |
| | | DE 602006011434 D1 | 11 February 2010 |
| | | JP 2008530012 A | 07 August 2008 |
| | | JP 4906738 B2 | 28 March 2012 |
| | | US 2006178388 A1 | 10 August 2006 |
| | | US 2010029649 A1 | 04 February 2010 |
| | | US 7923556 B2 | 12 April 2011 |
| | | AT 453639 T | 15 January 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MURRAY et al.** *Nat. Rev. Cancer*, 2014, vol. 14 (12), 801-814
- **BERSTENETAL.** *Nat. Rev. Cancer*, 2013, vol. 13 (12), 827-841
- **REYES et al.** *Science*, 1992, vol. 256 (5060), 1193-1195
- **NGUYEN et al.** *Front. Immunol.*, 2014, vol. 5, 511
- **GRAMATZKIETAL**. *Oncogene*, 2009, vol. 28 (28), 2593-2605
- **BUIETAL**. *Oncogene*, 2009, vol. 28 (41), 3642-3651
- **ESSERETAL.** *Trends. Immunol.*, 2009, vol. 30 (9), 447-454
- **QUINTANA et al.** *Nature*, 2008, vol. 453 (7191), 65-71
- **MEZRICH et al.** *J. Immunol.*, 2010, vol. 185 (6), 3190-3198
- **KIMURA et al.** *J. Exp. Med.*, 2009, vol. 206 (9), 2027-2035
- **WANG et al.** *Clin. Exp. Immunol.*, 2014, vol. 177 (2), 521-530
- **; WEIET**. *Lab. Invest.*, 2014, vol. 94 (5), 528-535
- **NGUYEN et al.** *Proc. Natl. Acad. Sci. USA*, 2010, vol. 107 (46), 19961-19966
- **OPITZ et al.** *Nature*, 2011, vol. 478 (7368), 197-203
- **BERGE et al.** Pharmaceutical Salts. *Journal of Pharmaceutical Science*, 1977, vol. 66, 1-19
- **BUCKLEY, S. M. K. et al.** In vivo bioimaging with tissue-specific transcription factor activated luciferase reporters.. *Sci. Rep.*, 2015, vol. 5, 11842